(19) Europäisches Patentamt
European Patent Office
Office européen des brevets

(11) EP 3 066 076 B1

(12) **EUROPEAN PATENT SPECIFICATION**

(45) Date of publication and mention of the grant of the patent:
**27.03.2019 Bulletin 2019/13**

(21) Application number: **14799928.8**

(22) Date of filing: **29.10.2014**

(51) Int Cl.:
***C07D 211/34*** *(2006.01)*

(86) International application number:
**PCT/US2014/062800**

(87) International publication number:
**WO 2015/069507 (14.05.2015 Gazette 2015/19)**

# (54) PROCESS FOR THE PREPARATION OF METHYLPHENIDATE AND PHARMACEUTICAL SALTS THEREOF

VERFAHREN ZUR HERSTELLUNG VON METHYLPHENIDAT UND SEINER PHARMAZEUTISCHEN SALZE

PROCÉDÉ POUR LA PRÉPARATION DE MÉTHYLPHÉNIDATE ET SELS PHARMACEUTIQUES DE CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2013 US 201361901674 P**
**11.07.2014 US 201462023340 P**

(43) Date of publication of application:
**14.09.2016 Bulletin 2016/37**

(73) Proprietor: **Noramco, Inc.**
**Athens, GA 30601 (US)**

(72) Inventors:
- **BARR, Charla**
**Monroe, Georgia 30655 (US)**
- **DOBISH, Mark C.**
**Bogart, Georgia 30622 (US)**
- **SMITH, Brian J.**
**Wilmington, Delaware 19806 (US)**
- **STEFANICK, Stephen M.**
**Phillipsburg, New Jersey 08865 (US)**

(74) Representative: **Carpmaels & Ransford LLP**
**One Southampton Row**
**London WC1B 5HA (GB)**

(56) References cited:
**WO-A1-2004/069799**
**WO-A1-2010/080787**
**WO-A1-2011/067783**
**WO-A2-03/031411**
**WO-A2-2008/125914**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 066 076 B1

**Description**

FIELD OF THE INVENTION

The present invention is directed to a process for the preparation of methylphenidate, stereoisomer, mixture of stereoisomers and pharmaceutically acceptable salts thereof, more particularly, the sulfate and hydrochloride salts of methylphenidate, di-threo-methylphenidate and dex-methylphenidate. Methods of removing or reducing impurities from the above described process and/or resulting products are also disclosed.

BACKGROUND OF THE INVENTION

Methylphenidate (MPH; MPD) is a psychostimulant drug approved for the treatment of attention-deficit hyperactivity disorder (ADHD), postural orthostatic tachycardia syndrome and narcolepsy. Best known by its 1948 trademarked name of RITALIN, it was first approved by the FDA in 1955 for the treating ADHD.

RITALIN (di-threo-methylphenidate HCl), a cyclic amine, may be prepared by reacting a mixture of di-threo-ritalinic acid with sulfonyl chloride, in the presence of HCl, and further in methanol, with typical yields of about 70-75%. However, the process for preparation of RITALIN has a number of attributes which make it less advantageous for large scale / commercial manufacture, including but not limited to (a) off-gasing of $SO_2$ and HCl during methanol distillation, which results in corrosion of manufacturing equipment, (b) the use of sulfonyl chloride, a corrosive reagent, and / or (c) limited yield and / or purity.

REKHA, et al., Org. Process Res. Dev., 2009, pp769-773, Vol. 13 disclose a process for the esterification of carboxylic acids. More particularly, Rekha, et al., disclose a process wherein the carboxylic acid is reacted with dimethylcarbonate, in the presence of sulfuric acid. For nitrogen containing carboxylic acids (e.g. 2-aminobenzoic acid, 4-aminobenzoic acid, nicotinic acid and isonicotinic acid), Rekha et al., disclose reacting the carboxylic acid with 3.5 eq. dimethyl \carbonate in the presence of 1.4 eq. sulfuric acid. For monocarboxylic acids (e.g. 2-phenylacetic acid), Rekha et al., disclose reacting the carboxylic acid with 1.8 eq. dimethylcarbonate in the presence of 0.1-0.2 eq. sulfuric acid. The process(es) as described in Rekha et al., do not however, teach or suggest the use / addition of methanol to the reaction mixture. Additionally, Rekha et al., disclose that the amines such as aliphatic amines "...phenylglycine, and phenylalanine did not react at all because of high insolubility."

BOESTEN, W., et al., in PCT Publication WO 2007/039522 A2 disclose a process for the esterification of organic acids comprising "bringing the organic acid into contact with a strong acid and a solution comprising dialkylcarbonate in a reaction mixture". BOESTEN, et al., do not however disclose specific molar ratios of DMC:methanol and / or DMC:sulfuric acid for achieving improved yield, solubility and / or large scale handling.

There remains a need for a process for the preparation of methylphenidate, which process is suitable for large scale / commercial manufacture, which avoids the use of corrosive reagents such as sulfonyl chloride, and / or which results in high yields, short reaction times and / or ease in large scale handling.

SUMMARY OF THE INVENTION

In one embodiment, the present invention is directed to a process for the preparation of a compound of formula (I-S) (a sulfate salt of methylphenidate)

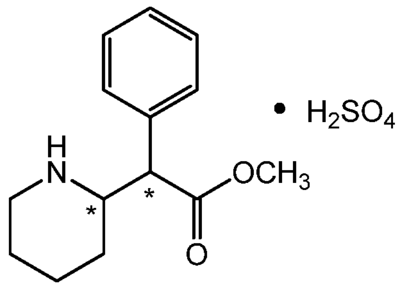

(I-S)

or a stereoisomer, mixture of stereoisomers, comprising the steps of

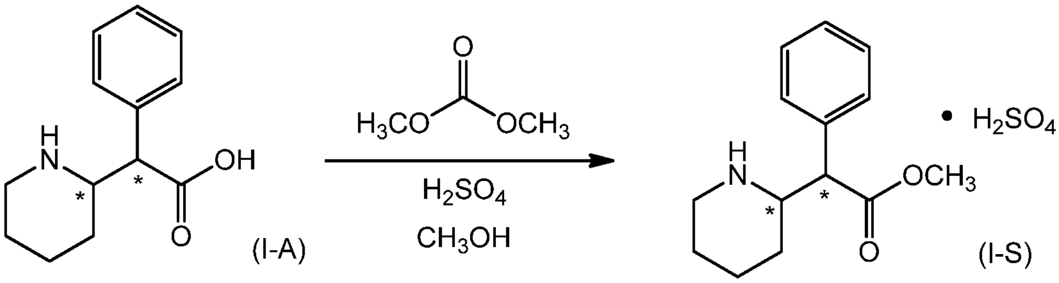


(I-A) (I-S)

providing a compound of formula (I-A) or stereoisomer or mixture of stereoisomers or pharmaceutically acceptable salt thereof (as illustrated in the above schematic) and reacting the compound of formula (I-A) with dimethylcarbonate to form a reaction mixture, for example, as depicted in the above reaction scheme; wherein the dimethylcarbonate is present in an amount in the range of from about 2.5 to about 8.0 molar equivalents (the term "equivalents" is also referred to herein as "equiv." or "eq");

adding methanol to the reaction mixture; wherein the methanol is present in an amount in the range of from about 0.75 to about 5.0 molar equivalents (relative to the moles of the compound of formula (I-A)); wherein the methanol is added as a separate component; and wherein the amount of methanol initially added to the reaction mixture, in one or more portions or aliquots, is independent of any methanol which may be later introduced into the reaction mixture;

adding sulfuric acid to the reaction mixture (optionally, after mixing the compound of formula (I-A), dimethylcarbonate and methanol); wherein the sulfuric acid is present in an amount in the range of from about 1.05 to about 2.5 molar equivalents (relative to the moles of the compound of formula (I-A));

wherein the reaction mixture is at a temperature in the range of from about room temperature to about 90°C; to yield the corresponding compound of formula (I-S).

In another embodiment, the present invention is directed to a process for the preparation of a compound of formula (I)

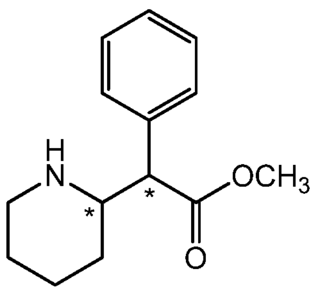

(I)

or a stereoisomer, mixture of stereoisomers or pharmaceutically acceptable salt thereof, comprising the steps of

<u>STEP A, comprising the steps of:</u>

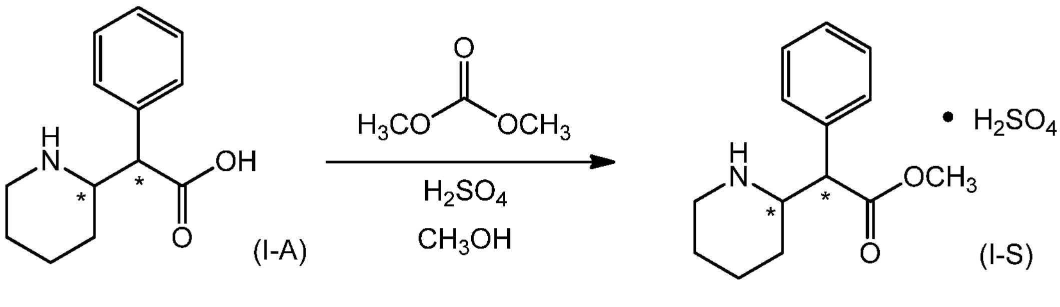


(I-A) (I-S)

i. reacting a compound of formula (I-A) or stereoisomer, mixture of stereoisomers or pharmaceutically acceptable salt thereof, with dimethylcarbonate to form a reaction mixture, for example, as depicted in the above reaction

scheme; wherein the dimethylcarbonate is present in an amount in the range of from about 2.5 to about 8.0 molar equivalents;

ii. adding methanol to the reaction mixture; wherein the methanol is present in an amount in the range of from about 0.75 to about 5.0 molar equivalents (relative to the moles of the compound of formula (I-A)); wherein the methanol is added as a separate component; and wherein the amount of methanol initially added, in one or more portions or aliquots, is independent of any methanol which may be later introduced into the reaction mixture;

iii. adding sulfuric acid to the reaction mixture; wherein the sulfuric acid is present in an amount in the range of from about 1.05 to about 2.5 molar equivalents (relative to the moles of the compound of formula (I-A));

wherein the reaction mixture is at a temperature in the range of from about room temperature to about 90°C; to yield the corresponding compound of formula (I-S) (i.e., the corresponding sulfate salt of the compound of formula (I-A));

STEP B:

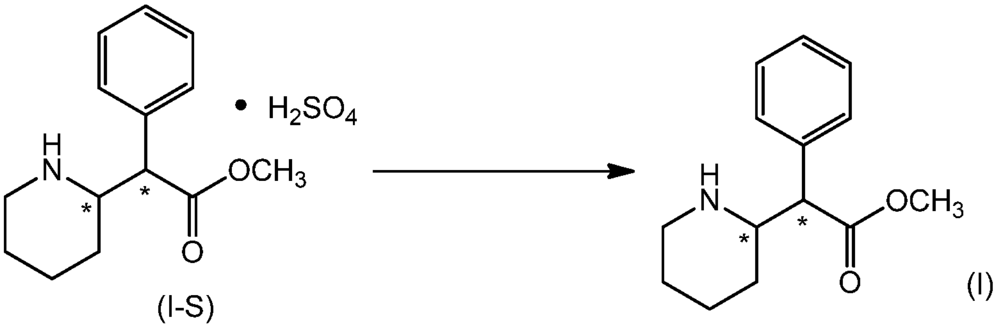

(I-S) (I)

reacting the compound of formula (I-S) for example, as depicted in the above reaction scheme, to yield the corresponding compound of formula (I) (for example by reacting the compound of formula (I-S) with a suitably selected base as described in more detail hereinafter).

In another embodiment, the present invention is directed to a process for the preparation a compound of formula (I)

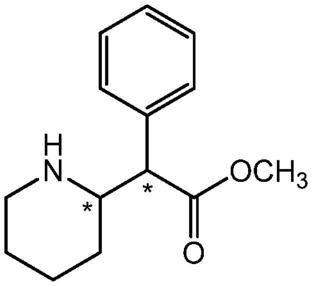

(I)

or a stereoisomer, mixture of stereoisomers or pharmaceutically acceptable salt thereof (also known as methyl 2-phenyl-2-(piperidin-2-yl)acetate or methylphenidate), comprising the steps of

STEP A, comprising the steps of:

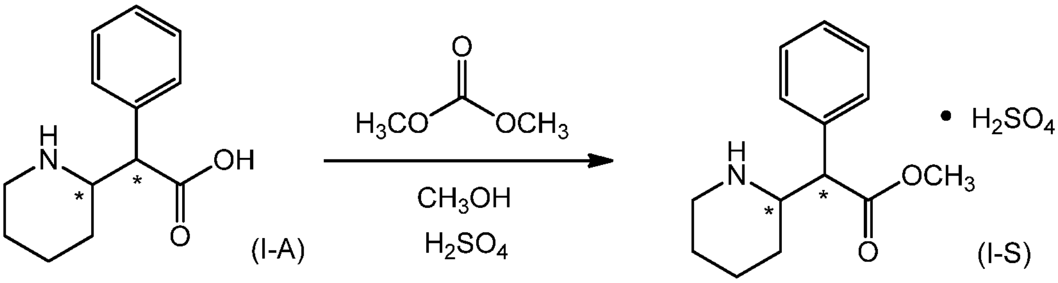


(I-A) (I-S)

reacting a compound of formula (I-A) or stereoisomer, mixture of stereoisomers or pharmaceutically acceptable salt thereof, with dimethylcarbonate to form a reaction mixture, for example, as depicted in the above reaction scheme; wherein the dimethylcarbonate is present in an amount in the range of from about 2.5 to about 8.0 molar equivalents (relative to the moles of the compound of formula (I-A));
adding methanol to the reaction mixture; wherein the methanol is present in an amount in the range of from about 0.75 to about 5.0 molar equivalents (relative to the moles of the compound of formula (I-A)); wherein said methanol is added as a separate component; and wherein the amount of methanol initially added, in one or more portions or aliquots, is independent of any methanol which may be later introduced into the reaction mixture;
adding sulfuric acid to the reaction mixture; wherein the sulfuric acid is present in an amount in the range of from about 1.05 to about 2.5 molar equivalents (relative to the moles of the compound of formula (I-A));
wherein the reaction mixture is at a temperature in the range of from about room temperature to about 90°C;
to yield the corresponding compound of formula (I-S) (i.e., the corresponding sulfate salt of the compound of formula (I-A));

STEP B:

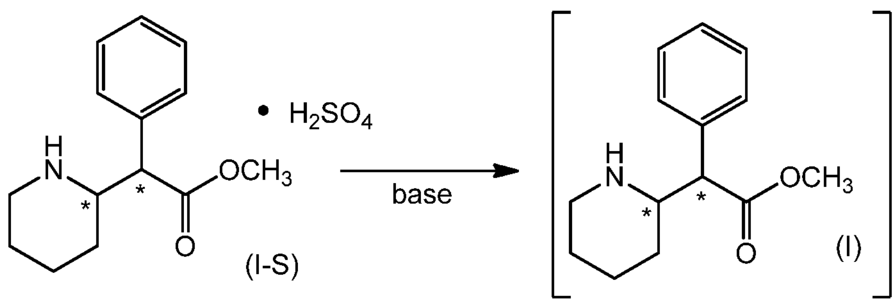


reacting the compound of formula (I-S) with a base (for example an inorganic base), for example, as depicted in the above reaction scheme; and then adding an organic solvent; to yield the corresponding compound of formula (I), in a biphasic mixture comprising an aqueous phase and an organic phase;
STEP C:
separating the phases of the biphasic mixture one from the other (optionally, to yield an aqueous layer and organic layer); wherein the compound of formula (I) is present in the organic phase (or layer); and
STEP D:

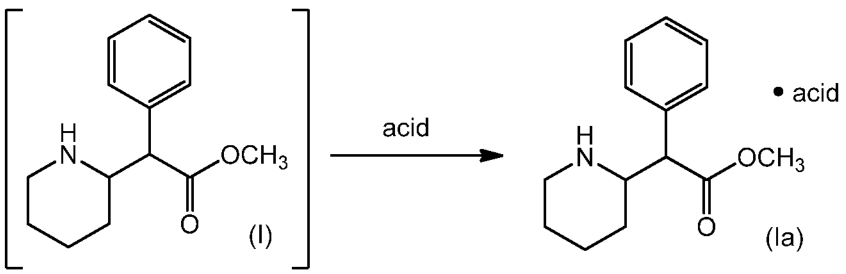


reacting the organic layer (containing the compound of formula (I)) with an acid, optionally in the presence of water, for example, as depicted in the above reaction scheme; to yield the compound of formula (Ia), the corresponding acid addition salt of the compound of formula (I).

Without being limited by theory, it is believed that the addition of water as described in more detail below aids in separating or isolating the impurity compounds of formulas (IMP-A) or (IMP-B) or (IMP-C) or (IMP-D), or salts of the compounds of formulas (IMP-A) or (IMP-B) or (IMP-C) or (IMP-D) by extraction into the water itself; and / or aids in controlling the particle size of the compound of formula (Ia) when isolated as a solid, optionally a crystalline solid.
In another embodiment, the present invention is directed to a process for the preparation of a compound of formula (I)

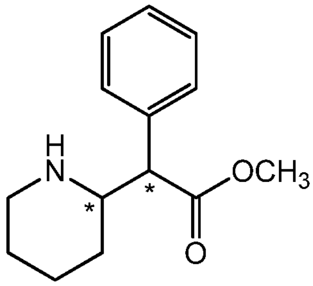

(I)

or a stereoisomer, mixture of stereoisomers or pharmaceutically acceptable salt thereof; comprising

STEP A:

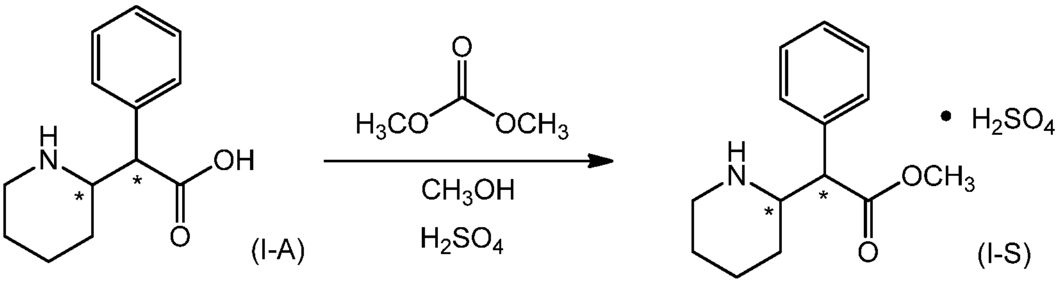


reacting a compound of formula (I-A) or stereoisomer, mixture of stereoisomers, or pharmaceutically acceptable salt thereof, with dimethylcarbonate to form a reaction mixture, for example, as depicted in the above reaction scheme; wherein the dimethylcarbonate is present in an amount in the range of from about 2.5 to about 8.0 molar equivalents (relative to the moles of the compound of formula (I-A));
wherein methanol is added to the reaction mixture; wherein the methanol is present in an amount in the range of from about 0.75 to about 5.0 molar equivalents (relative to the moles of the compound of formula (I-A)); wherein the said methanol is added as a separate component of the reaction; and wherein the amount of methanol initially added, in one or more portions or aliquots, is independent of any methanol which may be later introduced into the reaction mixture;
wherein sulfuric acid is added to the reaction mixture; wherein the sulfuric acid is present in an amount in the range of from about 1.05 to about 2.5 molar equivalents (relative to the moles of the compound of formula (I-A)); at a temperature in the range of from about room temperature to about 90°C; to yield the corresponding compound of formula (I-S) (the corresponding sulfate salt of the compound of formula (I-A));

STEP B:

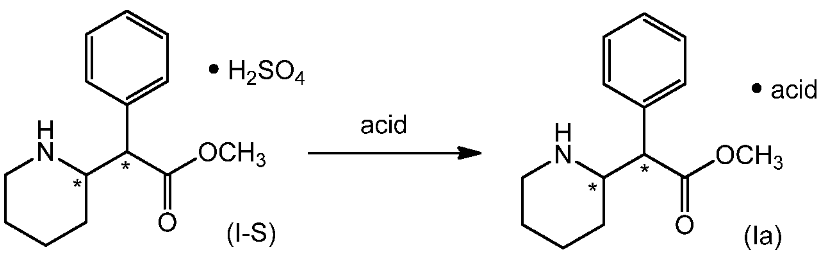


reacting the organic layer (containing the compound of formula (I-S)) with an acid, optionally in the presence of water, for example, as depicted in the above reaction scheme; to yield the compound of formula (Ia), the corresponding acid addition salt of the compound of formula (I).

As noted above, and similarly qualified, it is believed that the addition of water (described in more detail below) aids in separating or isolating the impurity compounds of formulas (IMP-A) or (IMP-B) or (IMP-C) or (IMP-D), or salts of

the compounds of formulas (IMP-A) or (IMP-B) or (IMP-C) or (IMP-D) by extraction into the water itself; and / or aids in controling the particle size of the compound of formula (Ia) when isolated as a solid, optionally as a crystalline solid.

In other embodiments, the present invention is directed to a process for the recrystallization of a compound of formula (I)

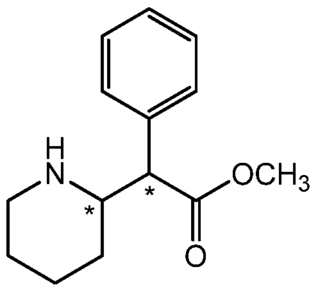

(I)

or stereoisomer, mixture of stereoisomers or pharmaceutically acceptable salt thereof; wherein the recrystallized product comprises a total of less than about 0.1 area % of impurity compounds selected from the group consisting of impurity compound of formula (IMP-5), impurity compound of formula (IMP-6), impurity compound of formula (IMP-15), impurity compound of formula (IMP-16), and mixtures thereof, as described herein.

In certain embodiments, the present invention is directed to processes for the recrystallization of a compound of formula (I) or a compound of formula (Ia), or stereoisomers or mixtures of stereoisomers thereof (for example, di-threo methylphenidate, di-threo-methylphenidate hydrochloride, dex-methylphenidate or dex-methylphenidate hydrochloride) wherein the amount of water added during recrystallization is selected to control the particle size of the final isolated solid, optionally crystalline solid.

In certain embodiments, the present invention is directed to processes for the recrystallization of a compound of formula (I) or a compound of formula (Ia), or stereoisomers or mixtures of stereoisomers thereof (for example, di-threo methylphenidate, di-threo-methylphenidate hydrochloride, dex-methylphenidate or dex-methylphenidate hydrochloride) wherein the amount of the recrystallization solvent (including any added water) which is distilled from the recrystallization mixture is controlled to maximize the yield of the final isolated product as a solid, optionally as a crystalline solid.

In certain embodiments, the present invention is directed to processes for the recrystallization of a compound of formula (I) or a compound of formula (Ia), or stereoisomers or mixtures of stereoisomers thereof (for example, di-threo methylphenidate, di-threo-methylphenidate hydrochloride, dex-methylphenidate or dex-methylphenidate hydrochloride) wherein (a) the amount of water added during recrystallization and (b) the amount of recrystallization solvent (including any added water) which is distilled from the recrystallization mixture are each selected to optimize (or attain desired specifications or targets of) both the particle size and yield of the final isolated product as a solid, optionally as a crystalline solid.

In other embodiments, the present invention is directed to a process for the recrystallization of di-threo-methylphenidate hydrochloride;

wherein the recrystallized di-threo-methylphenidate hydrochloride comprises a total of less than about 0.1 area % of one or more of impurity compounds selected from the group consisting of

an impurity compound of formula (IMP-5)

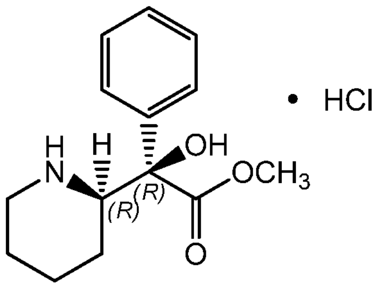

(IMP-5),

an impurity compound of formula (IMP-6)

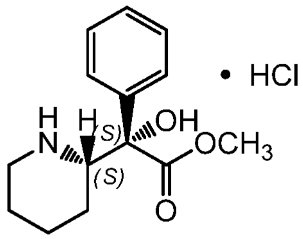

(IMP-6),

an impurity compound of formula (IMP-15)

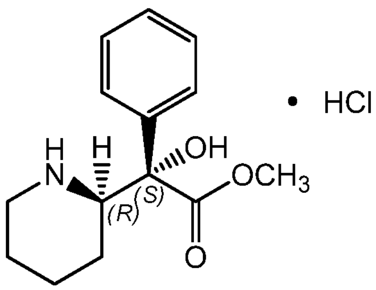

(IMP-15),

and an impurity compound of formula (IMP-16)

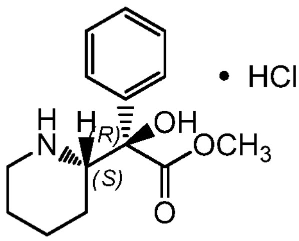

(IMP-16)

comprising the steps of:

(a) dissolving di-threo-methylphenidate hydrochloride in a mixture of an organic solvent and water; wherein the organic solvent is selected from the group consisting of ethanol and isopropanol; and wherein the water is present in an amount in the range of from about 0.1 wt/wt equiv. to about 0.6 wt/wt equiv.;
(b) heating the mixture to about reflux temperature;
(c) cooling the mixture to a temperature less than about room temperature; and
(d) isolating the di-threo-methylphenidate as a solid.

In other embodiments, the present invention is directed to a process for removing or reducing the amount of one or more of impurity compounds selected from the group consisting of
an impurity compound of formula (IMP-A)

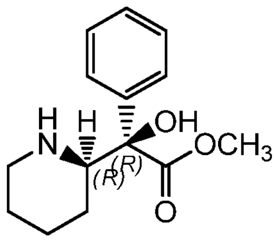

(IMP-A),

or a salt thereof;

an impurity compound of formula (IMP-B)

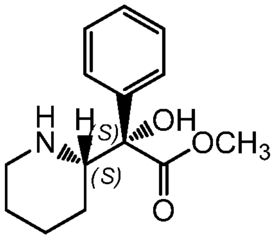

(IMP-B),

or a salt thereof;
an impurity compound of formula (IMP-C)

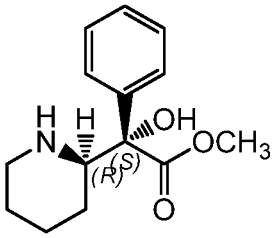

(IMP-C)

or a salt thereof;
and an impurity compound of formula (IMP-D)

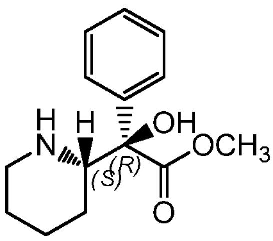

(IMP-D)

or a salt thereof; from a pharmaceutically acceptable salt of di-threo-methylphenidate;
comprising the steps of

1. providing a mixture of

    i. di-threo-methylphenidate sulfate;
    ii. an impurity compound selected from the group consisting of a sulfate salt of the impurity compound of formula (IMP-A), a sulfate salt of the impurity compound of formula (IMP-B), a sulfate salt of the impurity compound of formula (IMP-C), a sulfate salt of the impurity compound of formula (IMP-D), and mixtures thereof;

2. adding an organic solvent to the mixture of Step 1 to yield a second mixture; and
3. reacting the second mixture of Step 2 with an acid; in the presence of water; wherein the water is present in an amount in the range of from about 0.05 wt/wt equiv. to about 1.0 wt/wt equiv.;
to yield a product comprising a corresponding pharmaceutically acceptable salt of di-threo-methylphenidate in a mixture with a corresponding impurity compound selected from the group consisting of a corresponding pharmaceutically acceptable salt of the impurity compound of formula (IMP-A), a corresponding pharmaceutically acceptable salt of the impurity compound of formula (IMP-B), a corresponding pharmaceutically acceptable salt of the impurity compound of formula (IMP-C), a corresponding pharmaceutically acceptable salt of the impurity compound of formula (IMP-D), and mixtures thereof; and
4. optionally, separating (or removing) the water from the mixture of Step 3;

wherein the mixture of Step 1, is prepared according to a process comprising the steps of:

A. providing a composition comprising

i. di-threo-ritalinic acid or pharmaceutically acceptable salt thereof; and

ii. one or more of an impurity precursor compounds selected from the group consisting of an impurity compound of formula (IMP-1)

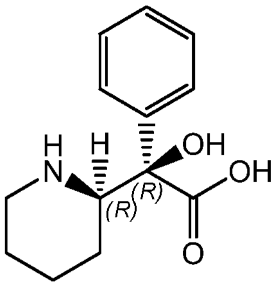

(IMP-1)

or a salt thereof, an impurity compound of formula (IMP-2)

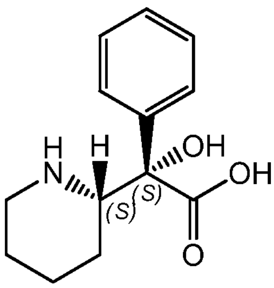

(IMP-2)

or a salt thereof, an impurity compound of formula (IMP-11)

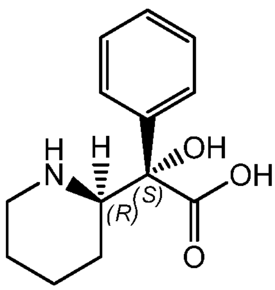

(IMP-11)

or a salt thereof, and an impurity compound of formula (IMP-12)

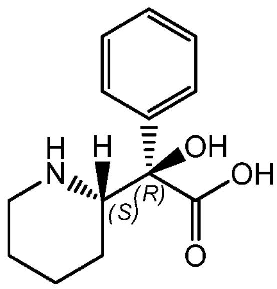

(IMP-12)

or a salt thereof;

B. reacting the composition of Step A with dimethylcarbonate, wherein the dimethylcarbonate is present in an amount

in the range of from about 2.5 to about 8.0 molar equivalents;
in the presence of methanol, wherein the methanol is present in an amount in the range of from about 0.75 to about 5.0 molar equivalents, wherein said methanol is added as a separate components, wherein the methanol is added in one or more portions or aliquots, and wherein the amount of methanol is independent of any methanol which may be later introduced into the reaction mixture;
in the presence of sulfuric acid, wherein the sulfuric acid is present in an amount in the range of from about 1.05 to about 2.5 molar equivalents;
at a temperature in the range of from about room temperature to about 90°C; to yield a mixture comprising:

i. di-threo-methylphenidate sulfate; and
ii. the impurity compound selected from the group consisting of a sulfate salt of the impurity compound of formula (IMP-A), a sulfate salt of the impurity compound of formula (IMP-B), a sulfate salt of the impurity compound of formula (IMP-C), a sulfate salt of the impurity compound of formula (IMP-D), and mixtures thereof.

In other embodiments, the present invention is directed to a process for removing or reducing the amount of one or more of an impurity compounds selected from the group consisting of
impurity compound of formula (IMP-A)

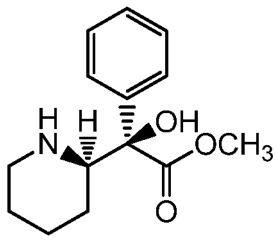

(IMP-A)

or a salt thereof, an impurity compound of formula (IMP-B)

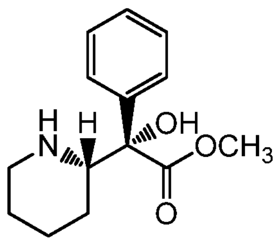

(IMP-B)

or a salt thereof, an impurity compound of formula (IMP-C)

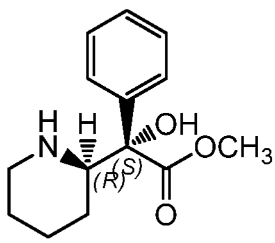

(IMP-C)

or salt thereof, and impurity compound of formula (IMP-D)

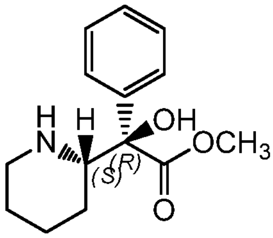

(IMP-D)

or a salt thereof; from di-threo-methylphenidate or a pharmaceutically acceptable salt of di-threo-methylphenidate; comprising the steps of

1. providing a mixture of

   i. di-threo-methylphenidate sulfate;
   ii. an impurity compound selected from the group consisting of the freebase impurity compound of formula (IMP-A), the freebase impurity compound of formula (IMP-B), and mixtures thereof;
   iii an organic phase comprising an organic solvent;

2. reacting the mixture of Step 1 with an acid; in the presence of water; wherein the water is present in an amount in the range of from about 0.05 wt/wt equiv. to about 1.0 wt/wt equiv.;
to yield a product comprising a corresponding pharmaceutically acceptable salt of di-threo-methylphenidate in a mixture with a corresponding impurity compound selected from the group consisting of a corresponding pharmaceutically acceptable salt of the impurity compound of formula (IMP-A), a corresponding pharmaceutically acceptable salt of the impurity compound of formula (IMP-B), a corresponding pharmaceutically acceptable salt of the impurity compound of formula (IMP-C), a corresponding pharmaceutically acceptable salt of the impurity compound of formula (IMP-D), and mixtures thereof; and 3. optionally, separating (or removing) the water from the mixture of Step 2;

wherein the mixture of Step 1, is prepared according to a process comprising the steps of:

A. providing a composition comprising

   a. di-threo-ritalinic acid or pharmaceutically acceptable salt thereof; and
   b. an impurity precursor selected from the group consisting of an impurity compound of formula (IMP-1)

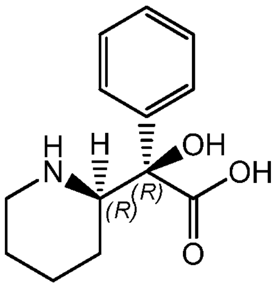

(IMP-1)

or a salt thereof, an impurity compound of formula (IMP-2)

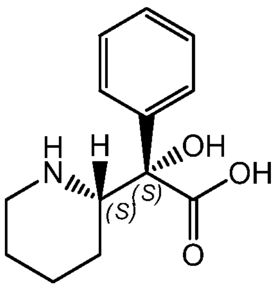

(IMP-2)

or a salt thereof, an impurity compound of formula (IMP-11)

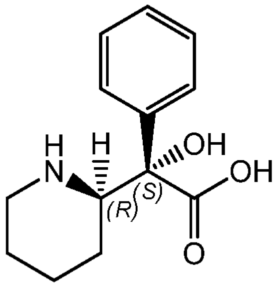

(IMP-11)

or a salt thereof, and an impurity compound of formula (IMP-12)

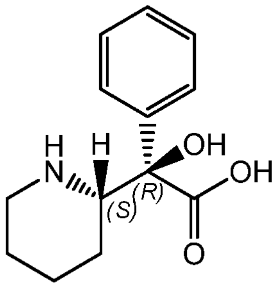

(IMP-12)

or a salt thereof, and mixtures thereof;

B. reacting the composition of Step A with dimethylcarbonate, wherein the dimethylcarbonate is present in an amount in the range of from about 2.5 to about 8.0 molar equivalents;
the presence of methanol, wherein the methanol is present in an amount in the range of from about 0.75 to about 5.0 molar equivalents, wherein said methanol is added as a separate components, wherein the methanol is added in one or more portions or aliquots, and wherein the amount of methanol is independent of any methanol which may be later introduced into the reaction mixture;
in the presence of sulfuric acid, wherein the sulfuric acid is present in an amount in the range of from about 1.05 to about 2.5 molar equivalents;
at a temperature in the range of from about room temperature to about 90°C; to yield a mixture comprising

a. di-threo-methylphenidate sulfate; and
b. an impurity compound selected from the group consisting of a sulfate salt of the impurity compound of formula (IMP-A), a sulfate salt of the impurity compound of formula (IMP-B), a sulfate salt of the impurity compound of formula (IMP-C), a sulfate salt of the impurity compound of formula (IMP-D), and mixtures thereof;

C. reacting the mixture prepared in Step B with a base; and adding an organic solvent; to yield a mixture of di-threo-methylphenidate free base and the impurity compound selected from the group consisting of the freebase impurity compound of formula (IMP-A), the freebase impurity compound of formula (IMP-B), the freebase impurity compound of formula (IMP-C), the freebase impurity compound of formula (IMP-D), and mixtures thereof; in a biphasic mixture comprising an aqueous phase and an organic phase;
D. separating the phases of the biphasic mixture one from the other; wherein the di-threo-methylphenidate free base, and the impurity compound selected from the group consisting of the freebase impurity compound of formula (IMP-A), the freebase impurity compound of formula (IMP-B), the freebase impurity compound of formula (IMP-C), the freebase impurity compound of formula (IMP-D), and mixtures thereof, are present in the organic phase.

In other embodiments, the present invention is directed to a process for removing or reducing the amount of an impurity compound of formulas (IMP-A)

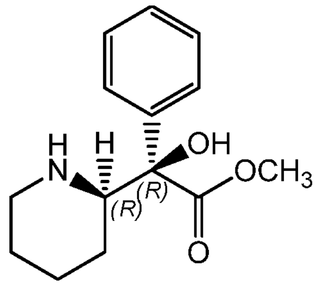

(IMP-A),

or a salt thereof, an impurity compound of formula (IMP-B),

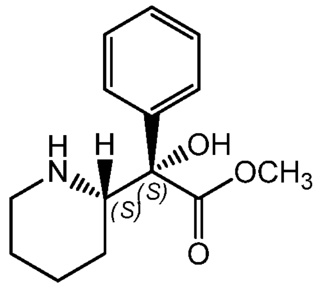

(IMP-B),

or a salt thereof, an impurity compound of formula (IMP-C)

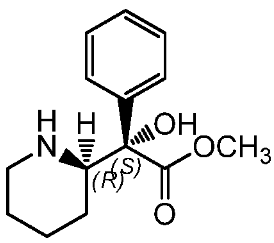

(IMP-C)

or a salt thereof, an impurity compound of formula (IMP-D)

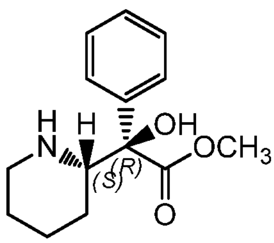

(IMP-D)

or a salt thereof, or mixtures thereoffrom a process of preparing a compound of formula (I)

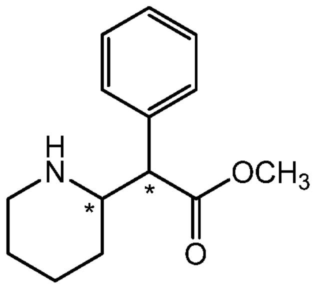

(I)

or a stereoisomer, mixture of stereoisomers or pharmaceutically acceptable salt thereof, comprising the steps of:

a. providing a composition comprising:

i. a compound of formula (I-A)

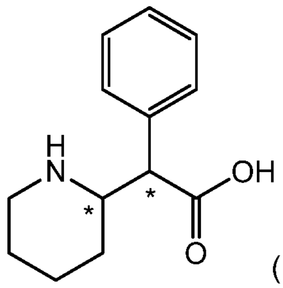

(I-A);

or stereoisomer, mixture of stereoisomers or pharmaceutically acceptable salt thereof; and
ii. a compound selected from the group consisting of an impurity compound of formula (IMP-1)

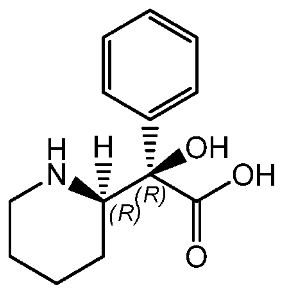

(IMP-1),

an impurity compound of formula (IMP-2)

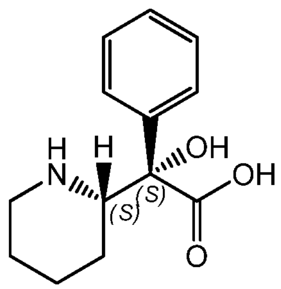

(IMP-2),

an impurity compound of formula (IMP-11)

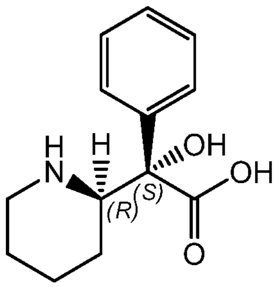

(IMP-11)

an impurity compound of formula (IMP-12)

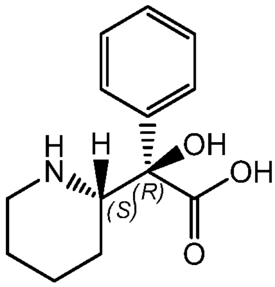

(IMP-12)

or mixtures thereof;

b. reacting the composition of Step a. with dimethylcarbonate to form a reaction mixture; wherein the dimethylcarbonate is present in an amount in the range of from about 2.5 to about 8.0 molar equivalents;
c. adding methanol to the reaction mixture; wherein the methanol is present in an amount in the range of from about 0.75 to about 5.0 molar equivalents; wherein said methanol is added as a separate component; and wherein the amount of methanol added, in one or more portions or aliquots, is independent of any methanol which may be later introduced into the reaction mixture;
d. adding sulfuric acid to the reaction mixture; wherein the sulfuric acid is present in an amount in the range of from about 1.05 to about 2.5 molar equivalents;
wherein the reaction mixture is at a temperature in the range of from about room temperature to about 90°C; to yield the corresponding compound of formula (I-S);

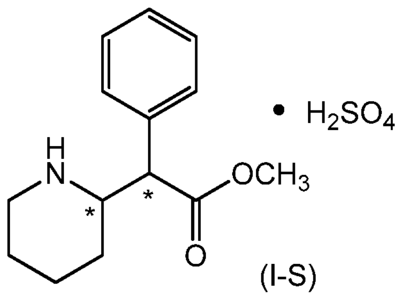

(I-S) ,

in a mixture with a corresponding sulfate salt of the impurity compound of formula (IMP-A), a corresponding sulfate salt of the impurity compound of formula (IMP-B), a corresponding sulfate salt of the impurity compound of formula (IMP-C), a corresponding sulfate salt of the impurity compound of formula (IMP-D), or mixtures thereof;
e. reacting the mixture of Step d. with a base; and then adding an organic solvent; to yield the corresponding compound of formula (I) as a free base, in a mixture with a freebase impurity compound of formula (IMP-A), a freebase impurity compound of formula (IMP-B), a freebase impurity compound of formula (IMP-C), a freebase impurity compound of formula (IMP-D), and mixtures thereof, in a biphasic mixture comprising an aqueous phase and an organic phase;
f. separating the phases of the biphasic mixture one from the other; wherein: i. the compound of formula (I) and ii. the freebase impurity compound of formula (IMP-A), the freebase impurity compound of formula (IMP-B), the freebase

impurity compound of formula (IMP-C), the freebase impurity compound of formula (IMP-D), and mixtures thereof, are present in the organic phase;

g. reacting an acid with the organic layer comprising: i. the compound of formula (I) and ii. the impurity compound of formula (IMP-A), the impurity compound of formula (IMP-B), the impurity compound of formula (IMP-C), the impurity compound of formula (IMP-D), and mixtures thereof; in the presence of water; wherein the water is present in an amount in the range of from about 0.05 wt/wt equiv. to about 1.0 wt/wt equiv.; to yield a corresponding acid addition salt of the compound of formula (I) in a mixture with a corresponding acid addition salt of the impurity compound of formula (IMP-A), a corresponding acid addition salt of the impurity compound of formula (IMP-B), a corresponding acid addition salt of the impurity compound of formula (IMP-C), a corresponding acid addition salt of the impurity compound of formula (IMP-D), and mixtures thereof wherein the corresponding acid addition salt of the impurity compound of formula (IMP-A), the corresponding acid addition salt of the impurity compound of formula (IMP-B), the corresponding acid addition salt of the impurity compound of formula (IMP-C), the corresponding acid addition salt of the impurity compound of formula (IMP-D), and mixtures thereof are extracted into the water; and

h. separating the water comprising the corresponding acid addition salt of the impurity compound of formula (IMP-A), the corresponding acid addition salt of the impurity compound of formula (IMP-B), the corresponding acid addition salt of the impurity compound of formula (IMP-C), the corresponding acid addition salt of the impurity compound of formula (IMP-D), and mixtures thereof from the corresponding acid addition salt of compound of formula (I) from the mixture of Step g.

In other embodiments, the present invention is directed to a process for removing or reducing the amount of a impurity compound of formula (IMP-A)

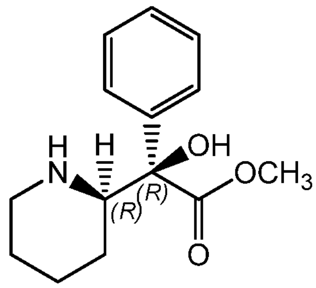

(IMP-A),

or a salt thereof, an impurity compound of formula (IMP-B)

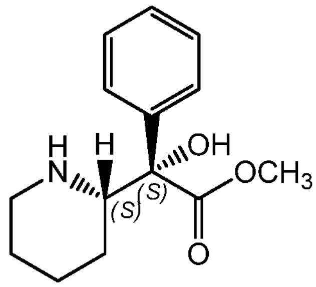

(IMP-B),

or a salt thereof, an impurity compound of formula (IMP-C)

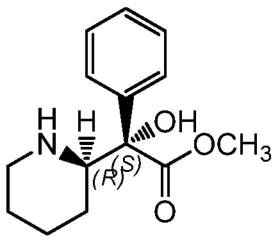

(IMP-C)

or a salt thereof, an impurity compound of formula (IMP-D)

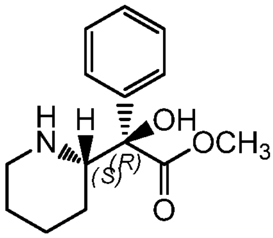

(IMP-D)

or a salt thereof, or mixtures thereof, from a process of preparing a compound of formula (I)

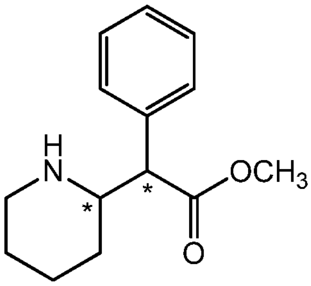

(I)

or a stereoisomer, mixture of stereoisomers or pharmaceutically acceptable salt thereof, comprising the steps of:

a. providing a composition comprising:

i. a compound of formula (I-A)

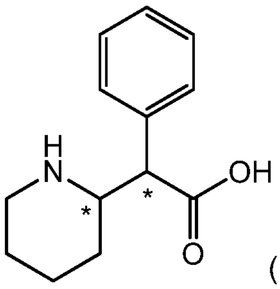

(I-A);

or stereoisomer, mixture of stereoisomers or pharmaceutically acceptable salt thereof; and
ii. an impurity compound selected from the group consisting of an impurity compound of formula (IMP-1),

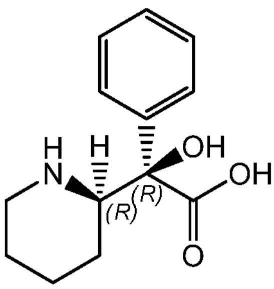

(IMP-1),

an impurity compound of formula (IMP-2),

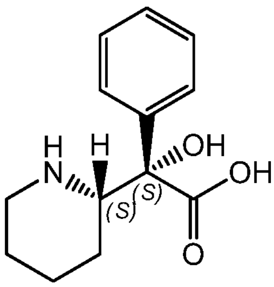

(IMP-2)

an impurity compound of formula (IMP-11)

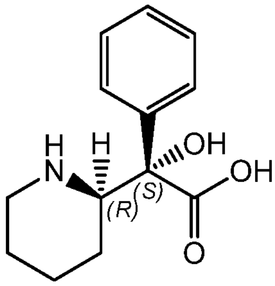

(IMP-11)

an impurity compound of formula (IMP-12)

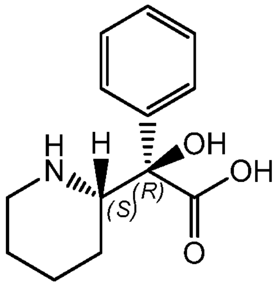

(IMP-12)

or mixtures thereof.

b. reacting the composition of Step a. with dimethylcarbonate to form a reaction mixture; wherein the dimethylcarbonate is present in an amount in the range of from about 2.5 to about 8.0 molar equivalents;
c. adding methanol to the reaction mixture; wherein the methanol is present in an amount in the range of from about 0.75 to about 5.0 molar equivalents; wherein said methanol is added as a separate component; and wherein the amount of methanol added, in one or more portions or aliquots, is independent of any methanol which may be later introduced into the reaction mixture;
d. adding sulfuric acid to the reaction mixture; wherein the sulfuric acid is present in an amount in the range of from about 1.05 to about 2.5 molar equivalents;
wherein the reaction mixture is at a temperature in the range of from about room temperature to about 90°C; to yield the corresponding compound of formula (I-S);

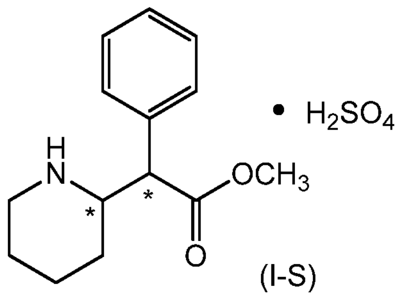

(I-S)

in a mixture with a corresponding sulfate salt of the impurity compound of formula (IMP-A), a corresponding sulfate salt of the impurity compound of formula (IMP-B), a corresponding sulfate salt of the impurity compound of formula (IMP-C), a corresponding sulfate salt of the impurity compound of formula (IMP-D), and mixtures thereof;

e. adding an organic solvent the mixture of Step d. to form a second mixture;

f. reacting the second mixture of Step e. with an acid; in the presence of water; wherein the water is present in an amount in the range of from about 0.05 wt/wt equiv. to about 1.0 wt/wt equiv.; to yield a corresponding acid addition salt of the compound of formula (I) in a mixture with a corresponding acid addition salt of the compound of formula (IMP-1), a corresponding acid addition salt of the compound of formula (IMP-2), a corresponding acid addition salt of the compound of formula (IMP-11), a corresponding acid addition salt of the compound of formula (IMP-12), and mixtures thereof wherein the corresponding acid addition salt of the impurity compound of formula (IMP-A), the corresponding acid addition salt of the impurity compound of formula (IMP-B), the corresponding acid addition salt of the impurity compound of formula (IMP-C), the corresponding acid addition salt of the impurity compound of formula (IMP-D), and mixtures thereof are extracted into the water; and

g. separating the water comprising a corresponding acid addition salt of the impurity compound of formula (IMP-1), a corresponding acid addition salt of the impurity compound of formula (IMP2), a corresponding acid addition salt of the impurity compound of formula (IMP-11), a corresponding acid addition salt of the impurity compound of formula (IMP-12), and mixtures thereof from the corresponding acid addition salt of compound of formula (I) from the mixture of Step f.

In certain embodiments, the present invention is directed to processes for the preparation of a compound of formula (I), a compound of formula (Ia), and stereoisomers or mixtures of stereoisomers thereof, wherein one or more process parameter(s) (for example, the amount of water added during any step of the process) are varied or controlled to yield the desired product as a solid, optionally a crystalline solid.

In additional embodiments, the compound of formula (I), compound of formula (Ia) or stereoisomer or mixture of stereoisomers thereof (for example di-threo-methylphenidate, di-threo-methylphenidate hydrochloride, dex-methylphenidate or dex-methylphenidate hydrochloride) is isolated as a solid, optionally a crystalline solid, in a form which exhibits a particle size distribution meeting the following specifications (when measured using an air jet sieve method): (a) about 51% to about 86% retain on a 325 mesh sieve, (b) about 35% to about 75% retain on a 200 mesh sieve, (c) less than about 30% retain on a 80 mesh sieve, and (d) less than about 1% retain on an 20 mesh sieve.

In another embodiment, the solid, optionally crystalline solid, is isolated in a form which exhibits a particle size distribution meeting the following specifications (when measured using an air jet sieve method): (a) about 65% to about 95% retain on a 325 mesh sieve, (b) about 50% to about 85% retain on a 200 mesh sieve, (c) less than about 20% retain on a 80 mesh sieve, and (d) less than about 1% retain on an 20 mesh sieve.

In another embodiment, the solid, optionally crystalline solid, is isolated in a form which exhibits a particle size distribution meeting the following specifications (when measured using an air jet sieve method): (a) greater than 86% retain on a 325 mesh sieve, (b) greater than about 75% retain on a 200 mesh sieve, (c) less than about 30% retain on a 80 mesh sieve, and (d) less than about 1% retain on an 20 mesh sieve.

In other embodiments, the present invention is directed to a product prepared according to any of the processes described herein.

Illustrative of the invention is a pharmaceutical composition comprising a pharmaceutically acceptable carrier and a product prepared according to any of the processes described herein. An illustration of the invention is a pharmaceutical composition made by mixing a product prepared according to any of the processes described herein and a pharmaceutically acceptable carrier. Further illustrating the present invention is a process for making a pharmaceutical composition comprising mixing a product prepared according to any of the processes and a pharmaceutically acceptable carrier.

Exemplifying the methods of using the compounds and / or compositions of present invention are methods of treating central nervous system disorders including, but not limited to, narcolepsy or attention deficit disorder (also known

as Minimal Brain Dysfunction in Children, Hyperkinetic Child Syndrome, Minimal Brain Damage, Minimal Cerebral Dysfunction or Minor Cerebral Dysfunction) comprising administering to a subject in need thereof a therapeutically effective amount of any of the products or pharmaceutical compositions described above.

In an embodiment, the present invention is directed to a product prepared according to any of the processes described herein for use as a medicament. In another embodiment, the present invention is directed to a product prepared according to any of the processes described herein for use in the treatment of central nervous system disorders including, but not limited to, narcolepsy or attention deficit disorder.

Another example of the invention is the use of a product prepared according to any of the processes described herein in the preparation of a medicament for treating central nervous system disorders including, but not limited to: (a) attention deficit disorder, or (b) narcolepsy, in a subject in need thereof. In another example, the present invention is directed to a compound as described herein for use in a methods for treating central nervous system disorders including, but not limited to, disorders selected from attention deficit disorder and narcolepsy, in a subject in need thereof.

## DETAILED DESCRIPTION OF THE INVENTION

The processes of the present invention can comprise, consist of, or consist essentially of the steps, essential elements and limitations of the invention described herein, as well any of the additional or optional ingredients, components, or limitations described herein. The term "comprising" (and its grammatical variations) as used herein is used in the inclusive sense of "having" or "including" and not in the exclusive sense of "consisting only of."

The terms "a" and "the" as used herein are understood to encompass the plural as well as the singular.

As used herein, unless otherwise noted, the term "**$C_{1-4}$alkyl**" shall include straight and branched chain compositions of between 1 and 4 carbon atoms including methyl, ethyl, n-propyl, isopropyl, n-butyl, isobutyl and t-butyl. In an embodiment, the $C_{1-4}$alkyl is selected from the group consisting of methyl, ethyl and isopropyl.

In an embodiment, the present invention is directed to an improved process for the preparation of a compound of formula (I)

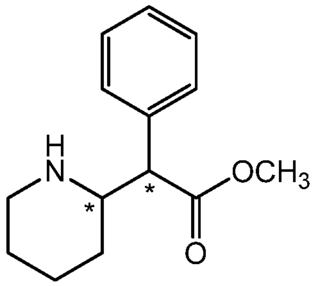

(I)

also known as methyl 2-phenyl-2-(piperidin-2-yl)acetate or methylphenidate, or a stereoisomer, a mixture of stereoisomers or a pharmaceutically acceptable salt thereof, optionally an HCl salt, optionally an HCl salt of the di-threo mixture of isomers, optionally an HCl salt of the dex-isomer, as described in more detail herein.

In certain embodiments, the process of the present invention is particularly advantageous for large scale, commercial and / or continuous manufacture of the compound of formula (I), optionally the di-threo mixture of stereoisomers of the compound of formula (I) or the dex-isomer of the compound of formula (I). In one embodiment, the process of the present invention is advantageous with respect to the reduced amount of solvent used in the preparation of the compound of formula (I), resulting in easier handling and higher throughput (more product per unit volume of the product stream). In another embodiment, the process of the present invention yields a product stream (e.g. the organic phase/layer of a biphasic mixture) wherein the product stream contains the desired product, substantially free of unreacted starting material(s) and by-products. Thus, the process of the present invention advantageously results in a product which is substantially pure, eliminating the need for additional purification steps.

In an embodiment, the present invention is directed to a process for the preparation of a compound of formula (I) or a stereoisomer, a mixture of stereoisomers or a pharmaceutically acceptable salt thereof, optionally an HCl salt, optionally an HCl salt of a di-threo mixture of stereoisomers of the compound of formula (I) or an HCl salt of the dex-isomer of the compound of formula (I), as herein described, wherein the compound of formula (I) or a stereoisomer, a mixture of stereoisomers or a pharmaceutically acceptable salt thereof is prepared with a calculated yield of greater than about 60%, optionally with a calculated yield of greater than about 70%, optionally with a calculated yield of greater than about 75%, optionally with a calculated yield of greater than about 80%, optionally with a calculated yield of greater than about 85%, optionally with a calculated yield of greater than about 90%, optionally still with a calculated yield of greater than about 95 %, most optionally with a calculated yield of greater than about 97%.

In an embodiment, the present invention is directed to a process for the preparation of a compound of formula (I) or a stereoisomer, a mixture of stereoisomers or a pharmaceutically acceptable salt thereof, optionally an HCl salt, optionally an HCl salt of the di-threo mixture of stereoisomers of the compound of formula (I) or an HCl salt of the dex-isomer of the compound of formula (I), as herein described, wherein the compound of formula (I) or a stereoisomer, a mixture of stereoisomers or a pharmaceutically acceptable salt thereof is prepared with a purity of greater than about 60%, optionally greater than about 70%, optionally greater than about 75%, optionally greater than about 80%, optionally greater than about 85%, optionally greater than about 90%, optionally still greater than about 95%, most optionally greater than about 97%, as measured for example, by HPLC, LCMS or other suitable method.

One skilled in the art will recognize that the compound of formula (I) contains two stereogenic centers (or, stereo-centers), as denoted herein by the star ("*"). Thus, the compound of formula (I) may exist in four diastereomeric forms. More particularly, the compound of formula (I) exists in two erythro-forms

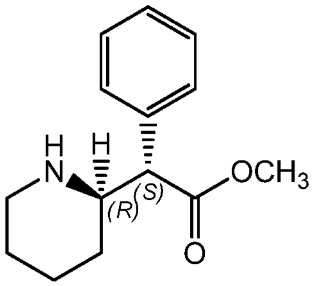

(2S, 2R)-erythro-methylphenidate

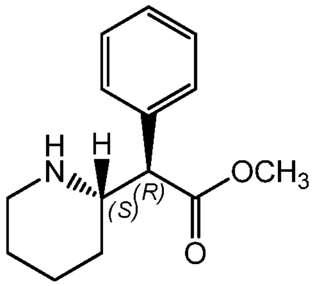

(2R, 2S)-erythro-methylphenidate

and two threo-forms:

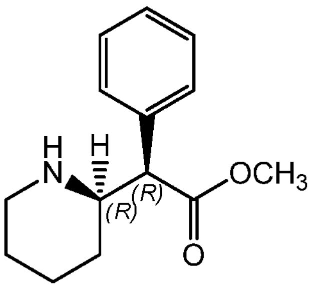

(2R, 2R)-threo-methylphenidate
also known as dex-methylphenidate

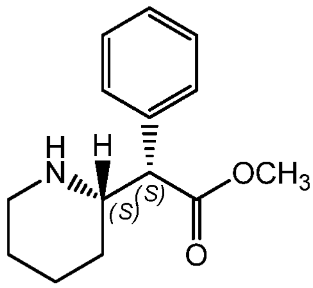

(2S, 2S)-threo-methylphenidate

As used herein, unless otherwise noted, the terms "**di-threo-ritalinic acid**" and "**di-threo-2-phenyl-2-(piperidin-2-yl)acetic acid**" shall mean a 50 / 50 mixture (or about 50 / 50 mixture) of (2R, 2R)-threo-ritalinic acid and (2S, 2S)-threo-ritalinic acid. As used herein, unless otherwise noted, the term **"dex-ritalinic acid"** shall mean (2R,2R)-ritalinic acid diastereomer.

As used herein, unless otherwise noted, the terms "**di-erythro-ritalinic acid**" and "**di-erythro-2-phenyl-2-(piperidin-2-yl)acetic acid**" shall mean a 50 /50 mixture (or about 50 / 50 mixture) of (2S, 2R)-threo-ritalinic acid and (2R, 2S)-threo-ritalinic acid.

As used herein, unless otherwise noted, the term **"di-threo-methylphenidate"** shall mean a 50 / 50 mixture (or about 50 / 50 mixture) of (2R,2R)-threo-methylphenidate and (2S,2S)-threo-methylphenidate. As used herein, unless otherwise noted, the term **"dex-methylphenidate"** shall mean (2R,2R)-threo-methylphenidate diastereomer.

As used herein, unless otherwise noted, the term **"di-erythro-methylphenidate"** shall mean a 50 / 50 mixture (or about 50 / 50 mixture) of (2S, 2R)-erythro-methylphenidate and (2R, 2S)-erythro-methylphenidate.

In an embodiment, the present invention is directed to a process for the preparation of a compound of formula (I), wherein the compound of formula (I) is di-threo-methylphenidate. In another embodiment, the present invention is directed to a process for the preparation of a compound of formula (I), wherein the compound of formula (I) is (2R,2R)-threo-methylphenidate (also known as dex-methylphenidate). In another embodiment, the present invention is directed to a process for the preparation of a compound of formula (I), wherein the compound of formula (I) is (2S,2S)-threo-methylphenidate. In another embodiment, the present invention is directed to a process for the preparation of a mixture

(e.g. a racemic mixture) of a compound of formula (I), wherein the compound of formula (I) is a mixture of (2R, 2R)-threo-methylphenidate and (2S, 2S)-threo-methylphenidate.

In an embodiment, the present invention is directed to a process for the preparation of a compound of formula (I), wherein the compound of formula (I) is a mixture of di-erythro-methylphenidate and di-threo-methylphenidate.

In an embodiment, the present invention is directed to process for the preparation of a compound of formula (I), optionally di-threo-methylphenidate, optionally dex-methylphenidate, optionally an HCl salt of di-threo-methylphenidate, optionally an HCl salt of dex-methylphenidate, wherein the product (i.e. the compound of formula (I)) is prepared as a crystalline form.

Although not intended to be authoritative as to the identity of any impurities which may be present and without being bound by any proposed theory, it is theorized that precursor impurity compounds of formula (IMP-1), formula (IMP-2), formula (IMP-11) and formula (IMP-12)

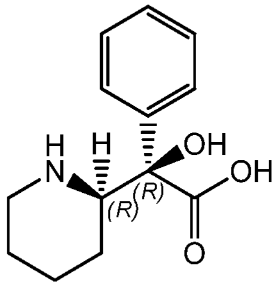

(IMP-1)

(R)-2-hydroxy-2-phenyl-2-((R)-piperidin-2-yl)acetic acid

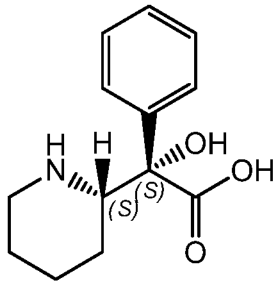

(IMP-2)

(S)-2-hydroxy-2-phenyl-2-((S)-piperidin-2-yl)acetic acid

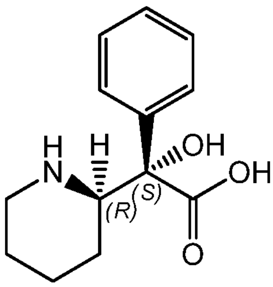

(IMP-11)

(S)-2-hydroxy-2-phenyl-2-((R)-piperidin-2-yl)acetic acid

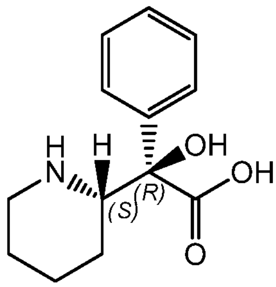

(IMP-12)

(R)-2-hydroxy-2-phenyl-2-((S)-piperidin-2-yl)acetic acid

may be present as by-products of the synthesis of di-threo-ritalinic acid. The precursor impurity compounds of formula (IMP-1), formula (IMP-2), formula (IMP-11) and formula (IMP-12) may be present in the di-threo-ritalinic acid starting material, for example in an amount of less than about 0.5 area% (as measured by HPLC), optionally in an amount of less than about 0.2 area%, optionally in an amount less than about 0.15 area %, optionally in an amount less than about 0.1 area %, optionally in an amount less than about 0.05 area %.

As qualified above, it is further theorized that in the process(es) of the present invention, the precursor impurity compounds of formula (IMP-1), formula (IMP-2), formula (IMP-11) and formula (IMP-12) undergo the same chemical transformations as the di-threo-ritalinic acid and di-threo-methylphenidate (as described in more detail hereinafter) such that when a mixture of di-threo ritalinic acid, and one or more of the impurity compounds of formula (IMP-1), formula (IMP-2), formula (IMP-11) and formula (IMP-12) is reacted with dimethylcarbonate, in the presence of methanol, in the presence of sulfuric acid, as described herein, the resulting product comprises a mixture of the di-threo-methylphenidate sulfate salt, and the corresponding sulfate salt impurity compounds of formula (IMP-3), formula (IMP-4), formula (IMP-13) and formula (IMP-15).

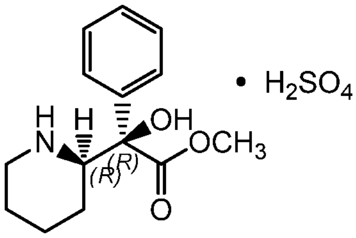

(IMP-3)

(R)-methyl 2-hydroxy-2-phenyl-2-((R)-piperidin-2-yl)acetate sulfate

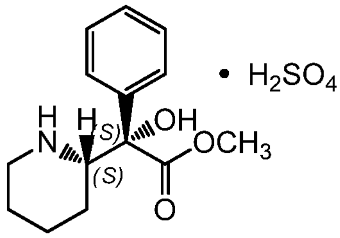

(IMP-4)

(S)-methyl 2-hydroxy-2-phenyl-2-((S)-piperidin-2-yl)acetate sulfate.

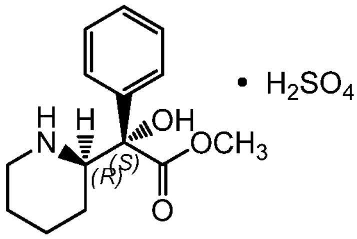

(IMP-13)

(S)-methyl 2-hydroxy-2-phenyl-2-((R)-piperidin-2-yl)acetate sulfate

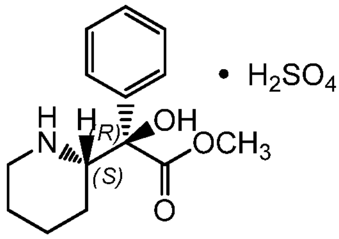

(IMP-14)

(R)-methyl 2-hydroxy-2-phenyl-2-((S)-piperidin-2-yl)acetate sulfate

As qualified above, it is further theorized that in the process(es) of the present invention, for example, in the preparation of di-threo-methylphenidate hydrochloride, as described in more detail hereinafter, the precursor impurity compounds of formula (IMP-1), formula (IMP-2), formula (IMP-11) and formula (IMP-12) (present in a mixture with di-threo-ritalinic acid starting material) are converted (under the reaction conditions described herein) to yield the corresponding impurity compounds of formula (IMP-5), formula (IMP-6), formula (IMP-15) and formula (IMP-16), wherein said compounds are the hydrochloride salts of impurity compounds of formulas (IMP-A) and (IMP-B), respectively.

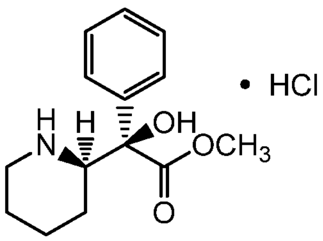

(IMP-5)

(R)-methyl 2-hydroxy-2-phenyl-2-((R)-piperidin-2-yl)acetate hydrochloride

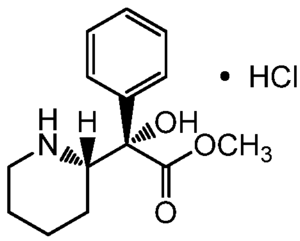

(IMP-6)

(S)-methyl 2-hydroxy-2-phenyl-2-((S)-piperidin-2-yl)acetate hydrochloride.

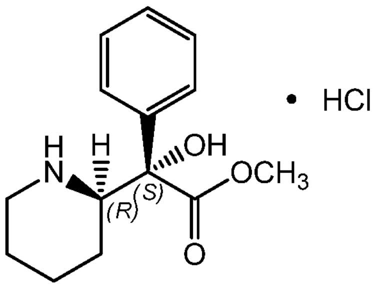

(IMP-15)

(S)-methyl 2-hydroxy-2-phenyl-2-((R)-piperidin-2-yl)acetate hydrochloride

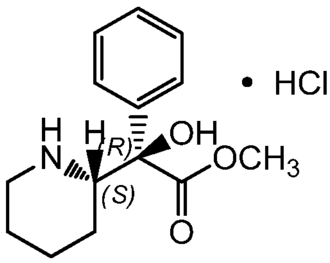

(IMP-16)

(R)-methyl 2-hydroxy-2-phenyl-2-((S)-piperidin-2-yl)acetate hydrochloride

As qualified above, it is further theorized that wherein the final di-threo methylphenidate is converted to its corresponding freebase, then the precursor impurity compounds of formula (IMP-1), formula (IMP-2), formula (IMP-11) and formula (IMP-12) are converted to the corresponding impurity compounds of formula (IMP-A), formula (IMP-B), formula (IMP-C) and formula (IMP-D).

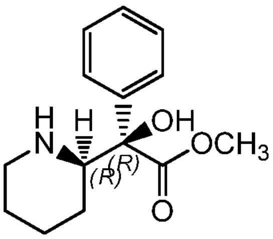

(IMP-A)

(R)-methyl 2-hydroxy-2-phenyl-2-((R)-piperidin-2-yl)acetate

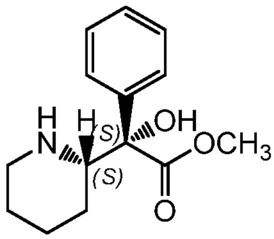

(IMP-B)

(S)-methyl 2-hydroxy-2-phenyl-2-((S)-piperidin-2-yl)acetate

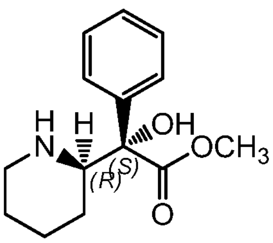

(IMP-C)

(S)-methyl 2-hydroxy-2-phenyl-2-((R)-piperidin-2-yl)acetate

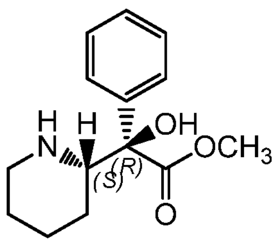

(IMP-D)

(R)-methyl 2-hydroxy-2-phenyl-2-((S)-piperidin-2-yl)acetate

As qualified above, it is further theorized that wherein the final di-threo methylphenidate is converted to its corresponding salt, optionally its corresponding pharmaceutically acceptable salt, then the precursor impurity compounds of formula (IMP-1), formula (IMP-2), formula (IMP-11) and formula (IMP-12) are converted to the corresponding salts of the impurity compounds of formula (IMP-A), formula (IMP-B), formula (IMP-C) and formula (IMP-D).

In an embodiment, the present invention is directed to a process for the preparation of di-threo-methylphenidate, comprising the step of providing a di-threo-ritalinic acid (as the starting material) which comprises less than about 0.2 % wt/wt of the impurity compound of formula (IMP-1), the impurity compound of formula (IMP-2), the impurity compound of formula (IMP-11), the impurity compound of formula (IMP-12) or mixtures thereof. In another embodiment, the present invention is directed to a process for the preparation of di-threo-methylphenidate, comprising the step of providing a di-threo-ritalinic acid (as the starting material) which comprises less than about 0.1 % wt/wt of the impurity compound of formula (IMP-1), the impurity compound of formula (IMP-2), the impurity compound of formula (IMP-11), the impurity

compound of formula (IMP-12), or mixtures thereof. In another embodiment, the present invention is directed to a process for the preparation of di-threo-methylphenidate, comprising the step of providing a di-threo-ritalinic acid (as the starting material) which comprises less than about 0.05 % wt/wt of the impurity compound of formula (IMP-1), the impurity compound of formula (IMP-2), the impurity compound of formula (IMP-11), the impurity compound of formula (IMP-12), or mixtures thereof.

In an embodiment, the present invention is directed to a process for the preparation of di-threo-methylphenidate or pharmaceutically acceptable salt thereof, wherein the final di-threo-methylphenidate or pharmaceutically acceptable salt thereof comprises less than about 0.2 %wt/wt, optionally less than about 0.15% wt/wt, optionally less than about 0.1%wt/wt, optionally in an amount less than about 0.05 %wt/wt, of an impurity compound selected from the compounds (or the group of compounds consisting) of formula (IMP-5), formula (IMP-6), formula (IMP-15), formula (IMP-16), formula (IMP-A), formula (IMP-B), formula (IMP-C), formula (IMP-D), and salts of the compounds of formulas (IMP-A), formula (IMP-B), formula (IMP-C) and formula (IMP-D).

In an embodiment, the present invention is directed to a product prepared according to any of the processes described herein. In another embodiment, the present invention is directed to a product prepared according to a process as described herein, wherein the product is selected from the group consisting of di-threo-methylphenidate, di-threo-methylphenidate hydrochloride, dex-methylphenidate and dex-methylphenidate hydrochloride. In another embodiment, the present invention is directed to a product prepared according to a process as described herein, wherein the product is selected from the group consisting of di-threo-methylphenidate, di-threo-methylphenidate hydrochloride, dex-methylphenidate and dex-methylphenidate hydrochloride, optionally the product is selected from the group consisting of di-threo-methylphenidate and di-threo-methylphenidate hydrochloride, or optionally the product is selected from the group consisting of dex-methylphenidate and dex-methylphenidate hydrochloride; and wherein, in each case, the product comprises a total of less than about 0.1 area % of an impurity compound selected from the compounds (or the group of compounds consisting) of formula (IMP-5), formula (IMP-6), formula (IMP-15), formula (IMP-16), formula (IMP-A), formula (IMP-B), formula (IMP-C), formula (IMP-D) and salts of the compounds of formulas (IMP-A), formula (IMP-B), formula (IMP-C) and formula (IMP-D), optionally, a total of less than about 0.075 area %, optionally, a total of less than about 0.05 area % as determined by HPLC as described herein.

Where the compounds of the present invention have at least one **chiral center,** they may accordingly exist as enantiomers. Where the compounds possess two or more chiral centers, they may additionally exist as diastereomers. It is to be understood that all such isomers and mixtures thereof are encompassed within the scope of the present invention. Optionally, wherein the compound is present as an enantiomer, the enantiomer is present at an enantiomeric excess of greater than or equal to about 80%, optionally, at an enantiomeric excess of greater than or equal to about 90%, optionally still, at an enantiomeric excess of greater than or equal to about 95%, optionally still, at an enantiomeric excess of greater than or equal to about 98%, optionally, at an enantiomeric excess of greater than or equal to about 99%. Similarly, in those embodiments wherein the compound is present as a diastereomer, the diastereomer is optionally present at an diastereomeric excess of greater than or equal to about 80%, optionally, at an diastereomeric excess of greater than or equal to about 90%, optionally still, at an diastereomeric excess of greater than or equal to about 95%, optionally still, at an diastereomeric excess of greater than or equal to about 98%, optionally, at an diastereomeric excess of greater than or equal to about 99%.

Furthermore, some of the crystalline forms for the compounds of the present invention may optionally exist as polymorphs and as such are intended to be included in the present invention. In addition, some of the compounds of the present invention may optionally form solvates with water (i.e., hydrates) or common organic solvents, and such solvates are also intended to be encompassed within the scope of this invention.

It is further intended that within the scope of the present invention, any element, in particular when mentioned in relation to a compound of formula (I), shall optionally comprise all isotopes and isotopic mixtures of said element, either naturally occurring or synthetically produced, either with natural abundance or in an isotopically enriched form. For example, a reference to hydrogen optionally includes within its scope $^{1}H$, $^{2}H$ (D), and $^{3}H$ (T). Similarly, references to carbon and oxygen optionally include within their scope respectively $^{12}C$, $^{13}C$ and $^{14}C$ and $^{16}O$ and $^{18}O$. The isotopes may be radioactive or non-radioactive. Radio-labeled compounds of formula (I) may optionally comprise a radioactive isotope selected from the group of $^{3}H$, $^{11}C$, $^{18}F$, $^{122}I$, $^{123}I$, $^{125}I$, $^{131}I$, $^{75}Br$, $^{76}Br$, $^{77}Br$ and $^{82}Br$. Optionally, the radioactive isotope is selected from the group of $^{3}H$, $^{11}C$ and $^{18}F$.

Abbreviations used in the specification, particularly the Schemes and Examples, are as follows:

| | | |
|---|---|---|
| DMC | = | Dimethylcarbonate |
| IPA | = | Isopropanol |
| IpOAc | = | Isopropyl Acetate |
| HPLC | = | High Performance Liquid Chromatography |
| MeOH | = | Methanol |

(continued)

| | | |
|---|---|---|
| MPH or MPD | = | Methylphenidate |
| OSA | = | Sodium 1-Octanesulfonate |
| TEA | = | Triethylamine |

As used herein, unless otherwise noted, the term **"isolated form"** shall mean that the compound is present in a form which is separate from any solid mixture with another compound(s), solvent system or biological environment. In an embodiment of the present invention, the product prepared according to any of the processes described herein (for example, di-threo-methylphenidate, dex-methylphenidate, an HCI salt of di-threo-methylphenidate or an HCI salt of dex-methylphenidate) is present in an isolated form.

As used herein, unless otherwise noted, the term **"substantially pure"** shall mean that the mole percent of impurities in the isolated compound is less than about 5 mole percent, optionally less than about 2 mole percent, optionally, less than about 0.5 mole percent, or optionally, less than about 0.1 mole percent. In an embodiment of the present invention the product prepared according to any of the processes described herein (for example, di-threo-methylphenidate, dex-methylphenidate, an HCI salt of di-threo-methylphenidate or an HCI salt of dex-methylphenidate) is present as a substantially pure form.

As used herein, unless otherwise noted, the term **"substantially free of unreacted starting material(s) and by-products"** when referring to a product sample or stream shall mean that the mole percent of unreacted starting materials and (undesired) by-products present in the product sample or product is less than about 10 mole percent, optionally less than about 5 mole percent, optionally less than about 2 mole percent, optionally, less than about 1 mole percent.

As used herein, unless otherwise noted, the term **"substantially free of a corresponding salt form(s)"** when used to described the compound of formula (I) shall mean that mole percent of the corresponding salt form(s) in the isolated base of formula (I) is less than about 5 mole percent, optionally less than about 2 mole percent, optionally, less than about 0.5 mole percent, or optionally less than about 0.1 mole percent. In an embodiment of the present invention, the product prepared according to any of the processes described herein (for example, di-threo-methylphenidate, dex-methylphenidate, an HCI salt of di-threo-methylphenidate or an HCI salt of dex-methylphenidate) is present in a form which is substantially free of corresponding salt form(s).

As used herein, unless otherwise noted, the terms **"treating", "treatment"** and the like, shall include the management and care of a subject or patient (optionally mammal, optionally human) for the purpose of combating a disease, condition, or disorder and includes the administration of a compound of the present invention to prevent the onset of the symptoms or complications, alleviate the symptoms or complications, or eliminate the disease, condition, or disorder.

As used herein, unless otherwise noted, the term **"prevention"** shall include (a) reduction in the frequency of one or more symptoms; (b) reduction in the severity of one or more symptoms; (c) the delay or avoidance of the development of additional symptoms; and / or (d) delay or avoidance of the development of a disorder or condition.

One skilled in the art will recognize that wherein the present invention is directed to methods of prevention, a subject in need of thereof (i.e. a subject in need of prevention) shall include any subject or patient (optionally a mammal, optionally a human) who has experienced or exhibited at least one symptom of the disorder, disease or condition to be prevented. Further, a subject in need thereof may additionally be a subject (optionally a mammal, optionally a human) who has not exhibited any symptoms of the disorder, disease or condition to be prevented, but who has been deemed by a physician, clinician or other medical profession to be at risk of developing said disorder, disease or condition. For example, the subject may be deemed at risk of developing a disorder, disease or condition (and therefore in need of prevention or preventive treatment) as a consequence of the subject's medical history, including, but not limited to, family history, pre-disposition, co-existing (comorbid) disorders or conditions, genetic testing, and the like.

The term **"subject"** as used herein, refers to an animal, optionally a mammal, most optionally a human, who has been the object of treatment, observation or experiment. Optionally, the subject has experienced and / or exhibited at least one symptom of the disease or disorder to be treated and / or prevented.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term **"composition"** means the proportion and/or combination of elements, chemicals, compounds, components and/or ingredients, whether active, inactive, or both, and whether chemical and/or non-chemical, that are employed to form a substance or material that is liquid or semi-liquid, semi-solid or solid by, for example, combining, mixing, blending, or the like, such as a tablet or solution containing a product made as described here.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

As more extensively provided in this written description, terms such as **"reacting"** and **"reacted"** are used herein in reference to a chemical entity that is any one of: (a) the actually recited form of such chemical entity, and (b) any of the forms of such chemical entity in the medium in which the compound is being considered when named.

One skilled in the art will recognize that, where not otherwise specified, the reaction step(s) is performed under suitable conditions, according to known methods, to provide the desired product. One skilled in the art will further recognize that, in the specification and claims as presented herein, wherein a reagent or reagent class/type (e.g. base, solvent, etc.) is recited in more than one step of a process, the individual reagents are independently selected for each reaction step and may be the same or different from each other. For example wherein two steps of a process recite an organic or inorganic base as a reagent, the organic or inorganic base selected for the first step may be the same or different than the organic or inorganic base of the second step.

One skilled in the art will recognize that wherein a reaction step of the present invention may be carried out in a variety of solvents or solvent systems, said reaction step may also be carried out in a mixture of the suitable solvents or solvent systems. One skilled in the art will further recognize that where two consecutive reaction or process steps are run without isolation of the intermediate product (i.e. the product of the first of the two consecutive reaction or process steps), then the first and second reaction or process steps may be run in the same solvent or solvent system; or alternatively may be run in different solvents or solvent systems following solvent exchange, which may be completed according to known methods.

Examples of suitable solvents, bases, reaction temperatures, and other reaction parameters and components are provided in the detailed description which follows herein. One skilled in the art will recognize that the listing of said examples is not intended, and should not be construed, as limiting in any way the invention set forth in the claims which follow thereafter.

As used herein, **"reflux temperature"** means the temperature at which the reaction mixtures described herein refluxes or boils at atmospheric pressure.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

Additionally, chiral HPLC against a standard may be used to determine percent enantiomeric excess (%ee). The enantiomeric excess may be calculated as follows

$$[\ (\text{Rmoles-Smoles})/(\text{Rmoles+Smoles})\ ] \times 100\%$$

where Rmoles and Smoles are the R and S mole fractions in the mixture such that Rmoles+Smoles = 1. The enantiomeric excess may alternatively be calculated from the specific rotations of the desired enantiomer and the prepared mixture as follows:

$$ee = ([\alpha\text{-obs}]\ /\ [\alpha\text{-max}]) \times 100.$$

Application of the present invention to, for example, the di-threo-ritalinic acid stereoisomers, free of or substantially free of the di-erythro-ritalinic acid stereoisomers, will result in the production of the corresponding di-threo-methylphenidate stereoisomers, free of or substantially free of the di-erythro-methylphenidate stereoisomers. Application of the present invention to for example, the di-erythro-ritalinic acid stereoisomers, free of or substantially free of the di-threo-ritalinic acid stereoisomers, will result in the production of the corresponding di-erythro-methylphenidate stereoisomers, free of or substantially free of the di-threo-methylphenidate stereoisomers. Thus one skilled in the art will recognize that the process of the present invention result in no significant or measurable amount of racemization of the stereocenters.

Application of the present invention to, for example, the dex-ritalinic acid stereoisomer, free of or substantially free of other diastereomers or mixtures of diastereomers of ritalinic acid, will result in the production of the corresponding dex-methylphenidate, free of or substantially free of other diastereomers or mixtures of diastereomers of methylphenidate.

Except for the salts of the impurity compounds, the salts of the compounds prepared in accordance with the processes of the present invention are pharmaceutically acceptable salts. In certain embodiments, however, "salts" as used herein with reference to the impurity compounds also means "pharmaceutically acceptable salts". As used herein, "pharmaceutically-acceptable" means that which is useful in preparing a pharmaceutical compound or composition that is generally safe, non-toxic and neither biologically nor otherwise undesirable and includes that which is acceptable for veterinary use as well as human pharmaceutical use. Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include, but are not limited to, the following: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids which may be used in the preparation of pharmaceutically acceptable salts include, but are not limited to, the following: acids including acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucoronic acid, L-glutamic acid, $\alpha$-oxo-glutaric acid, glycolic acid, hipuric acid, hydrobromic acid, hydrochloric acid, (+)-L-lactic acid, ($\pm$)-DL-lactic acid, lactobionic acid, maleic acid, (-)-L-malic acid, malonic acid, ($\pm$)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitic acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid.

Representative bases which may be used in the preparation of pharmaceutically acceptable salts include, but are not limited to, the following: bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

One skilled in the art will recognize that as used herein the term "or pharmaceutically acceptable salt thereof" shall include stereoisomers or mixtures of stereoisomers of said pharmaceutically acceptable salt.

Synthesis Processes Details

The present invention is directed to a process for the preparation of compounds of formula (I) as, for example, depicted in Scheme 1 below.

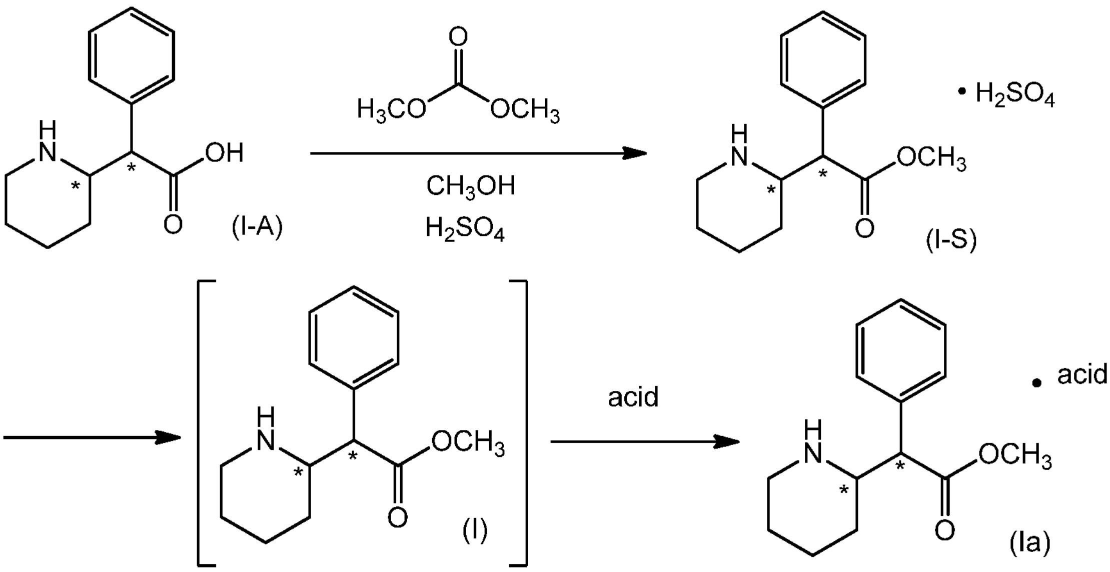


Scheme 1

STEP A:

Accordingly, in an embodiment, a compound of formula (I-A) (i.e. 2-phenyl-2-(piperidin-2-yl)acetic acid (also known as ritalinic acid) or a stereoisomer thereof or mixture of stereoisomers thereof, or a pharmaceutically acceptable salt thereof, is reacted with dimethylcarbonate to form a reaction mixture, for example, as depicted in the above reaction scheme;

wherein the dimethylcarbonate is present in an amount in the range of from about 2.5 to about 8.0 molar equivalents (relative to the moles of the compound of formula (I-A)) or any amount or range therein, optionally in an amount in the range of from about 3.5 to about 6.5 molar equivalents, or any amount or range therein, optionally in an amount in the range of from about 4.0 to about 5.5 molar equivalents, or any amount or range therein, for example in an amount of about 4.25 molar equivalents;

adding methanol to the reaction mixture; wherein the methanol is present in an amount in the range of from about 0.75 to about 5.0 molar equivalents (relative to the moles of the compound of formula (I-A)) or any amount or range therein, optionally in an amount in the range of from about 0.9 to about 4.0 molar equivalents, or any amount or range therein, optionally in an amount in the range of from about 1.0 to about 1.5 molar equivalents, or any amount or range therein, for example, in an amount of about 1.25 molar equivalents;

adding sulfuric acid to the reaction mixture; wherein the sulfuric acid is present in an amount in the range of from about 1.05 to about 2.5 molar equivalents (relative to the moles of the compound of formula (I-A)), or any amount or range therein, optionally in an amount in the range of from about 1.2 to about 2.0 molar equivalents, or any amount or range therein, optionally in an amount in the range of from about 1.4 to about 1.6 molar equivalents, or any amount or range therein, for example, in an amount of about 1.5 molar equivalents;

wherein the reaction mixture is at a temperature in the range of from about 22°C to about 90°C, optionally at a temperature in the range of from about 50°C to about 90°C, optionally at a temperature in the range of from about 70°C to about 85°C, for example, at about reflux temperature; to yield the corresponding compound of formula (I-S) (i.e. the corresponding sulfate salt of the compound of formula (I-A)), in a product mixture.

The present inventors have found that in the reaction of the compound of formula (I-A) with dimethylcarbonate; in the presence of sulfuric acid; some decomposition of the dimethylcarbonate may occur, resulting in the production of methanol. It is intended that in the processes of the present invention, the listed methanol is added to the reaction mixture as a separate component. It is further intended that in the processes of the present invention, the molar amounts of methanol added to the reaction mixture, in one or more portions or aliquots (as listed herein) are calculated relative to the moles of the compound of formula (I-A) and further, that the amount of methanol added is independent of any methanol which may be present and / or formed in the reaction mixture as a result of the decomposition of the dimethylcarbonate.

One skilled in the art will recognize that in addition to containing the compound of formula (I-S), the product mixture may additionally contain one or more of: sodium sulfate, methanol, unreacted ritalinic acid, unreacted dimethylcarbonate and unreacted sulfuric acid. One skilled in the art will further recognize that the reaction described above will additionally result in the formation of carbon dioxide, which is optionally vented from the reaction mixture.

In an embodiment, the molar ratio of methanol: DMC is in the range of from 0.094 (MeOH): 1.0 (DMC) to 2.0 (MeOH): 1.0 (DMC), or any amount or range therein. In another embodiment, the ratio of methanol: DMC is in the range of from 0.138 : 1.0 to 1.14:1.0, or any amount or range therein. In another embodiment, the ratio of methanol : DMC is in the range of from 0.20 : 1.0 to 0.375 :1.0, or any amount or range therein.

In an embodiment, the molar ratio of sulfuric acid ($H_2SO_4$) : DMC is in the range of from 0.131 ($H_2SO_4$) : 1.0 (DMC) to 1.0 ($H_2SO_4$) : 1.0 (DMC), or any amount or range therein. In another embodiment, the ratio of $H_2SO_4$: DMC is in the range of from 0.185 : 1.0 to 0.571 :1.0, or any amount or range therein. In another embodiment, the ratio of $H_2SO_4$: DMC is in the range of from 0.8 : 1.0 to 0.375:1.0, or any amount or range therein.

In an embodiment, the molar ratio of methanol: sulfuric acid ($H_2SO_4$) is in the range of from 0.3 (MeOH): 1.0 ($H_2SO_4$) to 4.76 (MeOH): 1.0 ($H_2SO_4$), or any amount or range therein. In another embodiment, the ratio of methanol : $H_2SO_4$ is in the range of from 0.45 : 1.0 to 3.33 :1.0, or any amount or range therein. In another embodiment, the ratio of methanol: $H_2SO_4$ is in the range of from 0.67 : 1.0 to 1.07 :1.0, or any amount or range therein.

In another embodiment, the present invention is directed to a process for the preparation of a compound of formula (I) wherein the molar ratio of DMC : methanol : sulfuric acid (relative to the moles of the compound of formula (I-A)) are as follows: 2.5 to 8.0 molar equivalents DMC : 0.75 to 5.0 molar equivalents MeOH : 1.05 to 2.5 molar equivalents of sulfuric acid.

In another embodiment, the present invention is directed to a process for the preparation of a compound of formula (I) wherein the molar ratio of DMC : methanol : sulfuric acid (relative to the moles of the compound of formula (I-A)) are as follows: 3.5 to 6.5 molar equivalents DMC : 0.90 to 4.0 molar equivalents MeOH : 1.2 to 2.0 molar equivalents of sulfuric acid.

In another embodiment, the present invention is directed to a process for the preparation of a compound of formula (I) wherein the molar ratio of DMC : methanol : sulfuric acid (relative to the moles of the compound of formula (I-A)) are as follows: 4.0 to 5.0 molar equivalents DMC : 1.0 to 1.5 molar equivalents MeOH : 1.4 to 1.6 molar equivalents of sulfuric acid.

In another embodiment, the present invention is directed to a process for the preparation of a compound of formula (I) wherein the molar ratio of DMC : methanol : sulfuric acid (relative to the moles of the compound of formula (I-A)) is about 4.5 molar equivalents DMC : 1.25 molar equivalents methanol: 1.5 molar equivalents sulfuric acid.

In another embodiment, the present invention is directed to a process for the preparation of a compound of formula (I) wherein the DMC is present in an amount of 1.75 wt/wt equivalents, the methanol is present in an amount of 0.175 wt/wt equivalents and the sulfuric acid is present in an amount of 1.5 wt/wt equivalents.

In an embodiment of the present invention, the sulfuric acid is added to the reaction mixture of the compound of formula (I-A) (i.e. ritalinic acid), DMC and methanol. In another embodiment of the present invention, the sulfuric acid is added to a reaction mixture of compound of formula (I-A) (i.e. ritalinic acid), DMC and methanol, wherein the reaction mixture is at a temperature in the range of from about 20°C to about reflux temperature. Optionally, the sulfuric acid is added after mixture of compound of formula (I-A) (i.e. ritalinic acid), DMC and methanol at about reflux temperature.

Although not intended to be authoritative as to the parameters of the reaction, it was observed that addition of the sulfuric acid to the reaction mixture of compound of formula (I-A) (i.e. ritalinic acid), DMC and methanol at about reflux temperature resulted in better control of the reaction exotherm and the carbon dioxide release of the reaction.

STEP B:

The product mixture containing the compound of formula (I-S) is reacted with a suitably selected base, optionally, a suitably selected inorganic base, such as sodium hydroxide, ammonium hydroxide, potassium hydroxide, sodium carbonate, potassium carbonate, calcium carbonate, sodium bicarbonate, potassium phosphate, and the like, optionally, sodium hydroxide, ammonium hydroxide, sodium carbonate or potassium carbonate, optionally sodium hydroxide, for example 18% sodium hydroxide; wherein the amount of base is at least sufficient to neutralize any remaining sulfuric acid and release the compound of formula (I) as a free base; optionally, the amount of base is sufficient to raise the pH of the resulting mixture to pH > about 8, optionally pH >9, optionally, pH in the range of from about pH 9 to about pH 10; (and wherein, in an embodiment of the present invention, the molar amount of the base is optionally equal to about the molar amount of sulfuric acid initially added to the ritalinic acid);

and to the resulting mixture is then added a suitably selected organic solvent such as isopropyl acetate, toluene, ethyl acetate, benzene, hexanes, xylene, cyclohexane, and the like; optionally, isopropyl acetate, ethyl acetate or toluene, optionally, isopropyl acetate; optionally, in an amount in the range of from 2.5 to 10 molar equivalents (relative to the moles of ritalinic acid), optionally in an amount in the range of from 4.0 to 6.0 molar equivalents, optionally in amount in the range of from 4.3 molar equivalents to 5.3 molar equivalents; to yield a biphasic mixture comprising an organic layer containing the compound of formula (I) and an aqueous layer.

Optionally, the organic solvent is selected to yield a clean biphasic mixture comprising an aqueous phase and an organic phase (a biphasic mixture which does not have or is substantially free of a rag or emulsion layer in between

the two layers of the biphasic layers) to facilitate separation of the phases one from the other. Optionally, (and particularly optionally for large scale / commercial and / or continuous manufacturing) the organic solvent is selected to yield a biphasic mixture, wherein the organic layer is the top layer.

One skilled in the art will recognize that when working with a biphasic mixture, separation of an aqueous phase from an organic phase (after the neutralization and/or the subsequent washing steps), may be impaired by the formation of **a third phase** (also known as **a rag or rag layer**). This third phase / rag layer is generally a stable, possibly voluminous, intermediate phase typically characterized by the presence of suspended particulates (solid or liquid), which occurs between the aqueous and the organic phases, and makes separation of the phases, one from the other, difficult. In extreme cases, this third phase or rag layer may even prevent separation of the phases one from the other. In the most adverse cases, (which is particularly disadvantageous on a manufacturing scale) the phase separation tank or tanks affected by the presence of the third phase or rag layer must be emptied completely and cleaned. The contents of the phase separation tank or tanks must then be worked-up (i.e., extractive work-up) or disposed of with great effort, and often with considerable associated costs. Moreover, under certain circumstances, the presence of the third phase or rag layer can also lead to the interruption of production (particularly continuous production), and potential loss of product yield.

One skilled in the art will further recognize that the presence of a relatively small amount of particulates at the separation stage may be tolerated from the perspective of plant operation (large scale manufacture), if the quantity is sufficiently low to allow the required separations to be carried out. Under continuous processing, the rate of build-up of the solids to form a third phase or rag layer may also be sufficiently low as to allow production to continue for some time before having to interrupt the process.

Thus, it is advantageous in the development of a batch or continuous process to employ solvent systems and phase separations which do not form or is substantially free of a third phase or rag layer.

The biphasic mixture is separated according to known methods, to yield the corresponding organic layer and the corresponding aqueous layer. One skilled in the art will recognize that in the biphasic mixture the organic layer contains the organic solvent, the compound of formula (I) and methanol.

In an embodiment of the present invention, the organic layer solvent is selected to yield an organic layer which is optionally substantially free of starting materials and / or side products. Optionally, the organic layer contains less than 5 mole %, optionally less than 3 mole %, optionally less than 1 mole %, optionally less than 0.75 mole %, optionally less than 0.5 mole % of starting materials and / or side products.

One skilled in the art will recognize that the aqueous layer contains water, and may further contain one or more of the following: unreacted ritalinic acid, unreacted DMC, unreacted MeOH, unreacted sulfuric acid and / or sodium sulfate.

One skilled in the art will further recognize that in addition to liberating the free base of the compound of formula (I), the reaction with a suitably selected base (optionally an inorganic base) and the separation of the phases of the resulting biphasic mixture, serves as a purification step for the product stream (or the organic phase containing product formed from the processes described herein).

The organic phase/layer is further, optionally purified according to known methods, for example by polish filter.

<u>STEP C:</u>

The compound of formula (I) (in the organic phase/layer) is optionally diluted with a suitably selected organic solvent such as isopropanol (IPA), isopropyl acetate, ethanol, methanol, and the like;

and then reacted with a suitably selected acid such as hydrochloric acid (HCl), sulfuric acid, phosphoric acid, acetic acid, hydrobromic acid, malic acid, citric acid, tartaric acid, and like, optionally HCl, for example, HCl gas ($HCl_{(g)}$), aqueous HCl or a molar solution of HCl in a suitably selected solvent;

optionally, in the presence of water, optionally in the presence of water in an amount in the range of from 0.05 to 1.0 wt/wt equiv. water (relative to the weight of the compound of formula (I-A); optionally in the presence of water in an amount in the range of from 0.05 to 0.8 wt/wt equiv.; optionally in the presence of water in an amount in the range of from 0.05 to 0.6 wt/wt equiv.; optionally in the presence of water in an amount in the range of from 0.05 to 0.5 wt/wt equiv.; optionally in the presence of water in an amount in the range of from 0.05 to 0.2 wt/wt equiv.; optionally in the presence of water in an amount in the range of from 0.1 to 0.5 wt/wt equiv.; optionally in the presence of water in an amount in the range of from 0.3 to 0.6 wt/wt equiv.; optionally in the presence of water in an amount in the range of from 0.2 to 0.4 wt/wt equiv.; optionally in the presence of water in an amount in the range of from 0.2 to 0.5 wt/wt equiv.; optionally in the presence of water in an amount of 0.4 wt/wt equiv.; optionally in the presence of water in an amount of 0.3 wt/wt equiv.; optionally in the presence of water in an amount of 0.1 wt/wt equiv.; to yield the corresponding acid-addition salt, a compound of formula (Ia).

The resulting mixture is optionally cooled and the acid addition salt of the compound of formula (I), isolated according to known methods, for example, by crystallization or evaporation of solvent.

In an embodiment of the present invention, the suitably selected acid (optionally, HCl as a gas, aqueous solution

or molar solution) is added to the organic phase/layer containing the compound of formula (I). In another embodiment of the present invention, the organic layer containing the compound of formula (I) is diluted with a suitably selected organic solvent such as isopropanol, isopropyl acetate, ethanol, methanol, and the like; and then added to a molar solution of HCl.

In an embodiment of the present invention, the suitably selected acid is aqueous HCl. In another embodiment of the present invention, the suitably selected acid is HCl gas in isopropanol.

In an embodiment of the present invention, the mixture of HCl gas in isopropanol is added to the reaction mixture over a period of time in the range of from about 15 minutes to about 3 hours, or any amount of time or range of times, therein. In another embodiment of the present invention, the mixture of HCl gas in isopropanol is added to the reaction mixture over a period of time in the range of from about 15 minutes to about 1 hours, for example over about 15 minutes, over about 20 minutes, over about 30 minutes, over about 15 to about 45 minutes, over about 20 to about 40 minutes or over about 45 to about 60 minutes.

In an embodiment of the present invention, the reaction mixture (comprising methylphenidate and an organic solvent) is added to a mixture of aqueous HCl in isopropanol. In another embodiment of the present invention, the reaction mixture (comprising methylphenidate and an organic solvent) is added to a mixture of aqueous HCl in isopropanol optionally, over a period of time in the range of from about 15 min to about 3 hours, more optionally over a period of time in the range of from about 30 min to about 2 hours, for optionally over a time in the range of from about 45 min to about 75 min, optionally over a time in the range of from about 1 hour to 2 hours, or any amount of time or range therein, continuously or via multiple batches.

The compound of formula (I) or compound of formula (Ia) (for example, the HCl salt of the compound of formula (I)) may be further purified according to known methods, for example by recrystallization from a suitably selected solvent or mixture of solvents such as $C_{1-4}$alkyl alcohol (for example, methanol, ethanol, isopropyl alcohol, and the like), isopropyl acetate, a mixture of $C_{1-4}$alkyl alcohol and isopropyl acetate (for example a mixture of methanol and isopropyl acetate, a mixture of ethanol and isopropyl acetate, a mixture of isopropanol and isopropyl acetate, and the like), and the like; optionally in the presence of water; wherein the water is present in for example, an amount in the range of from 0.05 and 1.0 wt/wt equiv. (relative to the weight of the compound of formula (I-A)); optionally in the presence of water in an amount in the range of from 0.05 to 0.8 wt/wt equiv.; optionally in an amount in the range of from 0.05 to 0.6 wt/wt equiv.; optionally in the presence of water in an amount in the range of from 0.05 to 0.5 wt/wt equiv.; optionally in the presence of water in an amount in the range of from 0.05 to 0.2 wt/wt equiv.; optionally in the presence of water in an amount in the range of from 0.1 to 0.5 wt/wt equiv.; optionally in the presence of water in an amount in the range of from 0.3 to 0.6 wt/wt equiv.; optionally in the presence of water in an amount in the range of from 0.2 to 0.5 wt/wt equiv.; optionally in the presence of water in an amount in the range of from 0.2 to 0.4 wt/wt equiv.; optionally in the presence of water in an amount of 0.4 wt/wt equiv.; optionally in the presence of water in an amount 0.3 wt/wt equiv.; optionally in the presence of water in an amount of 0.1 wt/wt equiv.

In certain embodiments, the compound of formula (Ia) (for example, the HCl salt of the compound of formula (I), optionally, the HCl salt of di-threo-methylphenidate, optionally, the HCl salt of dex-methylphenidate) is recrystallized from a suitably selected solvent or mixture of solvents, optionally in the presence of water, as described above; wherein the recrystallization further optionally comprises distillation of the recrystallization mixture, at about reflux temperature (for example, at a temperature in the range of from about 30°C to about 85°C, optionally at a temperature in the range of from about 45°C to about 65°C, optionally at about 55°C), to remove between 20% and 65% of the recrystallization solvent volume, or any amount or range thereof, optionally to remove between 30% and 50% of the recrystallization solvent volume, optionally to remove between 35% to 40% of the recrystallization solvent volume.

Although not intended to be authoritative as to the effect of any given reaction or recrystallization parameter on the physical properties or yield of the isolated product, and without being bound by any proposed theory, it is theorized that addition of higher amount(s) of water to the recrystallization mixture results in larger particle size for the isolated compound as a solid (for example, as a crystalline solid). However, addition of higher amount(s) of water may also results in decreased yield, as the solubility of the product in water may result in a greater amount of the product remaining dissolved in the recrystallization solvent mixture, rather than precipitating as a solid (optionally as a crystalline solid). Thus, in certain embodiments of the present invention, the recrystallization mixture is distilled to remove a portion of the recrystallization solvent mixture (including a portion of the water), to yield the product as a solid, optionally as a crystalline solid, wherein the yield and / or particle size are optimized and / or balanced to meet any desired specifications. In another embodiment of the present invention, the recrystallization mixture is distilled to remove a portion of the recrystallization solvent (including a portion of the water), as described herein, to yield the product as a solid, optionally as a crystalline solid, without affecting (e.g. decreasing or increasing) the particle size of the isolated product.

The present invention is further directed to a process for the preparation of a compound of formula (IA) as, for example, depicted in Scheme 2, below.

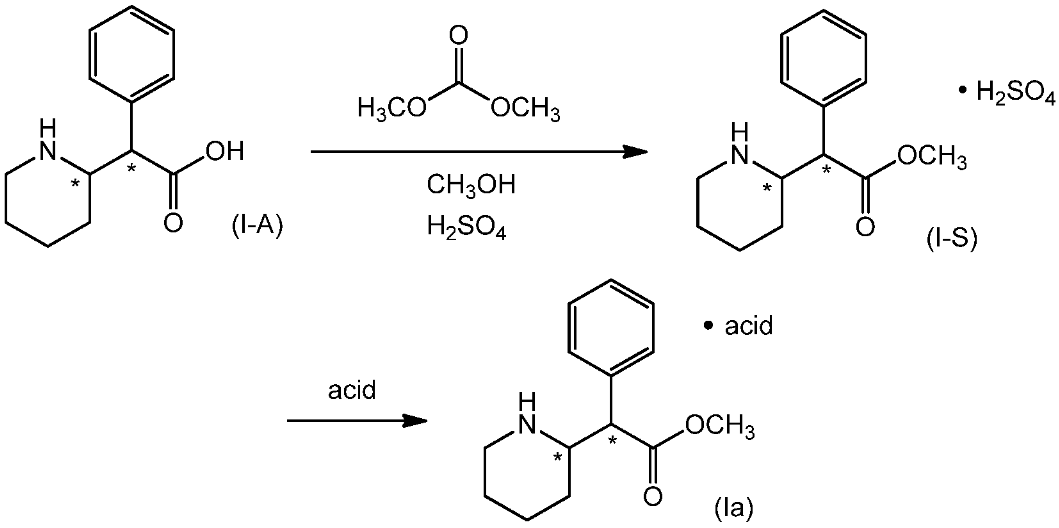

(I-A) (I-S) (Ia)

Scheme 2

STEP A:

Accordingly, a compound of formula (I-A) (i.e. 2-phenyl-2-(piperidin-2-yl)acetic acid (also known as ritalinic acid) or a stereoisomer thereof or mixture of stereoisomers thereof, or a pharmaceutically acceptable salt thereof, is reacted with dimethylcarbonate to form a reaction mixture; to which mixture methanol is added; and to which mixture sulfuric acid is added; at a temperature in the range of from about 22°C to about 90°C, or any amount or range therein; according to the process as, for example, depicted in Scheme 1, above; to yield the corresponding compound of formula (I-S) (i.e. the corresponding sulfate salt of the compound of formula (I-A)), in a product mixture.

STEP B:

To the product mixture (prepared from the reaction of ritalinic acid with DMC, in the presence of methanol and sulfuric acid) is added first a suitably selected organic solvent such as isopropyl acetate, isopropyl alcohol, ethanol, methanol, and the like or mixtures thereof, optionally isopropyl acetate; optionally, in an amount in the range of from 4.0 to 25.0 molar equivalents (relative to the moles of ritalinic acid), optionally in an amount in the range of from 5.5 to 15.0 molar equivalents, optionally in amount in the range of from 7.0 molar equivalents to 12.0 molar equivalents;
and then reacted with a suitably selected acid such as hydrochloric acid (HCl), sulfuric acid, phosphoric acid, acetic acid, hydrobromic acid, malic acid, citric acid, tartaric acid, and like, optionally HCl, for example, HCl gas, aqueous HCl or a molar solution of HCl in a suitably selected solvent;
optionally, in the presence of water, optionally in the presence of water in an amount in the range of from 0.05 to 1.0 wt/wt equiv. water (relative to the weight of the compound of formula (I-A); optionally in the presence of water in an amount in the range of from 0.05 to 0.8 wt/wt equiv.; optionally in the presence of water in an amount in the range of from 0.1 to 0.6 wt/wt equiv.; optionally in the presence of water in an amount in the range of from 0.05 to 0.5 wt/wt equiv.; optionally in the presence of water in an amount in the range of from 0.05 to 0.2 wt/wt equiv.; optionally in the presence of water in an amount in the range of from 0.1 to 0.5 wt/wt equiv.;optionally in the presence of water in an amount in the range of from 0.3 to 0.6 wt/wt equiv.; optionally in the presence of water in an amount in the range of from 0.2 to 0.5 wt/wt equiv.; optionally in the presence of water in an amount in the range of from 0.2 to 0.4 wt/wt equiv.; optionally in the presence of water in an amount of 0.4 wt/wt equiv.; optionally in the presence of water in an amount of 0.3 wt/wt equiv.; optioinally in the presence of water in an amount of 0.1 wt/wt equiv.; to yield the corresponding acid-addition salt, a compound of formula (Ia).

Optionally, the acid-addition salt of the compound of formula (I) is isolated according to known methods, for example, by crystallization or evaporation of solvent.

In an embodiment of the present invention, the reaction mixture (comprising methylphenidate sulfate salt and an organic solvent) is added to a mixture of aqueous HCl in isopropanol. In another embodiment of the present invention, the reaction mixture (comprising methylphenidate sulfate salt and an organic solvent) is added to a mixture of aqueous HCl in isopropanol optionally, over a period of time in the range of from about 15 min to about 3 hours, more optionally over a period of time in the range of from about 30 min to about 2 hours, for optionally over a time in the range of from about 45 min to about 75 min, optionally over a time in the range of from about 1 hour to 2 hours, or any amount of time

or range therein, continuously or via multiple batches.

The compound of formula (I) or compound of formula (Ia) (for example, the HCl salt of the compound of formula (I)) may be further purified according to known methods, for example by recrystallization from a suitably selected solvent or mixture of solvents such as $C_{1-4}$alcohol (for example methanol, ethanol, isopropyl alcohol, and the like), isopropyl acetate, a mixture of $C_{1-4}$alkyl alcohol and isopropyl acetate (for example, a mixture of methanol and isopropyl acetate, a mixture of ethanol and isopropyl acetate, a mixture of isopropanol and isopropyl acetate, and the like), optionally in the presence of water; wherein the water is present in for example, an amount in the range of from 0.05 and 1.0 wt/wt equiv. (relative to the weight of the compound of formula (I-A)); optionally in the presence of water in an amount in the range of from 0.05 to 0.8 wt/wt equiv.; optionally in an amount in the range of from 0.05 to 0.6 wt/wt equiv.; optionally in the presence of water in an amount in the range of from 0.05 to 0.5 wt/wt equiv.; optionally in the presence of water in an amount in the range of from 0.05 to 0.2 wt/wt equiv.; optionally in the presence of water in an amount in the range of from 0.1 to 0.5 wt/wt equiv.; optionally in the presence of water in an amount in the range of from 0.3 to 0.6 wt/wt equiv.; optionally in the presence of water in an amount in the range of from 0.2 to 0.5 wt/wt equiv.; optionally in the presence of water in an amount in the range of from 0.2 to 0.4 wt/wt equiv.; optionally in the presence of water in an amount of 0.4 wt/wt equiv. optionally in the presence of water in an amount of 0.3 wt/wt equiv.; optionally in the presence of water in an amount of 0.1 wt/wt equiv.

In certain embodiments, the compound of formula (Ia) (for example, the HCl salt of the compound of formula (I), optionally, the HCl salt of di-threo-methylphenidate, optionally, the HCl salt of dex-methylphenidate) is recrystallized from a suitably selected solvent or mixture of solvents, optionally in the presence of water, as described above; wherein the recrystallization further optionally comprises distillation of the recrystallization mixture, at about reflux temperature (for example, at a temperature in the range of from about 30°C to about 85°C, optionally at a temperature in the range of from about 45°C to about 65°C, optionally at about 55°C), to remove between about 20% and about 65% of the recrystallization solvent volume, or any amount or range thereof, optionally to remove between about 30% and about 50% of the recrystallization solvent volume, optionally to remove between about 35% to about 40% of the recrystallization solvent volume.

In an embodiment of the present invention, the suitably selected acid is aqueous HCl. In another embodiment of the present invention, the suitably selected acid is HCl gas in isopropanol.

Although not intended to be authoritative as to the mechanism of the reaction(s) or the identity of any critical parameters of the reaction(s), the ratio of dimethylcarbonate to methanol and the amount of sulfuric acid were each determined to affect product yield, reaction rate and the physical manipulation of the product and starting materials. It is theorized, without being limited whatsoever thereby, that the use of methanol in combination with sulfuric acid, also increases the rate of reaction conversion. Additionally, the use of methanol in combination with sulfuric acid results in a reaction mixture which exhibits favorable stirring characteristics, while maximizing through-put in the reaction vessels and avoiding spontaneous crystallization or precipitation of starting material and / or product. Thus, the process of the present invention is advantageously suitable for large scale and / or commercial manufacture.

In an embodiment, the present invention is directed to a process for the recrystallization of di-threo-methylphenidate hydrochloride;
wherein the recrystallized di-threo-methylphenidate hydrochloride comprises a total of less than about 0.1 area % of an impurity compound selected from the group consisting of an impurity compound of formula (IMP-5), an impurity compound of formula (IMP-6), an impurity compound of formula (IMP-15), an impurity compound of formula (IMP-16), and mixtures thereof; comprising the steps of

(a) dissolving di-threo-methylphenidate hydrochloride in a mixture of an organic solvent and water; wherein the organic solvent is selected from the group consisting of ethanol and isopropanol; and wherein the water is present in an amount in the range of from 0.05 wt/wt equiv. to 0.6 wt/wt equiv., or any amount or range thereof (optionally in an amount in the range of from 0.1 wt/wt equiv. to 0.5 wt/wt equiv, optionally in an amount in the range of from 0.2 wt/wt equiv. to 0.4 wt/wt equiv. optionally in an amount of 0.3 wt/wt equiv.);
(b) heating the mixture to about reflux temperature;
(c) optionally distilling off between 20% and 65% by volume (optionally between 30% and 50%, optionally between 35% and 40%), or any amount or range thereof, of the mixture of an organic solvent and water;
(d) cooling the mixture to a temperature less than about room temperature; and
(e) isolating the di-threo-methylphenidate as a solid.

In another embodiment, the present invention is directed to a process for the recrystallization of di-threo-methylphenidate hydrochloride comprising the steps of

(a) dissolving di-threo-methylphenidate hydrochloride in a mixture of an organic solvent and water; wherein the organic solvent is selected from the group consisting of ethanol and isopropanol; and wherein the water is present

in an amount in the range of from 0.1 wt/wt equiv. to 0.6 wt/wt equiv., or any amount or range thereof (optionally in an amount in the range of from 0.1 wt/wt equiv. to 0.5 wt/wt equiv, optionally in an amount in the range of from 0.2 wt/wt equiv. to 0.4 wt/wt equiv. optionally in an amount of 0.3 wt/wt equiv.);
(b) heating the mixture to about reflux temperature;
(c) optionally distilling off between 20% and 65% by volume (optionally between 30% and 50%, optionally between 35% and 40%), or any amount or range thereof, of the mixture of an organic solvent and water;
(d) cooling the mixture to a temperature less than about room temperature; and
(e) isolating the di-threo-methylphenidate as a solid.

In an embodiment, the present invention is directed to a process for the recrystallization of di-threo-methylphenidate hydrochloride, wherein (a) the amount of water and / or (b) the amount of the mixture of an organic solvent and water (i.e. the recrystallization solvent) which is distilled off are controlled to maximize the yield of the isolated di-threo-methylphenidate and / or to control the particle size of the isolated di-threo-methylphenidate as a solid, optionally as a crystalline solid.

In an embodiment, the present invention is directed to a process for the recrystallization of dex-methylphenidate hydrochloride; comprising the steps of

(a) dissolving dex-methylphenidate hydrochloride in a mixture of an organic solvent and water; wherein the organic solvent is selected from the group consisting of ethanol and isopropanol; and wherein the water is present in an amount in the range of from 0.05 wt/wt equiv. to 0.6 wt/wt equiv., or any amount or range thereof (optionally in an amount in the range of from 0.1 wt/wt equiv. to 0.5 wt/wt equiv, optionally in an amount in the range of from 0.2 wt/wt equiv. to 0.4 wt/wt equiv. optionally in an amount of 0.3 wt/wt equiv.);
(b) heating the mixture to about reflux temperature;
(c) optionally distilling off between 20% and 65% by volume (optionally between 30% and 50%, optionally between 35% and 40%), or any amount or range thereof, of the mixture of an organic solvent and water;
(d) cooling the mixture to a temperature less than about room temperature; and
(e) isolating the dex-methylphenidate as a solid.

In an embodiment, the present invention is directed to a process for the recrystallization of dex-methylphenidate hydrochloride, as herein described, wherein (a) the amount of water and / or (b) the amount of the mixture of an organic solvent and water (i.e. the recrystallization solvent) which is distilled off are controlled to maximize the yield of the isolated dex-methylphenidate and / or to control the particle size of the isolated dex-methylphenidate as a solid, optionally as a crystalline solid.

## Pharmaceutical Compositions

Pharmaceutical compositions containing one or more of the compounds of the invention described herein as the active ingredient can be prepared by mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of the product prepared according to any of the processes described herein, as the active ingredient, the product is admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals,

the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.5 mg to about 100 mg or any amount or range therein, and may be given at a dosage of from about 5 mg/day to about 100 mg / day, or any amount or range therein, optionally from about 5.0 mg/day to about 60 mg/day, or any amount or range therein. The dosages, however, may be varied depending upon the requirement of the patients, the severity of the condition being treated and the compound being employed. The use of either daily administration or post-periodic dosing may be employed.

Optionally these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from about 0.5 mg to about 100 mg, or any amount or range therein (for example 5 mg, 10 mg, 20 mg, 30 mg, etc.) of the active ingredient of the present invention. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the compositions of the present compounds may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinylpyrrolidone or gelatin.

A pharmaceutical composition can comprise a product prepared according to any of the processes described herein and a pharmaceutically acceptable carrier. The pharmaceutical composition may contain between about 0.5 mg and about 100 mg of the compound, or any amount or range therein; optionally from about 5 mg to about 30 mg of the compound, or any amount or range therein, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixirs, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like or mixtures thereof. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like

or mixtures thereof. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like or mixtures thereof. For parenteral administration, sterile suspensions and solutions are desired. Isotonic preparations which generally contain suitable preservatives are employed when intravenous administration is desired.

To prepare a pharmaceutical composition of the present invention, a product prepared according to any of the processes described herein, as the active ingredient is, admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

The products prepared according to any of the processes described herein may be administered in any of the foregoing compositions and according to dosage regimens established in the art whenever treatment of the disclosed disorders is required.

The daily dosage of the products may be varied over a wide range from about 5 mg to about 100 mg per adult human per day (optionally from about 5 mg to about 60 mg per day), or any amount or range therein. For oral administration, the compositions are optionally provided in the form of tablets containing, 0.01, 0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the patient to be treated. An effective amount of the drug is ordinarily supplied at a dosage level of from about 5 mg/day to about 100 mg/day, or any amount or range therein. Optionally, the range is from about 5mg/day to about 60 mg/day, or any amount or range therein. Optionally, from about 10 mg/day to about 60 mg/day, or any amount or range therein (for example at about 5 mg/day, 10 mg/day, 20 mg/day, 30 mg/day, 60 mg/day, or any amount therein). The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both *in vivo* and *in vitro* trials using suitable, known and generally accepted cell and / or animal models are predictive of the ability of a test compound to treat or prevent a given disorder.

One skilled in the art will further recognize that human clinical trials including first-in-human, dose ranging and efficacy trials, in healthy patients and / or those suffering from a given disorder, may be completed according to methods well known in the clinical and medical arts.

## EXAMPLES

In the Examples which follow, some synthesis products are listed as having been isolated as a residue. It will be understood by one of ordinary skill in the art that the term "residue" does not limit the physical state in which the product was isolated and may include, for example, a solid, an oil, a foam, a gum, a syrup, and the like.

### Example 1: Synthesis of Di-threo-Methylphenidate Hydrochloride

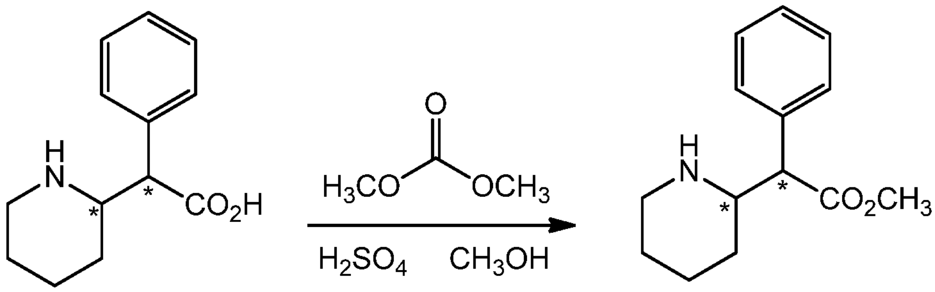


A reactor was charged with di-threo-ritalinic acid (25.0 g, 0.114 mol, 1.0 eq.), dimethylcarbonate (43.75 g, 0.486 mol, 4.26 eq.), and methanol (4.38 g, 0.137 mol, 1.20 mol eq.). To the resulting suspension was then added sulfuric acid (16.77 g, 0.171 mol, 1.44 eq.) at 22°C. The system was allowed to naturally exotherm to ~ 60 °C during the addition. The suspension was then heated to and maintained reflux temperature for 18 h 40 min. The suspension quickly turned to a pale yellow homogenous solution upon heating. Upon reaction completion the solution was cooled to room temperature (~ 22 °C) and isopropyl acetate (50.0 g, 0.490 mol, 4.30 eq.) was charged to the solution. 18% Sodium hydroxide was then added to adjust the pH of the mixture to pH ≥ 9, while maintaining an internal reaction temperature of ≤ 20°C. The internal reaction temperature was then adjusted to ~ 22°C and the mixture stirred for about 10 minutes. Agitation was ceased and the resulting biphasic mixture was allowed to separate over 20 minutes. The aqueous layer was removed and discarded. Water (37.5 g, 2.08 mol, 18.25 eq.) was added to the organic layer and the resulting mixture was stirred for 20 minutes. Agitation was ceased and the resulting biphasic mixture was allowed to settle for 20 minutes. The aqueous layer was removed and discarded to yield the organic layer (containing di-threo-methylphenidate base).

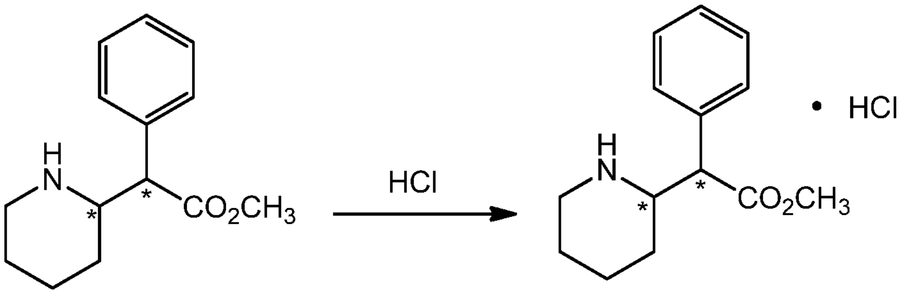


In a separate reactor IPA (50.0 g, 0.832 mol, 7.30 eq.) was cooled to ≤ 20°C and $HCl_{(g)}$ (4.16 g, 0.114 mol, 1.0 eq.) was slowly added to the IPA slowly while maintaining an internal temperature of ≤ 20°C. The IPA/HCl solution was then warmed to room temperature and the organic phase (containing di-threo-methylphenidate base) was added to the IPA/HCl solution via addition funnel. Isopropyl Acetate (6.25 g, 0.061 mol, 0.54 eq.) was used to rinse the addition funnel into the mixture. The mixture was stirred for 30 minutes at room temperature, then cooled to 0°C and stirred for 17.5 h. The resulting mixture was filtered and washed with isopropanol (25 g, 0.416 mol, 3.65 eq.). The resulting solids were dried under vacuum at 40°C to a constant weight to yield di-threo-methylphenidate hydrochloride as a white crystalline solid. (28.86 g, 94.2% yield)

**Example 2: Synthesis of Di-threo-Methylphenidate Sulfate Salt**

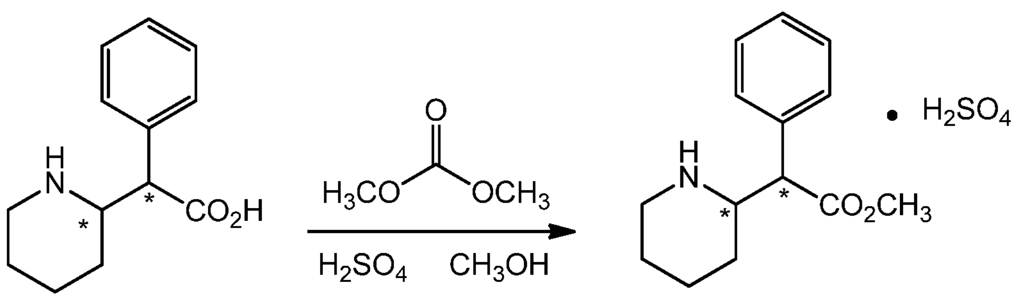


Di-threo-methylphenidate was prepared according to the process(es) of the present invention, varying reaction parameters including amount of DMC, amount of sulfuric acid, amount of MeOH, reaction time, reaction temperature,

etc., and measuring % unreacted di-threo-ritalinic acid, as detailed in Table 1 below.

One skilled in the art will recognize that % unreacted di-threo-ritalinic acid as listed in Table 1 below, provides an indirect measure of the product yield. One skilled in the art will further recognize that for any given example, the calculation of 100%-(% unreacted di-threo-ritalinic acid) provides a maximum theoretical yield for the desired di-threo-methylphenidate.

In each experiment listed in Table 1 below, was performed essentially in accordance with the procedure outlined in Example 1 and where the order of addition was as follows: (1) di-threo-ritalinic acid, (2) dimethylcarbonate, (3) methanol, (4) sulfuric acid; followed by work-up with an inorganic base and reaction with HCl.

**Table 1: Representative Synthesis Examples**

| DMC (mol eq.) | MeOH (mol eq.) | $H_2SO_4$ (mol eq.) | Total Reaction Time (h) | % Unreacted Di-threo-Ritalinic Acid |
|---|---|---|---|---|
| 6.51 | 13.52 | 2.37 | 21 | 0.62 |
| 0 | 1.975 | 2.37 | 20 | 15.81 |
| 4.87 | 10.27 | 1.44 | 22 | 8.74 |
| 6.51 | 13.52 | 1.44 | 23 | 3.00 |
| 6.51 | 13.52 | 1.92 | 23 | 1.00 |
| 3.65 | 5.48 | 1.44 | 18 | 6.38 |
| 3.65 | 0.86 | 1.25 | 20 | 6.81 |
| 3.04 | 0.68 | 1.15 | 20 | 20.44 |
| 4.87 | 1.37 | 1.44 | 18 | 1.64 |
| 4.87 | 0 | 1.68 | 22 | 2.82[a] |
| 4.26 | 1.20 | 1.44 | 21 | 1.14 |
| 5.00 | 1.50 | 1.54 | 20 | 1.10 |
| 4.87 | 0 | 1.44 | 22 | 2.11[a] |

[a] Although % unreacted di-threo-ritalinic acid for this experiment was less than 5%, the initial solubility of the reagents at the onset of the reaction was very poor resulting in reaction mixture that is difficult to handle in the synthesis process, thus these conditions were deemed not suitable for commercial / large scale manufacture. This result is consistent with REKHA, et al., when performing reactions on non-liquid amines as starting materials. REHKA, et al., notes that phenylglycine and phenylalanine, both crystalline solids, "did not react at all due to high insolubility."

## Example 3 - Prophetic Example

## Synthesis of Di-Threo-Methylphenidate Hydrochloride

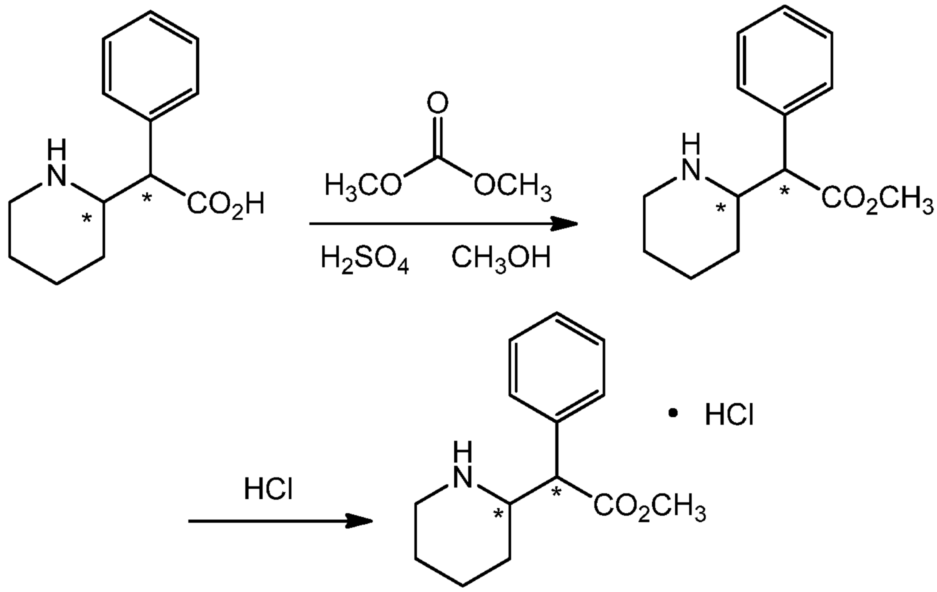


An appropriate size reactor is charged with di-threo-ritalinic acid (25.0 g, 0.114 mol, 1.0 eq.), dimethylcarbonate (43.75 g, 0.486 mol, 4.26 eq.), and methanol (4.38 g, 0.137 mol, 1.20 mol eq.). To the resulting suspension is then added sulfuric acid (16.77 g, 0.171 mol, 1.44 eq.) at a temperature in the range of 20°C - reflux. The suspension is then heated to maintain reflux for 12-22 hours. Upon reaction completion (as measured by a suitable analytical method, such as HPLC) the resulting solution is cooled to room temperature (~22°C) and isopropyl acetate (50.0 g, 0.490 mol, 4.30 eq.) is added to the solution. 18 % sodium hydroxide is then added to adjust the pH of the mixture to about pH ≥ 9, while maintaining an internal reaction temperature (of the reaction mixture) at ≤ 20°C. The internal reaction temperature is then adjusted as needed to 15-25°C and the resulting mixture stirred for at least 10 minutes. Agitation is ceased and the resulting biphasic mixture is allowed to separate over at least 20 minutes. The aqueous layer is removed and discarded. Water (37.5 g, 2.08 mol, 18.25 eq.) is added to the organic layer and the resulting mixture is stirred for at least 20 minutes. Agitation is ceased and the resulting biphasic mixture is allowed to settle for at least 20 minutes. The aqueous layer is removed and discarded. The organic solution is polished filtered and transferred to a new reaction vessel. Isopropyl acetate (6.25 g, 0.061 mol, 0.54 eq.) is used to rinse the original reactor and polished filtered as well. IPA (50.0 g, 0.832 mol, 7.30 eq.) is then added to the organic solution (which contains the di-threo-methylphenidate base in isopropyl acetate). HCl (4.16 g, 0.114, 1.0 eq.) is then added at a temperature in the range of 0°C - reflux over between 5-60 minutes (for example over about 45 minutes).

Alternatively, a solution of IPA and HCl is prepared by addition of HCl to IPA, while maintaining an internal temperature of ≥ 25°C. The organic solution (containing di-threo-methylphenidate base in isopropyl acetate) is then added to the IPA/HCl solution at a temperature in the range of 0°C - reflux over between 5-60 minutes (for example over about 45 minutes).

Once the addition is completed, the resulting mixture is stirred at the addition temperature for 1 hour and then cooled to 10°C. The mixture is then filtered and the residue dried under vacuum to a constant weight, to yield the desired di-thero-methylphenidate hydrochloride. Expected / Desired Yield = 90-95 %

## Example 4 - Preparation and Crystallization of Dex-Methylphenidate Hydrochloride

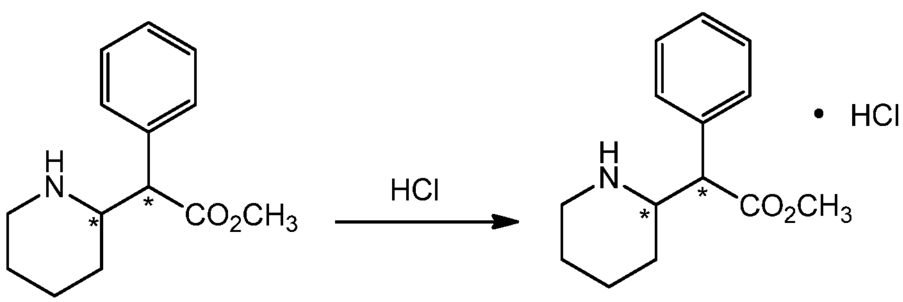

Dex-Methylphenidate in an isopropyl acetate solution was prepared from dex-ritalinic acid HCl (25 g scale), as previously described herein.

To a mixture of HCl (4.57) in isopropanol (50.0 g) was added water (10.0 g), and the resulting solution was heated to 40°C. The dex-methylphenidate in isopropyl acetate solution was added over 60 minutes. The resulting solution was held at 40°C for 30 minutes, cooled to 15°C over 30 minutes and then held at that temperature for 30 minutes. The resulting solids were filtered and washed with isopropyl acetate to yield dex-methylphenidate hydrochloride as a white crystalline solid (24.3 g, 78.9% yield).

The impurity level of the isolated solid was 0.05 area% from *in situ* HPLC measurement of 0.19 area% (before the recrystallization).

**Example 5- Preparation of Di-Threo-Methylphenidate Hydrochloride. Effect of %Water and % IPA on Yield and Impurity: DoE Experiments**

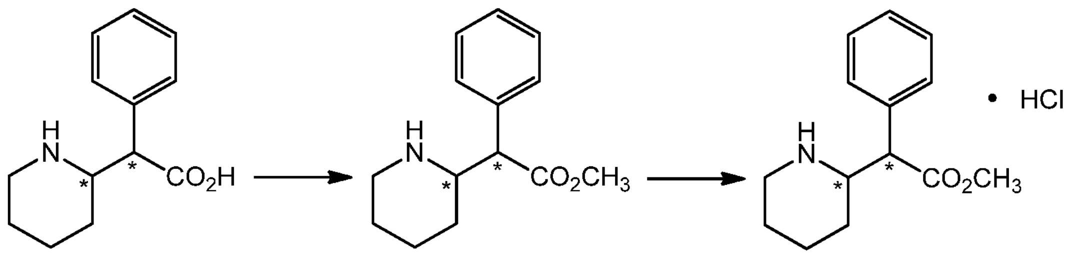

Di-threo-methylphenidate HCl was prepared according to the procedure as detailed below, varying the amount of isopropanol and water used when reacting di-threo-methylphenidate with HCl; and measuring, by HPLC, the area % of the impurity compounds of formula (IMP-5) and formula (IMP-6) present in the isolated di-threo methylphenidate hydrochloride salt product. The parameters and results from these experiments were as listed in Table 2, below.

Procedure:

A 250 mL 4-neck jacketed reactor was charged with di-threo-ritalinic acid (25 mg), the di-threo-ritalinic acid including the impurity compounds of formula (IMP-1) and formula (IMP-2). The reactor was then charged with dimethylcarbonate (5.0 mol. eq., 2.05 wt/wt eq.) and methanol (1.5 mol. eq., 0.219 wt/wt eq.). The reactor was then charged with sulfuric acid (1.6 mol. eq., 0.72 wt/wt eq.), in one portion at ~20°C. The jacket temperature was set at 85°C and the mixture held at this temperature for 16 hours. The resulting mixture was then cooled 15-20°C and isopropyl acetate added (1.75 wt/wt eq.). The mixture was cooled to 15°C, and 18% NaOH (1.05 mol. eq. wrt $H_2SO_4$, 1.68 mol. eq relative to di-threo-ritalinic acid) was added, at a rate which maintained the temperature of the reaction mixture at <30°C. The resulting mixture was stirred for 20 minutes, and then let stand to allow the resulting layers to settle. The lower, aqueous layer was drained and discarded. To the organic layer was then added deionized water (1.5 wt/wt eq.), the mixture stirred for 25-30 minutes, and then let stand to allow the resulting layers to settle. The lower, aqueous layer was drained and discarded. The organic layer containing di-threo-methylphenidate in isopropyl acetate was stored under refrigeration.

A solution of HCl gas (1.05 mol eq., 0.175 wt/wt eq.) in isopropanol was prepared at <30°C. To the solution was then added water (in an amount as listed in Table 2, below) and the mixture heated to 43-47°C. To the mixture was then added the organic layer containing di-threo-methylphenidate in isopropyl acetate, over between about 45-60 minutes. The resulting mixture was held at temperature for 30 minutes, then cooled to 15°C, over 30 minutes and the resulting suspension held at 15°C for 30 minutes. The suspension was filtered, the wet cake washed with isopropyl acetate (5 wt/wt equivalents) and the solids dried at 40°C under vacuum to constant weight.

Samples of the isolated solids, prepared as described above, were evaluated according to the HPLC method which follows to determine area % of desired product and impurity compounds of formula (IMP-5) and formula (IMP-6), as shown in Table 2 below.

**Table 2: Experimental Parameters and Product Impurity Results**

| Batch ID | IPA (wt/wt eq) | Water (wt/wt eq) | Yield (%) | Impurity (Area %) |
|---|---|---|---|---|
| 5-1 | 4.0 | 0.0 | 92.6 | 0.10 |

(continued)

| Batch ID | IPA (wt/wt eq) | Water (wt/wt eq) | Yield (%) | Impurity (Area %) |
|---|---|---|---|---|
| 5-2 | 2.0 | 0.4 | 80.4 | 0.04 |
| 5-3 | 3.0 | 0.2 | 88.4 | 0.06 |
| 5-4 | 4.0 | 0.4 | 81.1 | 0.04 |
| 5-5 | 3.0 | 0.2 | 88.8 | 0.07 |
| 5-6 | 2.0 | 0.0 | 93.8 | 0.10 |

HPLC Method:

The area % of the impurity compounds of formula (IMP-5) and formula (IMP-6) were determined using and Agilent 1100 series HPLC, with quaternary gradient pump, UV detection @220 nm, Waters Symmetry C8.5μm 3.9X150 mm, with C18 guard column (or equivalent), 40°C column temperature, flow rate of 2.75 mL/min (total run time 16 min) and injection volume of 15μL, and mobile phase gradient as listed below:

| Time (min) | Mobile Phase A | Mobile Phase B |
|---|---|---|
| 0 | 90% | 10% |
| 7 | 65% | 35% |
| 10 | 50% | 50% |
| 12 | 50% | 50% |
| 13 | 90% | 10% |
| 16 | 90% | 10% |

**Unadjusted buffer** solution was prepared by mixing mL water (3440 mL), $NH_4{\bullet}H_2PO_4$ and sodium 1-octanesulfonate (OSA) (5.53 g). **Mobile Phase A** was prepared by mixing the unadjusted buffer solution (3000 mL) and TEA (12.0 mL), adjusting the mixture to pH 2.90±0.02 with 85% $H_3PO_4$, then adding acetonitrile (490 mL) and mixing well. **Mobile Phase B** was prepared by mixing acetonitrile (1600 mL) and the unadjusted buffer solution (400 mL) and mixing well. **Testing samples** were prepared by dissolving 50 mg of isolated product in mobile phase A, to a final volume of 75 mL.

Under the above conditions, the relative retention time for ritalinic acid hydrochloride was 0.54; for methylphenidate hydrochloride was 1.00 and for the impurity compounds of formula (IMP-5) and formula (IMP-6) was 0.87.

**Example 6 - Preparation of Di-Threo-Methylphenidate Hydrochloride, Effect of %Water, % HCl and Temperature on Yield and Impurity; DoE Experiments**

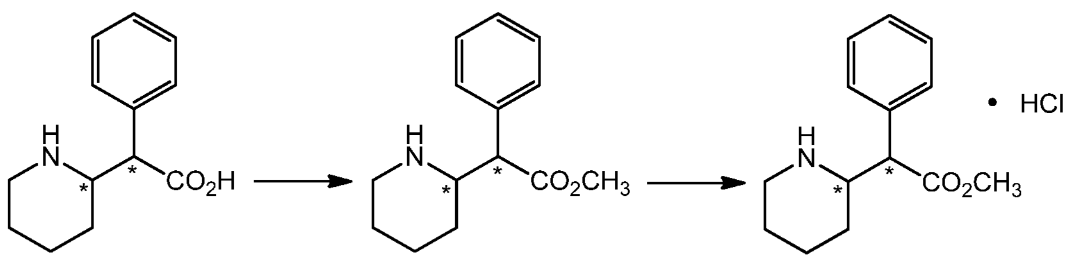

Di-threo-methylphenidate HCl was prepared according to the procedure as detailed below, varying the amount of HCl, the amount of water used when reacting di-threo-methylphenidate with HCl and the reaction temperature; and measuring, by HPLC, the area % of the impurity compounds of formula (IMP-5) and formula (IMP-6) present in the isolated di-threo methylphenidate hydrochloride salt product. The parameters and results from these experiments were as listed in Table 3, below.

Procedure:

A 250 mL 4-neck jacketed reactor was charged with di-threo-ritalinic acid (25 mg), the di-threo-ritalinic acid including the impurity compounds of formula (IMP-1) and formula (IMP-2). The reactor was then charged with dimethylcarbonate (5.0 mol. eq., 2.05 wt/wt eq.) and methanol (1.5 mol. eq., 0.219 wt/wt eq.). The resulting mixture was heated to about 70°C and the reactor was charged with sulfuric acid (1.6 mol. eq., 0.72 wt/wt eq.) via addition funnel. The jacket temperature was set at 85°C and the mixture held at this temperature for 16 hours. The resulting mixture was then cooled 15-20°C and isopropyl acetate added (1.75 wt/wt eq.). The mixture was cooled to 15°C, and 18% NaOH (1.05 mol. eq. wrt $H_2SO_4$, 1.68 mol. eq relative to di-threo-ritalinic acid) was added, at a rate which maintained the temperature of the reaction mixture at <30°C. The resulting mixture was stirred for 10-20 minutes, and then let stand to allow the resulting layers to settle. The lower, aqueous layer was drained and discarded. To the organic layer was then added deionized water (1.50 wt/wt eq.), the mixture stirred for 20-30 minutes, and then let stand to allow the resulting layers to settle. The lower, aqueous layer was drained and discarded. The organic layer containing di-threo-methylphenidate in isopropyl acetate was stored under refrigeration.

A solution of HCl gas (in an amount as listed in table 3, below)) in isopropanol was prepared at <30°C. To the solution was then added water (in an amount as listed in Table 3, below) and the mixture heated to temperature (as listed in Table 3, below). To the mixture was then added the organic layer containing di-threo-methylphenidate in isopropyl acetate, over between about 45-60 minutes. The resulting mixture was held at temperature for 30 minutes, then cooled to 15°C, over 30 minutes and the resulting suspension held at 15°C for 30 minutes. The suspension was filtered, the wet cake washed with isopropyl acetate (5 wt/wt equivalents) and the solids dried at 40°C under vacuum to constant weight.

The area % of the impurity compounds of formula (IMP-5) and formula (IMP-6) were determined using the HPLC method as described in Example 5 above.

**Table 3: Experimental Parameters and Product Impurity Results**

| Batch ID | HCl (wt/wt 1.05eq) | Water (wt/wt eq) | Temp (°C) | Yield (%) | Impurity (Area %) |
|---|---|---|---|---|---|
| 6-1 | 1.00 | 0.4 | 40 | 78.3 | 0.04 |
| 6-2 | 1.00 | 0.1 | 50 | 92.1 | 0.12 |
| 6-3 | 1.10 | 0.7 | 50 | 60.7 | 0.02 |
| 6-4 | 1.10 | 0.7 | 30 | 63.1 | 0.03 |
| 6-5 | 1.10 | 0.1 | 50 | 91.3 | 0.11 |
| 6-6 | 1.10 | 0.1 | 30 | 91.4 | 0.13 |
| 6-7 | 1.10 | 0.7 | 50 | 63.7 | 0.03 |
| 6-8 | 1.00 | 0.7 | 30 | 58.6 | 0.02 |
| 6-9 | 1.00 | 0.1 | 30 | 89.3 | 0.10 |
| 6-10 | 1.05 | 0.4 | 40 | 78.9 | 0.05 |

**Example 7 - Preparation of Di-threo-methylphenidate Hydrochloride**

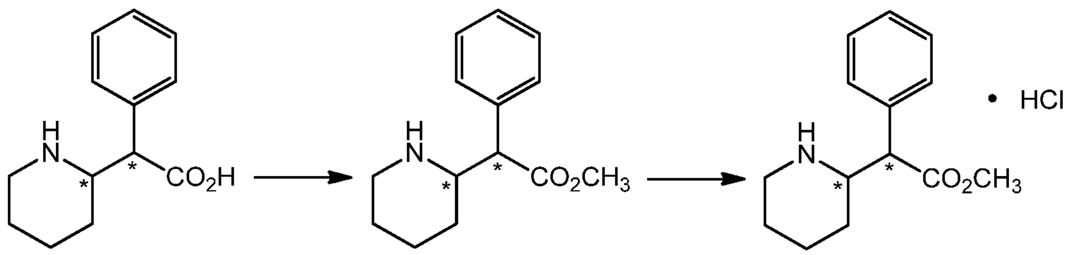

STEP 1: Preparation of Di-Threo-Methylphenidate

A 500 mL reactor was charged with di-threo-ritalinic acid (50 g, 0.228 mol), dimethylcarbonate (102.7 g, 5.0 mol. eq., 2.054 wt/wt eq.), and methanol (11.0 g, 1.5 mol. eq., 0.219 wt/wt eq.); the di-threo-ritalinic acid including the impurity compounds of formula (IMP-1) and formula (IMP-2). The jacket temperature was increased to 85°C (target internal temperature ~70 °C). To the reactor, at 68-73°C, was added sulfuric acid (35.8 g, 1.6 mol. eq., 0.715 wt/wt eq.) via addition funnel, while maintaining the temperature at <80°C. The reaction was stirred overnight (~ 16 hours) at a jacket temperature of 85°C and stir overnight.

The resulting mixture was then cooled to 15-20°C (jacket temperature ~12°C). To the resulting mixture was then added isopropyl acetate (100.0 g, 2.00 wt/wt eq.), followed by addition of 18% NaOH (85.0 g, 1.68 mol. eq., 1.70 wt/wt eq.) via addition funnel, while maintaining the temperature at ≤30°C. The internal reaction mixture temperature was adjusted to 20-25°C and the mixture stirred for about 15 minutes. The layers were allowed to settle for about 20 minutes and the lower, caustic layer separated and discarded. To the organic layer was then added deionized water (75.0 g, 1.50 wt/wt eq.) and the resulting mixture stirred for about 20 minutes. The layers were allowed to settle for about 20 minutes and the lower, water layer separated and discarded. The organic layer, containing di-threo-methylphenidate was transferred to a polyurethane bottle and retained.

STEP 2: Preparation of HCl in IPA Solution

A reactor was charged with IPA (500.0 g) and water (25.0 g, 0.1 wt/wt eq.) at ~20°C. Gaseous HCl (43.75 g, 1.05 mol. eq., 0.175 wt/wt eq.) was charged to the reactor at a rate of NMT 0.7 g/min at ≤ 30°C. The resulting mixture was used in Experiment 7-1.

Alternatively, aqueous (~37 wt%) HCl (118.25 g, 1.05 mol. eq., 0.473 wt/wt eq.) was charged to reach 0.4 wt/wt eq water. The solution was stored refrigerated prior to use. Said mixture was used in Experiment 7-2.

STEP 3: Preparation of Di-Threo-Methylphendiate Hydrochloride

A second reactor was charged with the solution of HCl/IPA/Water prepared in STEP 2 above (128.65 g containing 100 g IPA (2.00 wt/wt eq.), 23.6 g aqueous 37 wt% HCl (1.05 mol. eq., 0.175 wt/wt eq.), and 5.0 g water (0.1 wt/wt eq.). The internal temperature of the solution was adjusted to 40°C and the mixture held at this temperature for about 30 minutes. To the reactor was then added the organic layer containing the di-threo-methylphenidate, prepared in STEP 1 above, via addition funnel over between 45-75 minutes (about 49 minutes in the case of batch ID 7-1 in Table 4; and about 54 minutes in the case of batch ID 7-2 in Table 4), while maintaining the temperature at 40°C (± 5°C). The polyurethane container was rinsed with IpOAc (12.5 g, 0.25 wt/wt eq.), and the rinse transferred to the reactor. The resulting mixture was held at 40°C for 30 minutes, then cooled to 10-15°C, over 30 minutes (jacket temperature ~12°C), then stirred at 10-15°C for 30 minutes. The resulting suspension was filtered through a Buchner funnel and the wet cake washed with IpOAc (100.0 g, 2.0 wt/wt eq.). The solids were dried under full vacuum at 40°C for > 15 hours until constant weight.

As noted, the procedure described above was used to prepare the two above identified batches of di-threo-methylphenidate hydrochloride, starting from di-threo-ritalinic acid containing 0.11%wt/wt of the impurity compounds of formula (IMP-1) and (IMP-2) in the case of batch ID 7-1 and 0.13% wt./wt of the impurity compounds of formula (IMP-1) and (IMP-2) in the case of batch ID 7-2. A sample of each di-threo-methylphenidate hydrochloride product was then tested to determine area % of the impurity compounds of formula (IMP-5) and (IMP-6) in each batch, according to the HPLC method as described in Example 5, above. Yield and impurity levels for batch IDs 7-1 and 7-2 were as listed in Table 4, below.

**Table 4: Experimental Parameters and Product Impurity Results**

| Batch ID | Di-threo-ritalinic acid Impurity (wt/wt equiv.) | Water (wt/wt eq) | Yield (%) | Di-threo-methylphenidate HCl Impurity (Area %) |
|---|---|---|---|---|
| 7-1 | 0.11 | 0.4 | 76.1 | 0.05 |
| 7-2 | 0.13 | 0.4 | 76.7 | 0.07 |

**Example 8 - Re- Crystallization of Di-Threo-Methylphenidate Hydrochloride from Ethanol/Water**

A reactor was charged with di-threo-methylphenidate hydrochloride (50 g), where the di-threo-methylphenidate hydrochloride contained -0.1 area % of the impurity compounds of formula (IMP-5) and (IMP-6). To the reactor was then

added EtOH (4.2 wt/wt eq) and water (0.12 wt/wt eq), and the resulting mixture heated reflux (~80°C). The mixture was held at reflux until all of the di-threo-methylphenidate was observed to dissolve. The resulting mixture was cooled to 10°C over 1.5 hours and then held at 10°C for 30 minutes. The resulting suspension was filtered, the filter cake washed with ethanol (1.0 wt/wt eq) and the resulting solids dried under vacuum at 40°C, for >15 hours, until constant weight (Yield 40.75g, 81.5%); Area % impurity @ <0.05%.

**Example 9 - Re- Crystallization of Di-Threo-Methylphenidate Hydrochloride from Isopropanol/Water**

A reactor was charged with di-threo-methylphenidate hydrochloride (50 g), where the di-threo-methylphenidate hydrochloride contained -0.1 area % of the impurity compounds of formula (IMP-5) and (IMP-6). To the reactor was then added isopropanol (4.2 wt/wt eq) and water (0.51 wt/wt eq), and the resulting mixture heated reflux (~80°C). The mixture was held at reflux until all of the di-threo-methylphenidate was observed to dissolve. The resulting mixture was cooled to 10°C, over 1.5 hours and then held at 10°C for 30 minutes. The resulting suspension was filtered, the filter cake washed with isopropanol (1.0 wt/wt eq) and the resulting solids dried under vacuum at 40°C, for >15 hours, until constant weight (Yield 40.29g, 80.6%); Area % impurity @ <0.05%.

**Example 10 - Preparation of Di-threo-methylphenidate Hydrochloride**

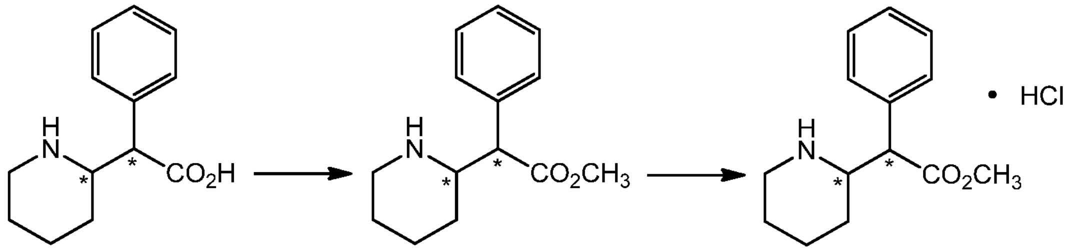

STEP 1: Preparation of Di-Threo-Methylphenidate

A 1 L reactor was charged with di-threo-ritalinic acid (125 g, 0.570 mol), dimethylcarbonate (256.7 g, 5.0 mol. eq., 2.054 wt/wt eq.), and methanol (27.5 g, 1.5 mol. eq., 0.219 wt/wt eq.). The jacket temperature was increased to 85°C (target internal temperature ~70°C). To the reactor, at 68-73°C, was added sulfuric acid (89.5 g, 1.6 mol. eq., 0.716 wt/wt eq.) via addition funnel, while maintaining the temperature at <80°C. The reaction was stirred overnight (~ 16 hours) at a jacket temperature of 85°C and stir overnight.

The resulting mixture was then cooled to 15-20°C (jacket temperature ~12°C). To the resulting mixture was then added isopropyl acetate (250.0 g, 2.00 wt/wt eq.), followed by addition of 18% NaOH (212.5 g, 1.68 mol. eq., 1.70 wt/wt eq.) via addition funnel, while maintaining the temperature at ≤30°C. The internal reaction mixture temperature was adjusted to 20-25°C and the mixture stirred for about 15 minutes. The layers were allowed to settle for about 20 minutes and the lower, caustic (aqueous) layer separated and discarded. To the organic layer was then added deionized water (187.5 g, 1.50 wt/wt eq.) and the resulting mixture stirred for about 20 minutes. The layers were allowed to settle for about 20 minutes and the lower, water layer separated and discarded. The organic layer, containing di-threo-methylphenidate was transferred to a polyurethane bottle and retained.

STEP 2A: Preparation of Di-Threo-Methylphendiate Hydrochloride (Distillation of -50% Solvent Volume)

A second reactor was charged with the solution of 37% HCl (59.0 g, 1.05 mol. eq., 0.47 wt/wt eq.) and IPA (250.0 g, 2.00 wt/wt eq.). The internal temperature of the solution was adjusted to 55°C. To this reactor was then added the organic layer containing the di-threo-methylphenidate, prepared in STEP 1 above, via addition funnel over 90 minutes (identified as Batch ID2A in Table 5 below), while maintaining the temperature at 55°C (± 5°C). The polyurethane container was rinsed with IpOAc (31.25 g, 0.25 wt/wt eq.), and the rinse transferred to the reactor. A vacuum controller was attached to the reactor and approximately 50% of the volume was removed by distillation at 50-55°C and 320-345 mbar. The resulting concentrated mixture was cooled to 10-15°C, over 30 minutes (jacket temperature ~12°C), then stirred at 10-15°C for 30 minutes. The resulting suspension was filtered through a Buchner funnel and the wet cake washed with IpOAc (125.0 g, 1.0 wt/wt eq.). The solids were dried under full vacuum at 40°C for > 15 hours until constant weight (87.2% yield).

STEP 2B: Preparation of Di-Threo-Methylphendiate Hydrochloride (Distillation of -30% Solvent Volume)

A second reactor was charged with the solution of 37% HCl (23.6 g, 1.05 mol. eq., 0.47 wt/wt eq.) and IPA (100.0 g, 2.00 wt/wt eq.). The internal temperature of the solution was adjusted to 55°C. To this reactor was then added the organic layer containing the di-threo-methylphenidate, prepared in STEP 1 (50 g scale ritalinic acid), via addition funnel over 28 minutes (identified as Batch ID 2B of Table 5), while maintaining the temperature at 55°C (± 5°C). The polyurethane container was rinsed with isopropyl acetate (12.5 g, 0.25 wt/wt eq.), and the rinse transferred to the reactor. A vacuum controller was attached to the reactor and approximately 30% of the volume was removed by distillation at 50-55 °C at reflux. The resulting concentrated mixture was cooled to 10-15°C, over 30 minutes (jacket temperature ~12°C), then stirred at 10-15°C for 30 minutes. The resulting suspension was filtered through a Buchner funnel and the wet cake washed with IpOAc (50.0 g, 1.0 wt/wt eq.). The solids were dried under full vacuum at 40°C for >15 hours until constant weight (88.9% yield).

Presented below in Table 5 are experimental parameters and product yield and impurity results for the Batch IDs 2A and 2B described above.

**Table 5: Experimental Parameters and Product Impurity**

| Batch ID | Water (wt/wt eq) | MPH Yield (%) | Di-threo-ritalinic acid as Impurity in MPH product (wt/wt %) |
|---|---|---|---|
| 2A (50% Vol. Distill.) | 0.3 | 87.2 | 0.07 |
| 2B (30% Vol. Distill.) | 0.3 | 86.9 | 0.05 |

**Formulation Example 1 - Prophetic Example Solid, Oral Dosage Form**

As a specific embodiment of an oral composition, 100 mg of the compound, as prepared in Example 1, is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A process for the recrystallization of di-threo-methylphenidate hydrochloride;
wherein the recrystallized di-threo-methylphenidate hydrochloride comprises a total of less than 0.1 area % of an impurity compound selected from the group consisting of
an impurity compound of formula (IMP-5)

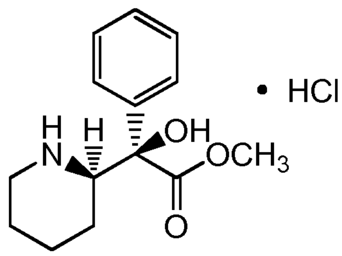

(IMP-5),

an impurity compound of formula (IMP-6)

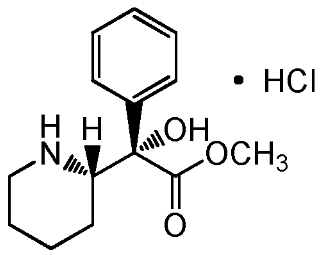

(IMP-6),

an impurity compound of formula (IMP-15)

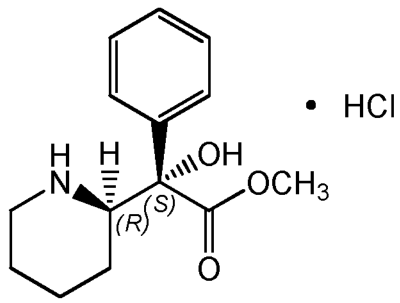

(IMP-15),

an impurity compound of formula (IMP-16)

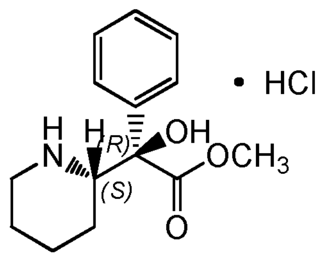

(IMP-16),

and mixtures thereof; comprising the steps of

(a) dissolving di-threo-methylphenidate hydrochloride in a mixture of an organic solvent and water; wherein the organic solvent is selected from the group consisting of ethanol and isopropanol; and wherein the water is present in an amount in the range of from 0.1 wt/wt equiv. to 0.6 wt/wt equiv.;
(b) heating the mixture to about reflux temperature;
(c) optionally distilling off between 20% and 65% by volume of the mixture of an organic solvent and water;
(c) cooling the mixture to a temperature less than about room temperature; and
(d) isolating the di-threo-methylphenidate as a solid.

**2.** A process as in Claim 1, wherein the organic solvent is ethanol; and wherein the water is present in an amount of 0.12 wt/wt equiv..

**3.** A process as in Claim 1, wherein the recrystallized di-threo-methylphenidate hydrochloride comprises a total of less than 0.05 area % of the impurity compound selected from the group consisting of the impurity compound of formula (IMP-5), the impurity compound of formula (IMP-6), the impurity compound of formula (IMP-15), the impurity compound of formula (IMP-16), and mixtures thereof.

**4.** A process for removing or reducing the amount of one or more of an impurity compound of formula (IMP-A)

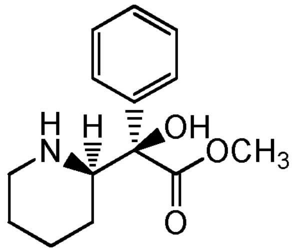

(IMP-A)

or a salt thereof, an impurity compound of formula (IMP-B)

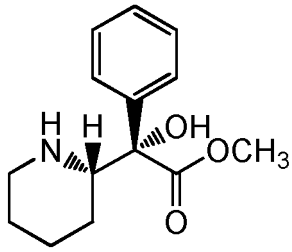

(IMP-B)

or a salt thereof, an impurity compound of formula (IMP-C)

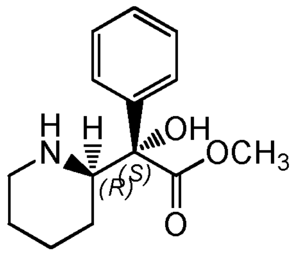

(IMP-C)

or a salt thereof, or an impurity compound of formula (IMP-D)

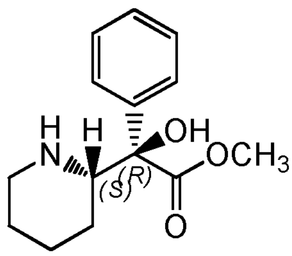

(IMP-D)

or a salt thereof,
from a pharmaceutically acceptable salt of di-threo-methylphenidate;
comprising the steps of

I. providing a mixture of

i. di-threo-methylphenidate sulfate;
ii. an impurity compound selected from the group consisting of a sulfate salt of the impurity compound of formula (IMP-A), a sulfate salt of the impurity compound of formula (IMP-B), a sulfate salt of the impurity compound of formula (IMP-C), a sulfate salt of the impurity compound of formula (IMP-D), and mixtures thereof;

II. adding an organic solvent to the mixture of Step 1 to yield a second mixture; and
III. reacting the second mixture of Step 2 with an acid; in the presence of water; wherein the water is present in an amount in the range of from 0.05 wt/wt equiv. to 1.0 wt/wt equiv.; to yield a product comprising a corresponding pharmaceutically acceptable salt of di-threo-methylphenidate in a mixture with a corresponding impurity compound selected from the group consisting of a corresponding pharmaceutically acceptable salt of the impurity compound of formula (IMP-A), a corresponding pharmaceutically acceptable salt of the impurity compound of formula (IMP-B), a corresponding pharmaceutically acceptable salt of the impurity compound of formula (IMP-C), a corresponding pharmaceutically acceptable salt of the impurity compound of formula (IMP-D), and mixtures thereof;

wherein the mixture of Step 1, is prepared according to a process comprising the steps of:

A. providing a composition comprising

i. di-threo-ritalinic acid or pharmaceutically acceptable salt thereof; and
ii. one or more of an impurity precursor selected from the group consisting of an impurity compound of formula (IMP-1)

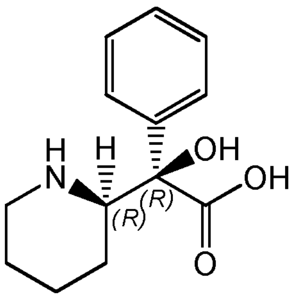

(IMP-1)

or a salt thereof, an impurity compound of formula (IMP-2)

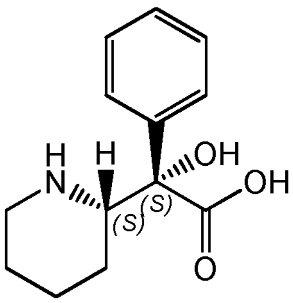

(IMP-2)

or a salt thereof, an impurity compound of formula (IMP-11)

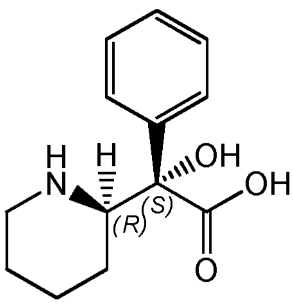

(IMP-11)

or a salt thereof, and an impurity compound of formula (IMP-12)

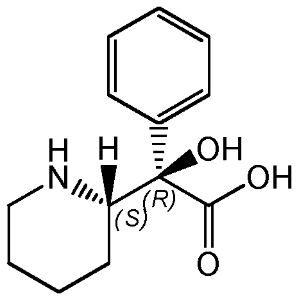

(IMP-12)

or a salt thereof;

B. reacting the composition of Step A with dimethylcarbonate, wherein the dimethylcarbonate is present in an amount in the range of from 2.5 to 8.0 molar equivalents; in the presence of methanol, wherein the methanol is present in an amount in the range of from 0.75 to 5.0 molar equivalents, wherein said methanol is added as a separate components, wherein the methanol is added in one or more portions or aliquots, and wherein the amount of methanol is independent of any methanol which may be later introduced into the reaction mixture; in the presence of sulfuric acid, wherein the sulfuric acid is present in an amount in the range of from 1.05 to 2.5 molar equivalents;

at a temperature in the range of from about room temperature to about 90°C; to yield a mixture comprising:

i. di-threo-methylphenidate sulfate; and

ii. the impurity compound selected from the group consisting of the sulfate salt of the impurity compound of formula (IMP-A), the sulfate salt of the impurity compound of formula (IMP-B), the sulfate salt of the impurity compound of formula (IMP-C), the sulfate salt of the impurity compound of formula (IMP-D), and mixtures thereof.

**5.** A process as in Claim 4, wherein the acid of Step 3 is hydrochloric acid.

**6.** A process as in Claim 4, wherein the amount of the impurity selected from the group consisting of the pharmaceutically acceptable salt of the impurity compound of formula (IMP-A), the pharmaceutically acceptable salt of the impurity compound of formula (IMP-B), the pharmaceutically acceptable salt of the impurity compound of formula (IMP-C), the pharmaceutically acceptable salt of the impurity compound of formula (IMP-D), and mixtures thereof; is present in an amount less than 0.05 area %.

**7.** A process for removing or reducing the amount of one or more of an impurity compound of formula (IMP-A)

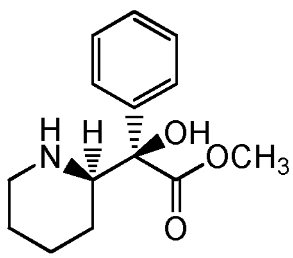

(IMP-A)

or a salt thereof, an impurity compound of formula (IMP-B)

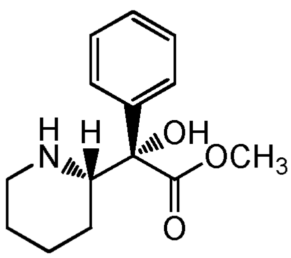

(IMP-B)

or a salt thereof, an impurity compound of formula (IMP-C)

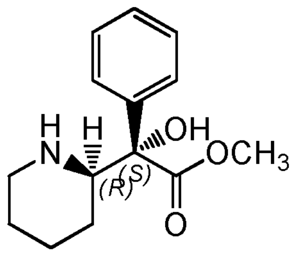

(IMP-C)

or a salt thereof, or an impurity compound of formula (IMP-D)

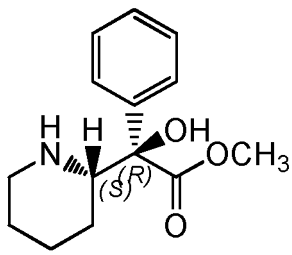

(IMP-D)

or a salt thereof; from di-threo-methylphenidate or a pharmaceutically acceptable salt of di-threo-methylphenidate; comprising the steps of

I providing a mixture of

i. di-threo-methylphenidate sulfate;
ii. an impurity compound selected from the group consisting a freebase impurity compound of formula (IMP-A), a freebase impurity compound of formula (IMP-B), a freebase impurity compound of formula (IMP-C), a freebase impurity compound of formula (IMP-D), and mixtures thereof;
iii an organic phase comprising an organic solvent;

II reacting the mixture of Step 1 with an acid; in the presence of water; wherein the water is present in an amount in the range of from 0.05 wt/wt equiv. to 1.0 wt/wt equiv.;
to yield a product comprising a corresponding pharmaceutically acceptable salt of di-threo-methylphenidate in a mixture with a corresponding impurity compound selected from the group consisting of a corresponding pharmaceutically acceptable salt of the impurity compound of formula (IMP-A), a corresponding pharmaceutically acceptable salt of the impurity compound of formula (IMP-B), a corresponding pharmaceutically acceptable salt of the impurity compound of formula (IMP-C), a corresponding pharmaceutically acceptable salt of the impurity compound of formula (IMP-D), and mixtures thereof;

wherein the mixture of Step 1, is prepared according to a process comprising the steps of:

A. providing a composition comprising

a. di-threo-ritalinic acid or pharmaceutically acceptable salt thereof; and
b. an impurity precursor selected from the group consisting of an impurity compound of formula (IMP-1)

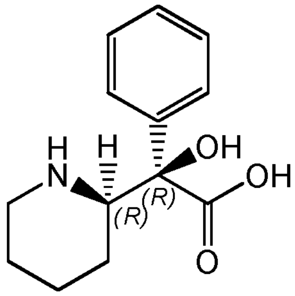

(IMP-1)

or a salt thereof, an impurity compound of formula (IMP-2)

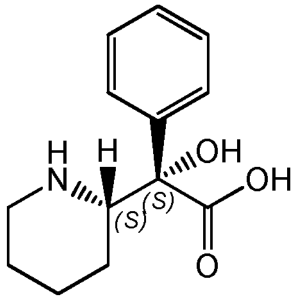

(IMP-2)

or a salt thereof, an impurity compound of formula (IMP-11)

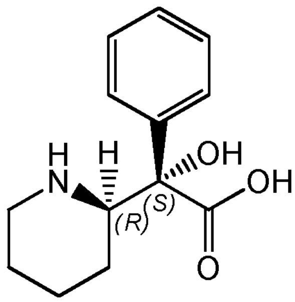

(IMP-11)

or a salt thereof, an impurity compound of formula (IMP-12)

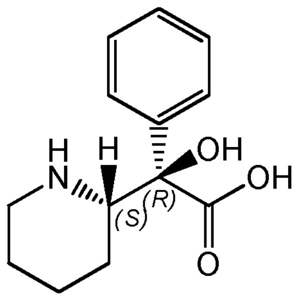

(IMP-12)

or a salt thereof, and mixtures thereof;

B. reacting the composition of Step A with dimethylcarbonate, wherein the dimethylcarbonate is present in an amount in the range of from 2.5 to 8.0 molar equivalents; the presence of methanol, wherein the methanol is present in an amount in the range of from 0.75 to 5.0 molar equivalents, wherein said methanol is added as a separate component, wherein the methanol is added in one or more portions or aliquots, and wherein the amount of methanol is independent of any methanol which may be later introduced into the reaction mixture;
in the presence of sulfuric acid, wherein the sulfuric acid is present in an amount in the range of from 1.05 to

2.5 molar equivalents;
at a temperature in the range of from room temperature to 90°C; to yield a mixture comprising

a. di-threo-methylphenidate sulfate; and
b. an impurity compound selected from the group consisting of a sulfate salt of the impurity compound of formula (IMP-A), a sulfate salt of the impurity compound of formula (IMP-B), a sulfate salt of the impurity compound of formula (IMP-C), a sulfate salt of the impurity compound of formula (IMP-D), and mixtures thereof;

C. reacting the mixture prepared in Step B with a base; and adding an organic solvent; to yield a mixture of di-threo-methylphenidate free base and the impurity compound selected from the group consisting of the freebase impurity compound of formula (IMP-A), the freebase impurity compound of formula (IMP-B), the freebase impurity compound of formula (IMP-C), the freebase impurity compound of formula (IMP-D), and mixtures thereof; in a biphasic mixture comprising an aqueous phase and an organic phase;
D. separating the phases of the biphasic mixture one from the other; wherein the di-threo-methylphenidate free base, and the impurity compound selected from the group consisting of the freebase impurity compound of formula (IMP-A), the freebase impurity compound of formula (IMP-B), the freebase impurity compound of formula (IMP-C), the freebase impurity compound of formula (IMP-D), and mixtures thereof, are present in the organic phase.

**8.** A process as in Claim 7, wherein the acid of Step 3 is hydrochloric acid.

**9.** A process as in Claim 7, wherein the amount of the impurity selected from the group consisting of the pharmaceutically acceptable salt of the impurity compound of formula (IMP-A), the pharmaceutically acceptable salt of the impurity compound of formula (IMP-B) the pharmaceutically acceptable salt of the impurity compound of formula (IMP-C), the pharmaceutically acceptable salt of the impurity compound of formula (IMP-D), and mixtures thereof; is present in an amount less than about 0.05 area %.

**10.** A process for removing or reducing the amount of an impurity compound of formulas (IMP-A)

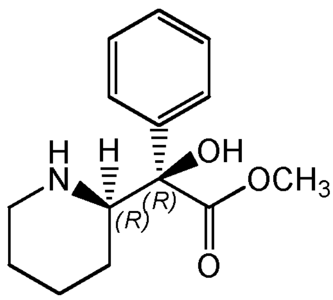

(IMP-A)

or a salt thereof, an impurity compound of formula (IMP-B)

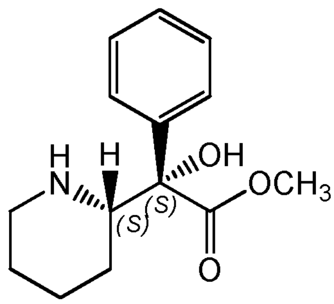

(IMP-B),

or a salt thereof, an impurity compound of formula (IMP-C)

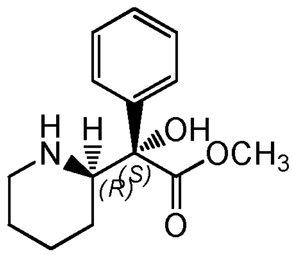

(IMP-C)

or a salt thereof, an impurity compound of formula (IMP-D)

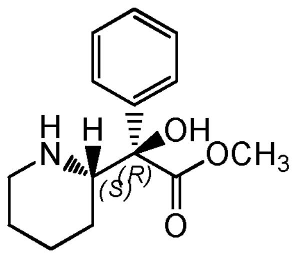

(IMP-D)

or a salt thereof, or mixtures thereof, from compound of formula (I)

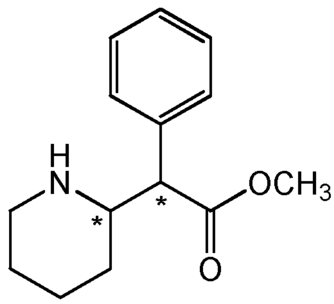

(I)

or a stereoisomer, mixture of stereoisomers or pharmaceutically acceptable salt thereof, comprising the steps of:

a. providing a composition comprising:

i. a compound of formula (I-A)

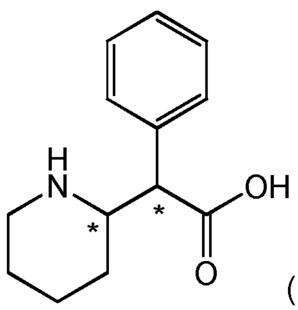

(I-A);

or stereoisomer, mixture of stereoisomers or pharmaceutically acceptable salt thereof; and

ii. an impurity compound selected from the group consisting of an impurity compound of formula (IMP-1)

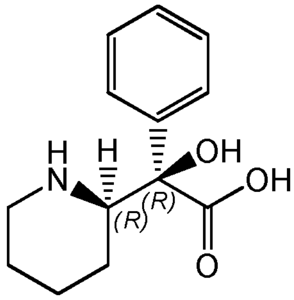

(IMP-1),

an impurity compound of formula (IMP-2)

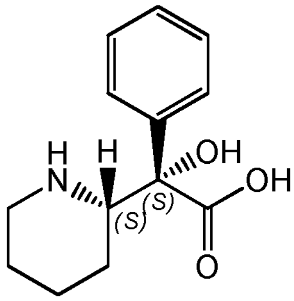

(IMP-2),

an impurity compound of formula (IMP-11)

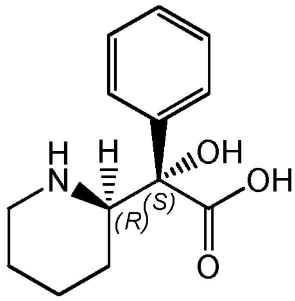

(IMP-11)

an impurity compound of formula (IMP-12)

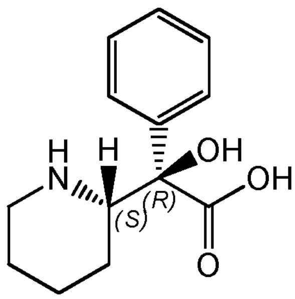

(IMP-12)

or mixtures thereof.

b. reacting the composition of Step a. with dimethylcarbonate to form a reaction mixture; wherein the dimethylcarbonate is present in an amount in the range of from 2.5 to 8.0 molar equivalents;

c. adding methanol to the reaction mixture; wherein the methanol is present in an amount in the range of from 0.75 to 5.0 molar equivalents; wherein said methanol is added as a separate component; and wherein the amount of methanol added, in one or more portions or aliquots, is independent of any methanol which may be later introduced into the reaction mixture;

d. adding sulfuric acid to the reaction mixture; wherein the sulfuric acid is present in an amount in the range of from 1.05 to 2.5 molar equivalents;

wherein the reaction mixture is at a temperature in the range of from room temperature to 90°C; to yield the corresponding compound of formula (I-S);

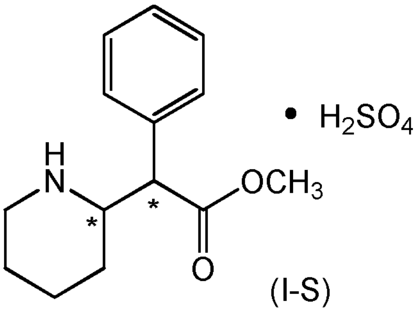

(I-S)

in a mixture with a corresponding sulfate salt of the impurity compound of formula (IMP-A), a corresponding sulfate salt of the impurity compound of formula (IMP-B), a corresponding sulfate salt of the impurity compound of formula (IMP-C), a corresponding sulfate salt of the impurity compound of formula (IMP-D), or mixtures thereof;

e. reacting the mixture of Step d. with a base; and then adding an organic solvent; to yield the corresponding compound of formula (I) as a free base, in a mixture with a freebase impurity compound of formula (IMP-A), a freebase impurity compound of formula (IMP-B), a freebase impurity compound of formula (IMP-C), a freebase impurity compound of formula (IMP-D), and mixtures thereof, in a biphasic mixture comprising an aqueous phase and an organic phase;

f. separating the phases of the biphasic mixture one from the other; wherein: i. the compound of formula (I) and ii. the freebase impurity compound of formula (IMP-A), the freebase impurity compound of formula (IMP-B), the freebase impurity compound of formula (IMP-C), the freebase impurity compound of formula (IMP-D), and mixtures thereof, are present in the organic phase;

g. reacting an acid with the organic layer comprising: i. the compound of formula (I) and ii. the impurity compound of formula (IMP-A), the impurity compound of formula (IMP-B), the impurity compound of formula (IMP-C), the impurity compound of formula (IMP-D), and mixtures thereof; in the presence of water; wherein the water is present in an amount in the range of from 0.05 wt/wt equiv. to 1.0 wt/wt equiv.; to yield a corresponding acid addition salt of the compound of formula (I) in a mixture with a corresponding acid addition salt of the impurity compound of formula (IMP-A), a corresponding acid addition salt of the impurity compound of formula (IMP-B), a corresponding acid addition salt of the impurity compound of formula (IMP-C), a corresponding acid addition salt of the impurity compound of formula (IMP-D), and mixtures thereof wherein the corresponding acid addition salt of the impurity compound of formula (IMP-A), the corresponding acid addition salt of the impurity compound of formula (IMP-B), the corresponding acid addition salt of the impurity compound of formula (IMP-C), the corresponding acid addition salt of the impurity compound of formula (IMP-D), and mixtures thereof are extracted into the water; and

h. separating the water comprising the corresponding acid addition salt of the impurity compound of formula (IMP-A), the corresponding acid addition salt of the impurity compound of formula (IMP-B), the corresponding acid addition salt of the impurity compound of formula (IMP-C), the corresponding acid addition salt of the impurity compound of formula (IMP-D), and mixtures thereof from the corresponding acid addition salt of compound of formula (I) from the mixture of Step g.

## Patentansprüche

1. Verfahren zur Umkristallisation von Di-threo-Methylphenidat-Hydrochlorid; wobei das umkristallisierte Di-threo-Methylphenidat-Hydrochlorid insgesamt weniger als 0,1 Flächen% einer Verunreinigungsverbindung umfasst, ausgewählt aus der Gruppe bestehend aus einer Verunreinigungsverbindung der Formel (IMP-5)

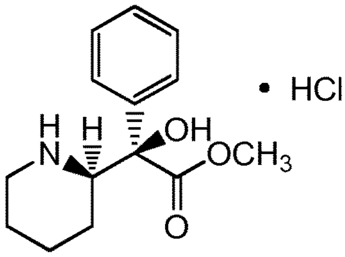

(IMP-5),

einer Verunreinigungsverbindung der Formel (IMP-6)

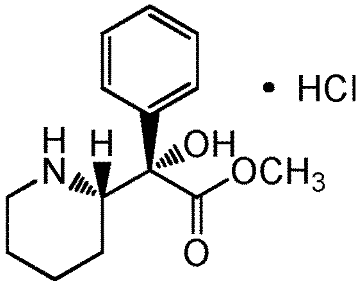

(IMP-6),

einer Verunreinigungsverbindung der Formel (IMP-15)

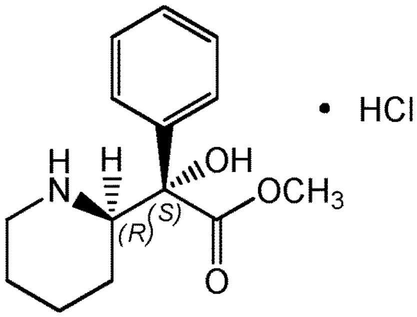

(IMP-15),

einer Verunreinigungsverbindung der Formel (IMP-16)

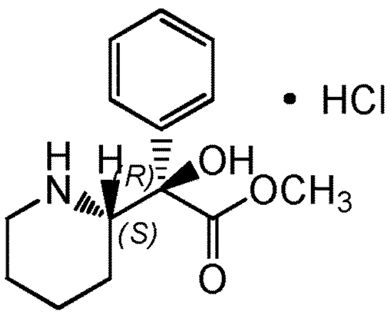

(IMP-16),

und Mischungen davon; umfassend die Schritte zum

(a) Auflösen von Di-threo-Methylphenidat-Hydrochlorid in einer Mischung aus einem organischen Lösemittel und Wasser; wobei das organische Lösemittel ausgewählt ist aus der Gruppe bestehend aus Ethanol und Isopropanol; und wobei das Wasser in einer Menge im Bereich von 0,1 Gew./Gew.-Äquiv. bis 0,6 Gew./Gew.-Äquiv. vorhanden ist;
(b) Erwärmen der Mischung auf etwa Rückflusstemperatur;

(c) gegebenenfalls Abdestillieren von zwischen 20 Vol.-% und 65 Vol.-% der Mischung aus einem organischen Lösemittel und Wasser;
(c) Abkühlen der Mischung auf eine Temperatur von weniger als etwa Raumtemperatur; und
(d) Isolieren des Di-threo-Methylphenidats als einen Feststoff.

**2.** Verfahren nach Anspruch 1, wobei das organische Lösemittel Ethanol ist; und wobei das Wasser in einer Menge von 0,12 Gew./Gew. Äquiv. vorhanden ist.

**3.** Verfahren nach Anspruch 1, wobei das umkristallisierte Di-threo-Methylphenidat-Hydrochlorid insgesamt weniger als 0,05 Flächen% der Verunreinigungsverbindung umfasst, ausgewählt aus der Gruppe bestehend aus der Verunreinigungsverbindung der Formel (IMP-5), der Verunreinigungsverbindung der Formel (IMP-6), der Verunreinigungsverbindung der Formel (IMP-15), der Verunreinigungsverbindung der Formel (IMP-16) und Mischungen davon.

**4.** Verfahren zum Entfernen oder Reduzieren der Menge einer oder mehrerer einer Verunreinigungsverbindung der Formel (IMP-A)

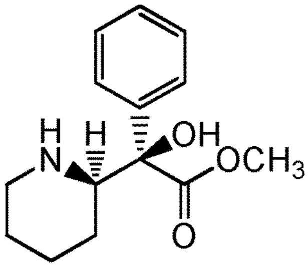

(IMP-A)

oder eines Salzes davon, einer Verunreinigungsverbindung der Formel (IMP-B)

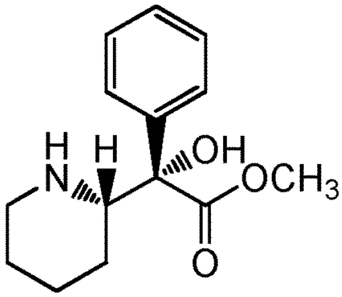

(IMP-B)

oder eines Salzes davon, einer Verunreinigungsverbindung der Formel (IMP-C)

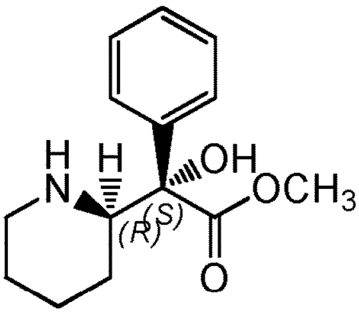

(IMP-C)

oder eines Salzes davon oder einer Verunreinigungsverbindung der Formel (IMP-D)

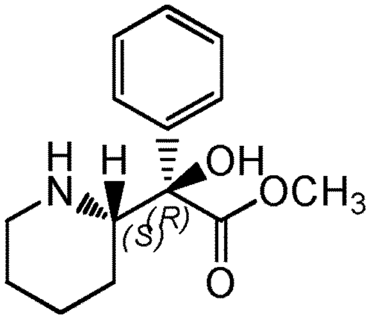

(IMP-D)

oder eines Salzes davon,
aus einem pharmazeutisch akzeptablen Salz von Di-threo-Methylphenidat; umfassend die Schritte zum

I. Bereitstellen einer Mischung aus

i. Di-threo-Methylphenidat-Sulfat;
ii. einer Verunreinigungsverbindung, ausgewählt aus der Gruppe bestehend aus einem Sulfatsalz der Verunreinigungsverbindung der Formel (IMP-A), einem Sulfatsalz der Verunreinigungsverbindung der Formel (IMP-B), einem Sulfatsalz der Verunreinigungsverbindung der Formel (IMP-C), einem Sulfatsalz der Verunreinigungsverbindung der Formel (IMP-D) und Mischungen davon;

II. Zugeben eines organischen Lösemittels zu der Mischung aus Schritt 1, um eine zweite Mischung zu ergeben; und III. Umsetzen der zweiten Mischung aus Schritt 2 mit einer Säure; in Gegenwart von Wasser; wobei das Wasser in einer Menge im Bereich von 0,05 Gew./Gew. Äquiv. bis 1,0 Gew./Gew. Äquiv. vorhanden ist;
um ein Produkt zu ergeben, das ein entsprechendes pharmazeutisch akzeptables Salz von Di-threo-Methylphenidat in einer Mischung mit einer entsprechenden Verunreinigungsverbindung umfasst, ausgewählt aus der Gruppe bestehend aus einem entsprechenden pharmazeutisch akzeptablen Salz der Verunreinigungsverbindung der Formel (IMP-A), einem entsprechenden pharmazeutisch akzeptablen Salz der Verunreinigungsverbindung der Formel (IMP-B), einem entsprechenden pharmazeutisch akzeptablen Salz der Verunreinigungsverbindung der Formel (IMP-C), einem entsprechenden pharmazeutisch akzeptablen Salz der Verunreinigungsverbindung der Formel (IMP-D) und Mischungen davon;
wobei die Mischung aus Schritt 1 gemäß einem Verfahren hergestellt wird, umfassend die Schritte zum:

A. Bereitstellen einer Zusammensetzung, umfassend

i. Di-threo-Ritalinsäure oder ein pharmazeutisch akzeptables Salz davon; und
ii. einen oder mehrere eines Verunreinigungsvorläufers, ausgewählt aus der Gruppe bestehend aus einer Verunreinigungsverbindung der Formel (IMP-1)

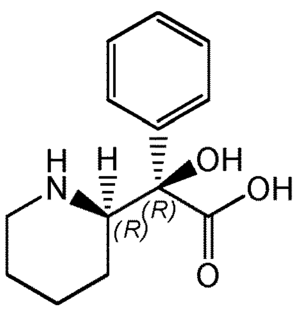

(IMP-1)

oder einem Salz davon, einer Verunreinigungsverbindung der Formel (IMP-2)

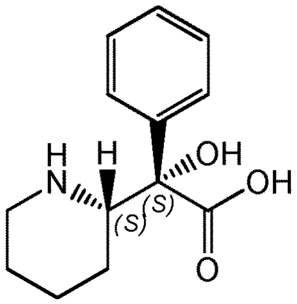

(IMP-2)

oder einem Salz davon, einer Verunreinigungsverbindung der Formel (IMP-11)

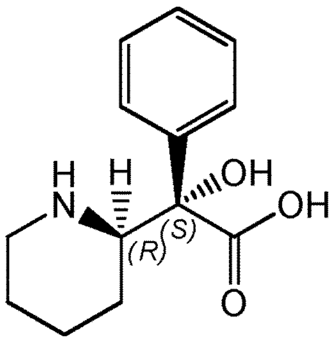

(IMP-11)

oder einem Salz davon und einer Verunreinigungsverbindung der Formel (IMP-12)

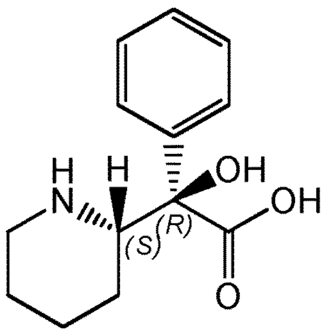

(IMP-12)

oder einem Salz davon;
B. Umsetzen der Zusammensetzung aus Schritt A mit Dimethylcarbonat, wobei das Dimethylcarbonat in einer Menge im Bereich von 2,5 bis 8,0 Moläquivalenten vorhanden ist;
in Gegenwart von Methanol, wobei das Methanol in einer Menge im Bereich von 0,75 bis 5,0 Moläquivalenten vorhanden ist, wobei das Methanol als separate Komponente zugegeben wird, wobei das Methanol in einer oder mehreren Portionen oder aliquoten Teilen zugegeben wird, und wobei die Menge an Methanol unabhängig von jedem Methanol ist, das später in die Reaktionsmischung eingeführt wird;
in Gegenwart von Schwefelsäure, wobei die Schwefelsäure in einer Menge im Bereich von 1,05 bis 2,5 Moläquivalenten vorhanden ist;
bei einer Temperatur im Bereich von etwa Raumtemperatur bis etwa 90 °C; um eine Mischung zu ergeben, umfassend:

i. Di-threo-Methylphenidat-Sulfat; und
ii. die Verunreinigungsverbindung, ausgewählt aus der Gruppe bestehend aus dem Sulfatsalz der Verunreinigungsverbindung der Formel (IMP-A), dem Sulfatsalz der Verunreinigungsverbindung der Formel (IMP-B), dem Sulfatsalz der Verunreinigungsverbindung der Formel (IMP-C), dem Sulfatsalz

der Verunreinigungsverbindung der Formel (IMP-D) und Mischungen davon.

**5.** Verfahren nach Anspruch 4, wobei die Säure aus Schritt 3 Chlorwasserstoffsäure ist.

**6.** Verfahren nach Anspruch 4, wobei die Menge der Verunreinigung, ausgewählt aus der Gruppe bestehend aus dem pharmazeutisch akzeptablen Salz der Verunreinigungsverbindung der Formel (IMP-A), dem pharmazeutisch akzeptablen Salz der Verunreinigungsverbindung der Formel (IMP-B), dem pharmazeutisch akzeptablen Salz der Verunreinigungsverbindung der Formel (IMP-C), dem pharmazeutisch akzeptablen Salz der Verunreinigungsverbindung der Formel (IMP-D) und Mischungen davon; in einer Menge von weniger als 0,05 Flächen% vorhanden ist.

**7.** Verfahren zum Entfernen oder Reduzieren der Menge einer oder mehrerer einer Verunreinigungsverbindung der Formel (IMP-A)

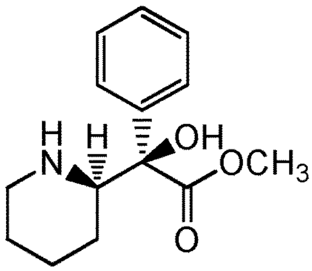

(IMP-A)

oder eines Salzes davon, einer Verunreinigungsverbindung der Formel (IMP-B)

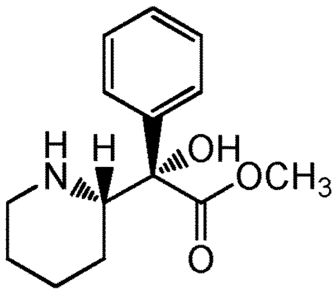

(IMP-B)

oder eines Salzes davon, einer Verunreinigungsverbindung der Formel (IMP-C)

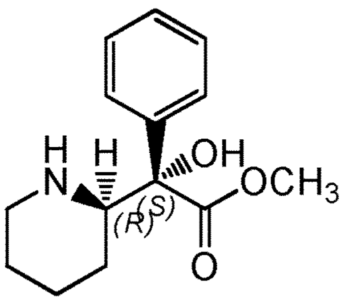

(IMP-C)

oder eines Salzes davon, oder einer Verunreinigungsverbindung der Formel (IMP-D)

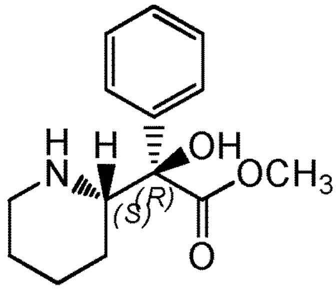

(IMP-D)

oder eines Salzes davon; aus Di-threo-Methylphenidat oder einem pharmazeutisch akzeptablen Salz von Di-threo-Methylphenidat;
umfassend die Schritte zum

I. Bereitstellen einer Mischung aus

i. Di-threo-Methylphenidat-Sulfat;
ii. einer Verunreinigungsverbindung, ausgewählt aus der Gruppe bestehend aus einer Freie-Base-Verunreinigungsverbindung der Formel (IMP-A), einer Freie-Base-Verunreinigungsverbindung der Formel (IMP-B), einer Freie-Base-Verunreinigungsverbindung der Formel (IMP-C), einer Freie-Base-Verunreinigungsverbindung der Formel (IMP-D) und Mischungen davon;
iii. einer organischen Phase, umfassend ein organisches Lösemittel;

II. Umsetzen der Mischung aus Schritt 1 mit einer Säure; in Gegenwart von Wasser; wobei das Wasser in einer Menge im Bereich von 0,05 Gew./Gew. Äquiv. bis 1,0 Gew./Gew. Äquiv. vorhanden ist;
um ein Produkt zu ergeben, das ein entsprechendes pharmazeutisch akzeptables Salz von Di-threo-Methylphenidat in einer Mischung mit einer entsprechenden Verunreinigungsverbindung umfasst, ausgewählt aus der Gruppe bestehend aus einem entsprechenden pharmazeutisch akzeptablen Salz der Verunreinigungsverbindung der Formel (IMP-A), einem entsprechenden pharmazeutisch akzeptablen Salz der Verunreinigungsverbindung der Formel (IMP-B), einem entsprechenden pharmazeutisch akzeptablen Salz der Verunreinigungsverbindung der Formel (IMP-C), einem entsprechenden pharmazeutisch akzeptablen Salz der Verunreinigungsverbindung der Formel (IMP-D) und Mischungen davon;
wobei die Mischung aus Schritt 1 gemäß einem Verfahren hergestellt wird, umfassend die Schritte zum:

A. Bereitstellen einer Zusammensetzung, umfassend

a. Di-threo-Ritalinsäure oder ein pharmazeutisch akzeptables Salz davon; und
b. einen Verunreinigungsvorläufer, ausgewählt aus der Gruppe bestehend aus einer Verunreinigungsverbindung der Formel (IMP-1)

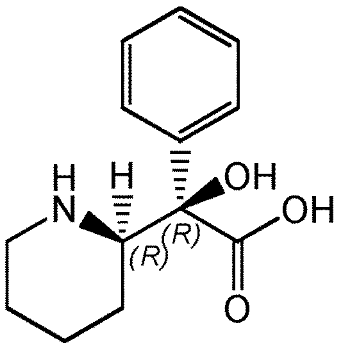

(IMP-1)

oder einem Salz davon, einer Verunreinigungsverbindung der Formel (IMP-2)

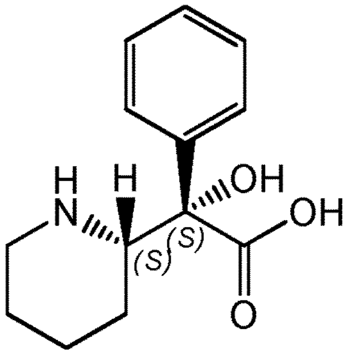

(IMP-2)

oder einem Salz davon, einer Verunreinigungsverbindung der Formel (IMP-11)

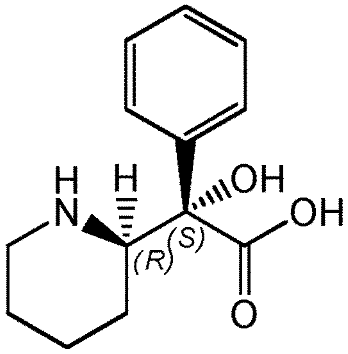

(IMP-11)

oder einem Salz davon, einer Verunreinigungsverbindung der Formel (IMP-12)

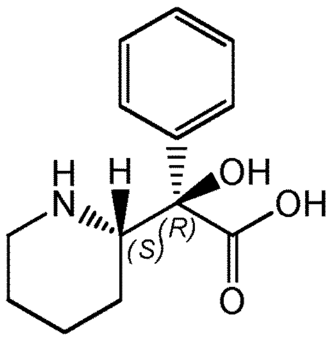

(IMP-12)

oder einem Salz davon und Mischungen davon;

B. Umsetzen der Zusammensetzung aus Schritt A mit Dimethylcarbonat, wobei das Dimethylcarbonat in einer Menge im Bereich von 2,5 bis 8,0 Moläquivalenten vorhanden ist;

die Gegenwart von Methanol, wobei das Methanol in einer Menge im Bereich von 0,75 bis 5,0 Moläquivalenten vorhanden ist,

wobei das Methanol als separate Komponente zugegeben wird, wobei das Methanol in einer oder mehreren Portionen oder aliquoten Teilen zugegeben wird, und wobei die Menge an Methanol unabhängig von jedem Methanol ist, das das später in die Reaktionsmischung eingeführt wird;

in Gegenwart von Schwefelsäure, wobei die Schwefelsäure in einer Menge im Bereich von 1,05 bis 2,5 Moläquivalenten vorhanden ist;

bei einer Temperatur im Bereich von Raumtemperatur bis 90 °C; um eine Mischung zu ergeben, umfassend:

a. Di-threo-Methylphenidat-Sulfat; und

b. eine Verunreinigungsverbindung, ausgewählt aus der Gruppe bestehend aus einem Sulfatsalz der Verunreinigungsverbindung der Formel (IMP-A), einem Sulfatsalz der Verunreinigungsverbindung der

Formel (IMP-B), einem Sulfatsalz der Verunreinigungsverbindung der Formel (IMP-C), einem Sulfatsalz der Verunreinigungsverbindung der Formel (IMP-D) und Mischungen davon;

C. Umsetzen der in Schritt B hergestellten Mischung mit einer Base; und Zugeben eines organischen Lösemittels; um eine Mischung aus Di-threo-Methylphenidat-Freie-Base und der Verunreinigungsverbindung, ausgewählt aus der Gruppe bestehend aus der Freie-Base-Verunreinigungsverbindung der Formel (IMP-A), der Freie-Base-Verunreinigungsverbindung der Formel (IMP-B), der Freie-Base-Verunreinigungsverbindung der Formel (IMP-C), der Freie-Base-Verunreinigungsverbindung der Formel (IMP-D), in einer zweiphasigen Mischung zu ergeben, umfassend eine wässrige Phase und eine organische Phase;
D. Trennen der Phasen der zweiphasigen Mischung voneinander; wobei die Di-threo-Methylphenidat-Freie-Base und die Verunreinigungsverbindung, ausgewählt aus der Gruppe bestehend aus der Freie-Base-Verunreinigungsverbindung der Formel (IMP-A), der Freie-Base-Verunreinigungsverbindung der Formel (IMP-B), der Freie-Base-Verunreinigungsverbindung der Formel (IMP-C), der Freie-Base-Verunreinigungsverbindung der Formel (IMP-D) und Mischungen davon in der organischen Phase vorhanden sind.

**8.** Verfahren nach Anspruch 7, wobei die Säure aus Schritt 3 Chlorwasserstoffsäure ist.

**9.** Verfahren nach Anspruch 7, wobei die Menge der Verunreinigung, ausgewählt aus der Gruppe bestehend aus dem pharmazeutisch akzeptablen Salz der Verunreinigungsverbindung der Formel (IMP-A), dem pharmazeutisch akzeptablen Salz der Verunreinigungsverbindung der Formel (IMP-B), dem pharmazeutisch akzeptablen Salz der Verunreinigungsverbindung der Formel (IMP-C), dem pharmazeutisch akzeptablen Salz der Verunreinigungsverbindung der Formel (IMP-D) und Mischungen davon; in einer Menge von weniger als etwa 0,05 Flächen% vorhanden ist.

**10.** Verfahren zum Entfernen oder Reduzieren der Menge einer Verunreinigungsverbindung der Formeln (IMP-A)

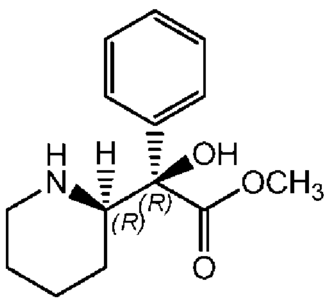

(IMP-A)

oder eines Salzes davon, einer Verunreinigungsverbindung der Formel (IMP-B)

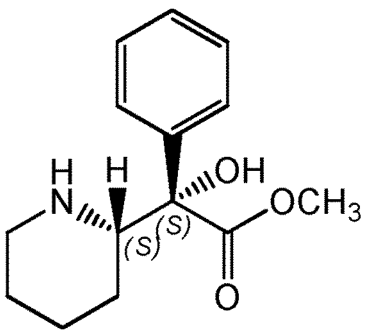

(IMP-B),

oder eines Salzes davon, einer Verunreinigungsverbindung der Formel (IMP-C)

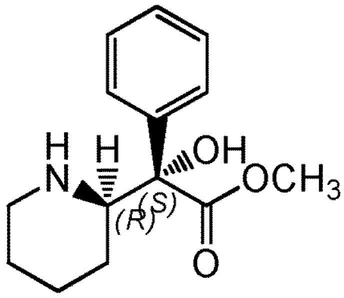

(IMP-C)

oder eines Salzes davon, einer Verunreinigungsverbindung der Formel (IMP-D)

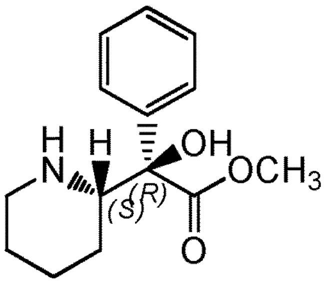

(IMP-D)

oder eines Salzes davon oder Mischungen davon aus einem Verfahren zum Herstellen einer Verbindung der Formel (I)

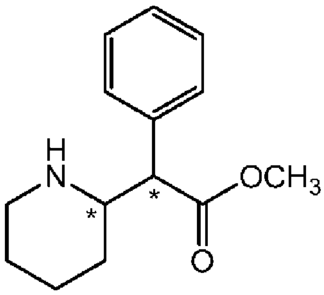

(I)

oder eines Stereoisomers, einer Mischung aus Stereoisomeren oder einem pharmazeutisch akzeptablen Salz davon, umfassend die Schritte zum:

a. Bereitstellen einer Zusammensetzung, umfassend

i. eine Verbindung der Formel (I-A)

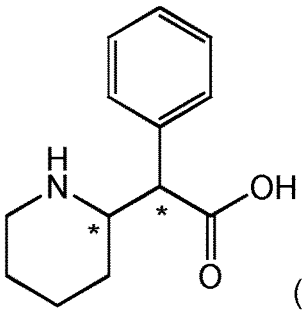

(I-A);

oder ein Stereoisomer, eine Mischung aus Stereoisomeren oder ein pharmazeutisch akzeptables Salz

davon; und

ii. eine Verunreinigungsverbindung, ausgewählt aus der Gruppe bestehend aus einer Verunreinigungsverbindung der Formel (IMP-1)

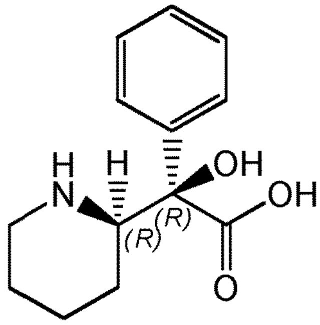

(IMP-1),

einer Verunreinigungsverbindung der Formel (IMP-2)

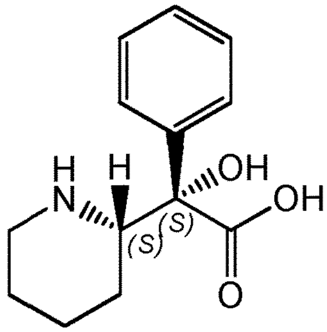

(IMP-2),

einer Verunreinigungsverbindung der Formel (IMP-11)

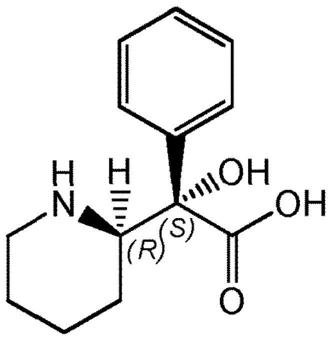

(IMP-11)

einer Verunreinigungsverbindung der Formel (IMP-12)

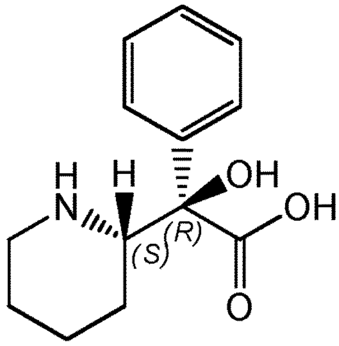

(IMP-12)

oder Mischungen davon.

b. Umsetzen der Zusammensetzung aus Schritt a. mit Dimethylcarbonat, um eine Reaktionsmischung zu bilden; wobei das Dimethylcarbonat in einer Menge im Bereich von 2,5 bis 8,0 Moläquivalenten vorhanden ist;
c. Zugeben von Methanol zu der Reaktionsmischung; wobei das Methanol in einer Menge im Bereich von 0,75 bis 5,0 Moläquivalenten vorhanden ist; wobei das Methanol als separate Komponente zugegeben wird; und wobei die Menge an in einer oder mehreren Portionen oder aliquoten Teilen zugegebenem Methanol unabhängig von jedem Methanol ist, das später in die Reaktionsmischung eingeführt wird;
d. Zugeben von Schwefelsäure zu der Reaktionsmischung; wobei die Schwefelsäure in einer Menge im Bereich von 1,05 bis 2,5 Moläquivalenten vorhanden ist;
wobei die Reaktionsmischung eine Temperatur im Bereich von Raumtemperatur bis 90 °C aufweist; um die entsprechende Verbindung der Formel (I-S):

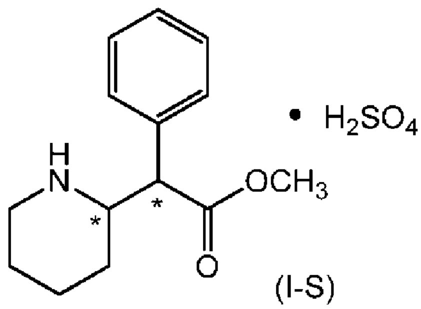

(I-S) ,

in einer Mischung mit einem entsprechenden Sulfatsalz der Verunreinigungsverbindung der Formel (IMP-A), einem entsprechenden Sulfatsalz der Verunreinigungsverbindung der Formel (IMP-B), einem entsprechenden Sulfatsalz der Verunreinigungsverbindung der Formel (IMP-C), einem entsprechenden Sulfatsalz der Verunreinigungsverbindung der Formel (IMP-D) oder Mischungen davon zu ergeben;
e. Umsetzen der Mischung aus Schritt d. mit einer Base; und dann Zugeben eines organischen Lösemittels; um die entsprechende Verbindung der Formel (I) als eine freie Base in einer Mischung mit einer Freie-Base-Verunreinigungsverbindung der Formel (IMP-A), einer Freie-Base-Verunreinigungsverbindung der Formel (IMP-B), einer Freie-Base-Verunreinigungsverbindung der Formel (IMP-C), einer Freie-Base-Verunreinigungsverbindung der Formel (IMP-D) und Mischungen davon in einer zweiphasigen Mischung zu ergeben, die eine wässrige Phase und eine organische Phase umfasst;
f. Trennen der Phasen der zweiphasigen Mischung voneinander; wobei: i. die Verbindung der Formel (I) und ii. die Freie-Base-Verunreinigungsverbindung der Formel (IMP-A), die Freie-Base-Verunreinigungsverbindung der Formel (IMP-B), die Freie-Base-Verunreinigungsverbindung der Formel (IMP-C), die Freie-Base-Verunreinigungsverbindung der Formel (IMP-D) und Mischungen davon in der organischen Phase vorhanden sind;
g. Umsetzen einer Säure mit der organischen Schicht, umfassend: i. die Verbindung der Formel (I) und ii. die Verunreinigungsverbindung der Formel (IMP-A), die Verunreinigungsverbindung der Formel (IMP-B), die Verunreinigungsverbindung der Formel (IMP-C), die Verunreinigungsverbindung der Formel (IMP-D) und Mischungen davon; in Gegenwart von Wasser; wobei das Wasser in einer Menge im Bereich von 0,05 Gew./Gew. Äquiv. bis 1,0 Gew./Gew. Äquiv. vorhanden ist; um ein entsprechendes Säureadditionssalz der Verbindung der Formel (I) in einer Mischung mit einem entsprechenden Säureadditionssalz der Verunreinigungsverbindung der Formel

(IMP-A), einem entsprechenden Säureadditionssalz der Verunreinigungsverbindung der Formel (IMP-B), einem entsprechenden Säureadditionssalz der Verunreinigungsverbindung der Formel (IMP-C), einem entsprechenden Säureadditionssalz der Verunreinigungsverbindung der Formel (IMP-D) und Mischungen davon zu ergeben, wobei das entsprechende Säureadditionssalz der Verunreinigungsverbindung der Formel (IMP-A), das entsprechende Säureadditionssalz der Verunreinigungsverbindung der Formel (IMP-B), das entsprechende Säureadditionssalz der Verunreinigungsverbindung der Formel (IMP-C), das entsprechende Säureadditionssalz der Verunreinigungsverbindung der Formel (IMP-D) und Mischungen davon in das Wasser extrahiert werden; und

h. Trennen des Wassers, umfassend das entsprechende Säureadditionssalz der Verunreinigungsverbindung der Formel (IMP-A), das entsprechende Säureadditionssalz der Verunreinigungsverbindung der Formel (IMP-B), das entsprechende Säureadditionssalz der Verunreinigungsverbindung der Formel (IMP-C), das entsprechende Säureadditionssalz der Verunreinigungsverbindung der Formel (IMP-D) und Mischungen davon, von dem entsprechenden Säureadditionssalz der Verbindung der Formel (I) aus der Mischung aus Schritt g.

## Revendications

**1.** Procédé de recristallisation du chlorhydrate de di-thréo-phénidate de méthyle ;
où le chlorhydrate de di-thréo-phénidate de méthyle recristallisé comprend un total de moins de 0,1 % en surface d'une impureté qui est un composé choisi dans le groupe constitué par
une impureté qui est un composé de formule (IMP-5)

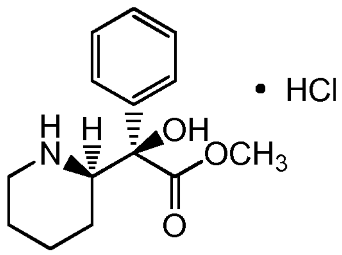

(IMP-5),

une impureté qui est un composé de formule (IMP-6)

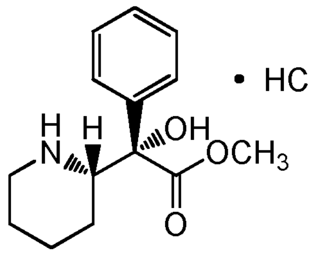

(IMP-6),

une impureté qui est un composé de formule (IMP-15)

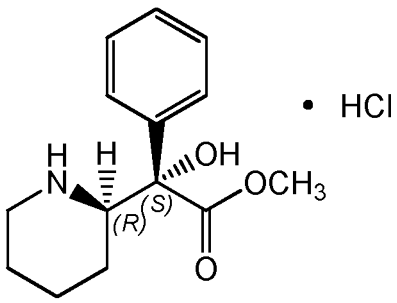

(IMP-15),

une impureté qui est un composé de formule (IMP-16)

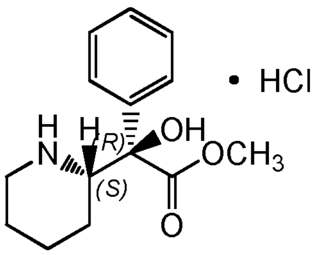

(IMP-16),

ainsi que leurs mélanges ; comprenant les étapes consistant à

(a) dissoudre le chlorhydrate de di-thréo-phénidate de méthyle dans un mélange d'un solvant organique et d'eau ; où le solvant organique est choisi dans le groupe constitué par l'éthanol et l'isopropanol ; et où l'eau est présente dans une proportion comprise dans l'intervalle allant de 0,1 équivalent massique à 0,6 équivalent massique ;
(b) chauffer le mélange jusqu'à une température environ égale à la température de reflux ;
(c) éventuellement éliminer par distillation entre 20 % et 65 % en volume du mélange d'un solvant organique et d'eau ;
(c) refroidir le mélange jusqu'à une température inférieure à environ la température ambiante ; et
(d) isoler le di-thréo-phénidate de méthyle sous forme d'un solide.

**2.** Procédé selon la Revendication 1, où le solvant organique est l'éthanol ; et où l'eau est présente dans une proportion de 0,12 équivalent massique.

**3.** Procédé selon la Revendication 1, où le chlorhydrate de di-thréo-phénidate de méthyle recristallisé comprend un total de moins de 0,05 % en surface de l'impureté qui est un composé choisi dans le groupe constitué par l'impureté qui est un composé de formule (IMP-5), l'impureté qui est un composé de formule (IMP-6), l'impureté qui est un composé de formule (IMP-15), l'impureté qui est un composé de formule (IMP-16), ainsi que leurs mélanges.

**4.** Procédé d'élimination et de réduction de la proportion d'un ou de plusieurs des membres du groupe constitué par une impureté qui est un composé de formule (IMP-A)

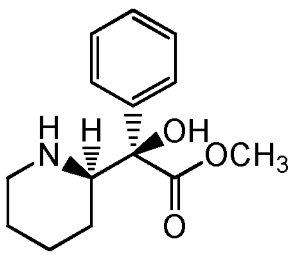

(IMP-A)

ou un sel de celui-ci, une impureté qui est un composé de formule (IMP-B)

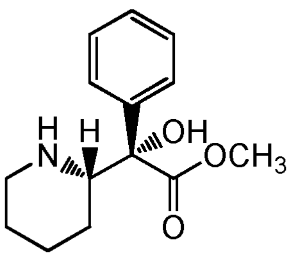

(IMP-B)

ou un sel de celui-ci, une impureté qui est un composé de formule (IMP-C)

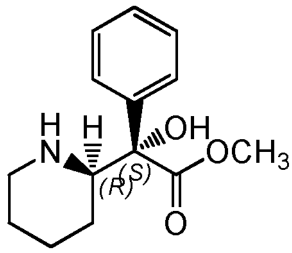

(IMP-C)

ou un sel de celui-ci, ou une impureté qui est un composé de formule (IMP-D)

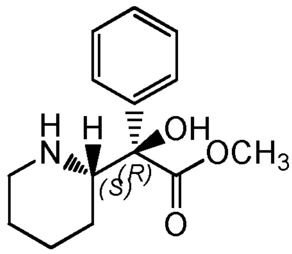

(IMP-D)

ou un sel de celui-ci,
d'avec un sel pharmaceutiquement acceptable de di-thréo-phénidate de méthyle ;
comprenant les étapes consistant à

I. se munir d'un mélange

i. de sulfate de di-thréo-phénidate de méthyle ;
ii. d'une impureté qui est un composé choisi dans le groupe constitué par un sel de sulfate de l'impureté qui est un composé de formule (IMP-A), un sel de sulfate de l'impureté qui est un composé de formule (IMP-B), un sel de sulfate de l'impureté qui est un composé de formule (IMP-C), un sel de sulfate de l'impureté qui est un composé de formule (IMP-D) et leurs mélanges ;

II. ajouter un solvant organique au mélange de l'Étape 1 pour obtenir un deuxième mélange ; et
III. faire réagir le deuxième mélange de l'Étape 2 avec un acide ; en présence d'eau ; où l'eau est présente dans une proportion comprise dans l'intervalle allant de 0,05 équivalent massique à 1,0 équivalent massique ; pour obtenir un produit comprenant un sel pharmaceutiquement acceptable correspondant de di-thréo-phénidate de méthyle dans un mélange avec une impureté correspondante qui est un composé choisi dans le groupe constitué par un sel pharmaceutiquement acceptable correspondant de l'impureté qui est un composé de formule (IMP-A), un sel pharmaceutiquement acceptable correspondant de l'impureté qui est un composé de formule (IMP-B), un sel pharmaceutiquement acceptable correspondant de l'impureté qui est un composé de formule (IMP-C), un sel pharmaceutiquement acceptable correspondant de l'impureté qui est un composé de formule (IMP-D) et leurs mélanges ;
où le mélange de l'Étape 1 est préparé selon un procédé comprenant les étapes consistant à :

A. se munir d'une composition comprenant

i. de l'acide di-thréo-ritalinique ou l'un des sels pharmaceutiquement acceptables de celui-ci ; et
ii. un ou plusieurs des membres du groupe constitué par un précurseur d'impureté choisi dans le groupe constitué par une impureté qui est un composé de formule (IMP-1)

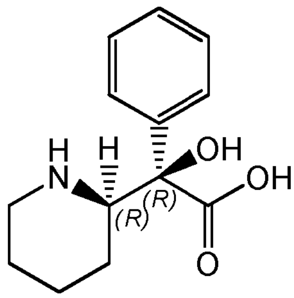

(IMP-1)

ou un sel de celui-ci, une impureté qui est un composé de formule (IMP-2)

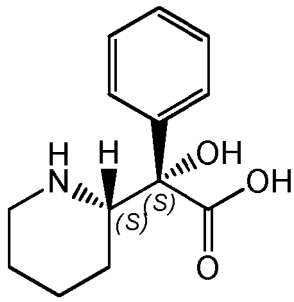

(IMP-2)

ou un sel de celui-ci, une impureté qui est un composé de formule (IMP-11)

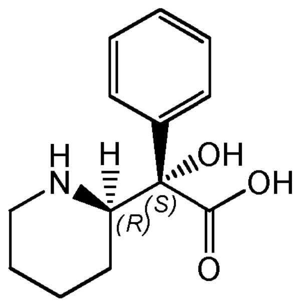

(IMP-11)

ou un sel de celui-ci, et une impureté qui est un composé de formule (IMP-12)

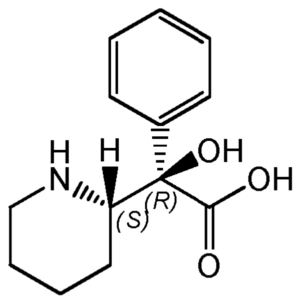

(IMP-12)

ou un sel de celui-ci ;

B. faire réagir la composition de l'Étape A avec du carbonate de diméthyle, où le carbonate de diméthyle est présent dans une proportion comprise dans l'intervalle allant de 2,5 à 8,0 équivalents molaires ;
en présence de méthanol, où le méthanol est présent dans une proportion comprise dans l'intervalle allant de 0,75 à 5,0 équivalents molaires, où ledit méthanol est ajouté sous forme de composant séparé, où le méthanol est ajouté en une ou plusieurs portions ou aliquotes, et où la proportion de méthanol est indépendante de tout méthanol pouvant être introduit ultérieurement dans le mélange réactionnel ;

en présence d'acide sulfurique, où l'acide sulfurique est présent dans une proportion comprise dans l'intervalle allant de 1,05 à 2,5 équivalents molaires ;
à une température comprise dans l'intervalle allant d'une température environ égale à la température ambiante à environ 90 °C ; pour obtenir un mélange comprenant :

i. du sulfate de di-thréo-phénidate de méthyle ; et
ii. l'impureté qui est un composé choisi dans le groupe constitué par le sel de sulfate de l'impureté qui est un composé de formule (IMP-A), le sel de sulfate de l'impureté qui est un composé de formule (IMP-B), le sel de sulfate de l'impureté qui est un composé de formule (IMP-C), le sel de sulfate de l'impureté qui est un composé de formule (IMP-D) et leurs mélanges.

**5.** Procédé selon la Revendication 4, où l'acide de l'Étape 3 est l'acide chlorhydrique.

**6.** Procédé selon la Revendication 4, où l'impureté choisie dans le groupe constitué par le sel pharmaceutiquement acceptable de l'impureté qui est un composé de formule (IMP-A), le sel pharmaceutiquement acceptable de l'impureté qui est un composé de formule (IMP-B), le sel pharmaceutiquement acceptable de l'impureté qui est un composé de formule (IMP-C), le sel pharmaceutiquement acceptable de l'impureté qui est un composé de formule (IMP-D) et leurs mélanges est présente dans une proportion inférieure à 0,05 % en surface.

**7.** Procédé d'élimination et de réduction de la proportion d'un ou de plusieurs des membres du groupe constitué par une impureté qui est un composé de formule (IMP-A)

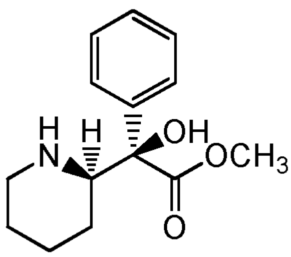

(IMP-A)

ou un sel de celui-ci, une impureté qui est un composé de formule (IMP-B)

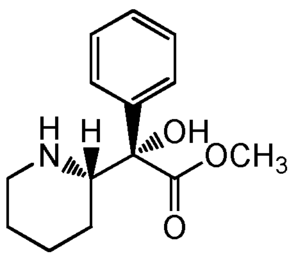

(IMP-B)

ou un sel de celui-ci, une impureté qui est un composé de formule (IMP-C)

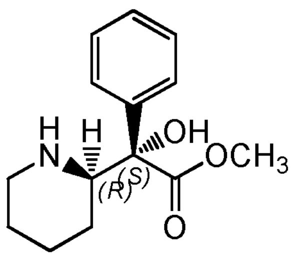

(IMP-C)

ou un sel de celui-ci, ou une impureté qui est un composé de formule (IMP-D)

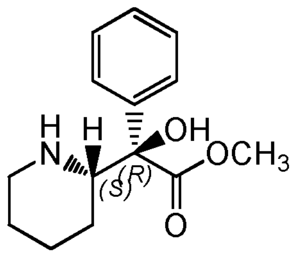

(IMP-D)

ou un sel de celui-ci ; d'avec le di-thréo-phénidate de méthyle ou l'un des sels pharmaceutiquement acceptables de di-thréo-phénidate de méthyle ; comprenant les étapes consistant à

I se munir d'un mélange

i. de sulfate de di-thréo-phénidate de méthyle ;
ii. d'une impureté qui est un composé choisi dans le groupe constitué par une impureté sous forme de base libre qui est un composé de formule (IMP-A), une impureté sous forme de base libre qui est un composé de formule (IMP-B), une impureté sous forme de base libre qui est un composé de formule (IMP-C), une impureté sous forme de base libre qui est un composé de formule (IMP-D) et leurs mélanges ;
iii d'une phase organique comprenant un solvant organique ;

II faire réagir le mélange de l'Étape 1 avec un acide ; en présence d'eau ; où l'eau est présente dans une proportion comprise dans l'intervalle allant de 0,05 équivalent massique à 1,0 équivalent massique ;
pour obtenir un produit comprenant un sel pharmaceutiquement acceptable correspondant de di-thréo-phénidate de méthyle dans un mélange avec une impureté correspondante qui est un composé choisi dans le groupe constitué par un sel pharmaceutiquement acceptable correspondant de l'impureté qui est un composé de formule (IMP-A), un sel pharmaceutiquement acceptable correspondant de l'impureté qui est un composé de formule (IMP-B), un sel pharmaceutiquement acceptable correspondant de l'impureté qui est un composé de formule (IMP-C), un sel pharmaceutiquement acceptable correspondant de l'impureté qui est un composé de formule (IMP-D) et leurs mélanges ;
où le mélange de l'Étape 1 est préparé selon un procédé comprenant les étapes consistant à :

A. se munir d'une composition comprenant

a. de l'acide di-thréo-ritalinique ou l'un des sels pharmaceutiquement acceptables de celui-ci ; et
b. un précurseur d'impureté choisi dans le groupe constitué par une impureté qui est un composé de formule (IMP-1)

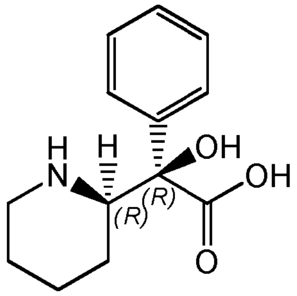

(IMP-1)

ou un sel de celui-ci, une impureté qui est un composé de formule (IMP-2)

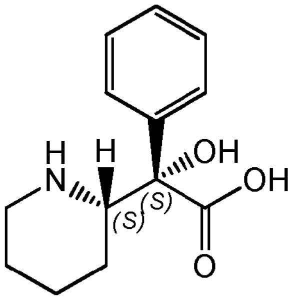

(IMP-2)

ou un sel de celui-ci, une impureté qui est un composé de formule (IMP-11)

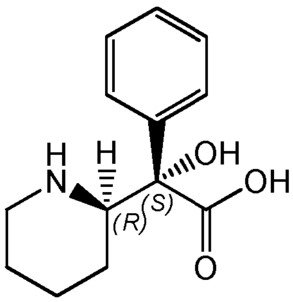

(IMP-11)

ou un sel de celui-ci, une impureté qui est un composé de formule (IMP-12)

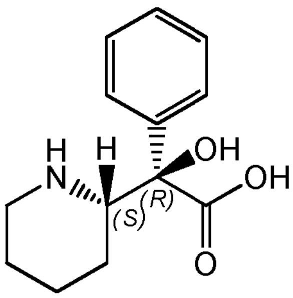

(IMP-12)

ou un sel de celui-ci, et leurs mélanges ;
B. faire réagir la composition de l'Étape A avec du carbonate de diméthyle, où le carbonate de diméthyle est présent dans une proportion comprise dans l'intervalle allant de 2,5 à 8,0 équivalents molaires ;
la présence de methanol, où le méthanol est présent dans une proportion comprise dans l'intervalle allant de 0,75 à 5,0 équivalents molaires, où ledit méthanol est ajouté sous forme de composant séparé, où le méthanol est ajouté en une ou plusieurs portions ou aliquotes, et où la proportion de méthanol est indépendante de tout méthanol pouvant être introduit ultérieurement dans le mélange réactionnel ;
en présence d'acide sulfurique, où l'acide sulfurique est présent dans une proportion comprise dans l'intervalle allant de 1,05 à 2,5 équivalents molaires ;
à une température comprise dans l'intervalle allant de la température ambiante à 90 °C ; pour obtenir un mélange comprenant :

a. du sulfate de di-thréo-phénidate de méthyle ; et
b. d'une impureté qui est un composé choisi dans le groupe constitué par un sel de sulfate de l'impureté qui est un composé de formule (IMP-A), un sel de sulfate de l'impureté qui est un composé de formule (IMP-B), un sel de sulfate de l'impureté qui est un composé de formule (IMP-C), un sel de sulfate de l'impureté qui est un composé de formule (IMP-D) et leurs mélanges ;

C. faire réagir le mélange préparé dans l'Étape B avec une base ; et ajouter un solvant organique ; pour obtenir un mélange de di-thréo-phénidate de méthyle sous forme de base libre et de l'impureté est un composé choisi dans le groupe constitué par l'impureté sous forme de base libre est un composé de formule

(IMP-A), l'impureté sous forme de base libre est un composé de formule (IMP-B), l'impureté sous forme de base libre est un composé de formule (IMP-C), l'impureté sous forme de base libre est un composé de formule (IMP-D) et leurs mélanges ; dans un mélange biphasique comprenant une phase aqueuse et une phase organique ;

D. séparer les phases du mélange biphasique l'une de l'autre ; où le di-thréo-phénidate de méthyle sous forme de base libre et l'impureté qui est un composé choisi dans le groupe constitué par l'impureté sous forme de base libre est un composé de formule (IMP-A), l'impureté sous forme de base libre est un composé de formule (IMP-B), l'impureté sous forme de base libre est un composé de formule (IMP-C), l'impureté sous forme de base libre est un composé de formule (IMP-D) et leurs mélanges sont présents dans la phase organique.

**8.** Procédé selon la Revendication 7, où l'acide de l'Étape 3 est l'acide chlorhydrique.

**9.** Procédé selon la Revendication 7, où l'impureté choisie dans le groupe constitué par le sel pharmaceutiquement acceptable de l'impureté qui est un composé de formule (IMP-A), le sel pharmaceutiquement acceptable de l'impureté qui est un composé de formule (IMP-B), le sel pharmaceutiquement acceptable de l'impureté qui est un composé de formule (IMP-C), le sel pharmaceutiquement acceptable de l'impureté qui est un composé de formule (IMP-D) et leurs mélanges est présente dans une proportion inférieure à environ 0,05 % en surface.

**10.** Procédé d'élimination ou de réduction de la proportion d'une impureté qui est un composé de formule (IMP-A)

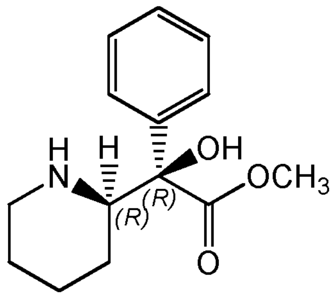

(IMP-A)

ou un sel de celui-ci, une impureté qui est un composé de formule (IMP-B)

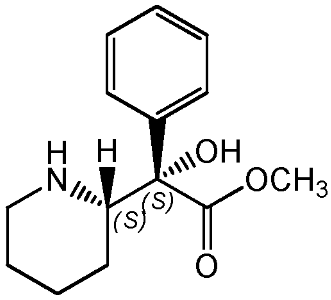

(IMP-B),

ou un sel de celui-ci, une impureté qui est un composé de formule (IMP-C)

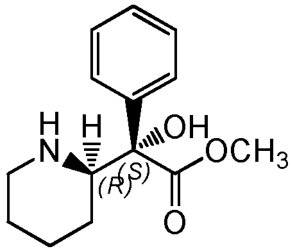

(IMP-C)

ou un sel de celui-ci, une impureté qui est un composé de formule (IMP-D)

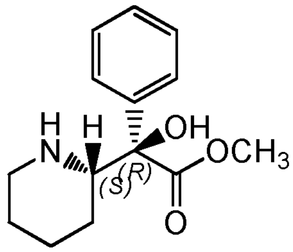

(IMP-D)

ou un sel de celui-ci, ou leurs mélanges, d'avec le composé de formule (I)

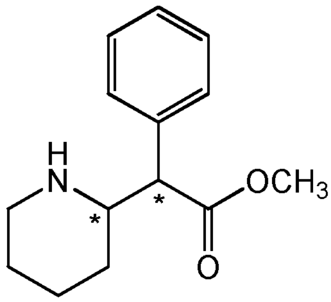

(I)

ou l'un des stéréoisomères, mélanges de stéréoisomères ou sels pharmaceutiquement acceptables de celui-ci, comprenant les étapes consistant à :

a. se munir d'une composition comprenant :

i. un composé de formule (I-A)

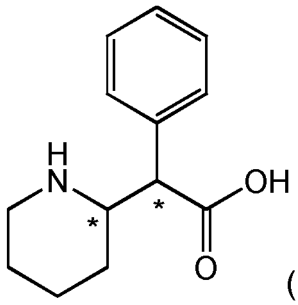

(I-A) ;

ou un stéréoisomère, mélange de stéréoisomères ou sel pharmaceutiquement acceptable de celui-ci ; et
ii. une impureté qui est un composé choisi dans le groupe constitué par une impureté qui est un composé de formule (IMP-1)

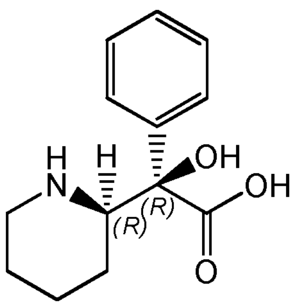

(IMP-1),

une impureté qui est un composé de formule (IMP-2)

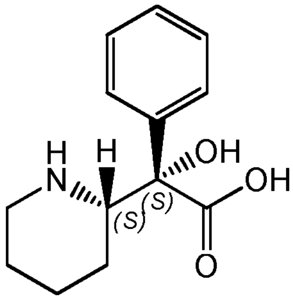

(IMP-2),

une impureté qui est un composé de formule (IMP-11)

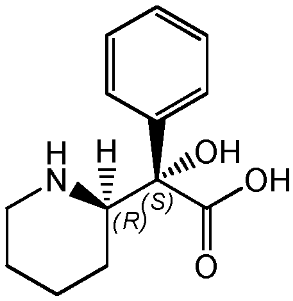

(IMP-11)

une impureté qui est un composé de formule (IMP-12)

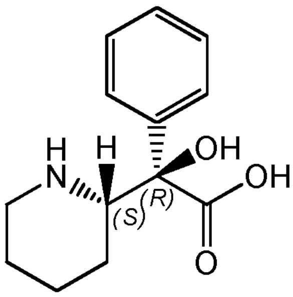

(IMP-12)

ou leurs mélanges.

b. faire réagir la composition de l'Étape a. avec du carbonate de diméthyle pour former un mélange réactionnel ; où le carbonate de diméthyle est présent dans une proportion comprise dans l'intervalle allant de 2,5 à 8,0 équivalents molaires ;

c. ajouter du méthanol au mélange réactionnel ; où le méthanol est présent dans une proportion comprise dans l'intervalle allant de 0,75 à 5,0 équivalents molaires ; où ledit méthanol est ajouté sous forme de composant séparé ; et la proportion de méthanol ajoutée, en une ou plusieurs portions ou aliquotes, est indépendante de tout méthanol pouvant être introduit ultérieurement dans le mélange réactionnel ;

d. ajouter de l'acide sulfurique au mélange réactionnel ; où l'acide sulfurique est présent dans une proportion comprise dans l'intervalle allant de 1,05 à 2,5 équivalents molaires ;

où le mélange réactionnel est à une température comprise dans l'intervalle allant de la température ambiante à 90 °C ; pour obtenir le composé correspondant de formule (I-S) ;

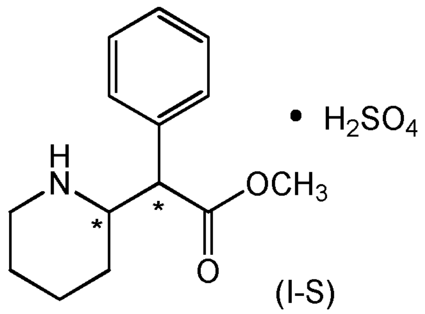

(I-S)

dans un mélange avec un sel de sulfate correspondant de l'impureté qui est un composé de formule (IMP-A), un sel de sulfate correspondant de l'impureté qui est un composé de formule (IMP-B), un sel de sulfate correspondant de l'impureté qui est un composé de formule (IMP-C), un sel de sulfate correspondant de l'impureté qui est un composé de formule (IMP-D) ou leurs mélanges ;

e. faire réagir le mélange de l'Étape d. avec une base ; puis ajouter un solvant organique ; pour obtenir le composé correspondant de formule (I) sous forme de base libre, dans un mélange avec une impureté sous forme de base libre qui est un composé de formule (IMP-A), une impureté sous forme de base libre qui est un composé de formule (IMP-B), une impureté sous forme de base libre qui est un composé de formule (IMP-C), une impureté sous forme de base libre qui est un composé de formule (IMP-D) et leurs mélanges, dans un mélange biphasique comprenant une phase aqueuse et une phase organique ;

f. séparer les phases du mélange biphasique l'une de l'autre ; où : i. le composé de formule (I) et ii. l'impureté sous forme de base libre qui est un composé de formule (IMP-A), l'impureté sous forme de base libre qui est un composé de formule (IMP-B), l'impureté sous forme de base libre qui est un composé de formule (IMP-C), l'impureté sous forme de base libre qui est un composé de formule (IMP-D) et leurs mélanges, sont présents dans la phase organique ;

g. faire réagir un acide avec la phase organique comprenant : i. le composé de formule (I) et ii. l'impureté qui est un composé de formule (IMP-A), l'impureté qui est un composé de formule (IMP-B), l'impureté qui est un composé de formule(IMP-C), l'impureté qui est un composé de formule (IMP-D) et leurs mélanges ; en présence d'eau ; où l'eau est présente dans une proportion comprise dans l'intervalle allant de 0,05 équivalent massique à 1,0 équivalent massique ; pour obtenir un sel d'addition acide correspondant du composé de formule (I) dans un mélange avec un sel d'addition acide correspondant de l'impureté qui est un composé de formule (IMP-A), un sel d'addition acide correspondant de l'impureté qui est un composé de formule (IMP-B), un sel d'addition acide correspondant de l'impureté qui est un composé de formule (IMP-C), un sel d'addition acide correspondant de l'impureté qui est un composé de formule (IMP-D) et leurs mélanges, où le sel d'addition acide correspondant de l'impureté qui est un composé de formule (IMP-A), le sel d'addition acide correspondant de l'impureté qui est un composé de formule (IMP-B), le sel d'addition acide correspondant de l'impureté qui est un composé de formule (IMP-C), le sel d'addition acide correspondant de l'impureté qui est un composé de formule (IMP-D) et leurs mélanges sont extraits dans l'eau ; et

h. séparer l'eau comprenant le sel d'addition acide correspondant de l'impureté qui est un composé de formule (IMP-A), le sel d'addition acide correspondant de l'impureté qui est un composé de formule (IMP-B), le sel d'addition acide correspondant de l'impureté qui est un composé de formule (IMP-C), le sel d'addition acide correspondant de l'impureté qui est un composé de formule (IMP-D) et leurs mélanges du sel d'addition acide correspondant du composé de formule (I) d'avec le mélange de l'Étape g.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007039522A2 A, BOESTEN, W.


### Non-patent literature cited in the description


- **REKHA et al.** *Org. Process Res. Dev.,* 2009, vol. 13, 769-773
- The Handbook of Pharmaceutical Excipients. American Pharmaceutical Association
- Pharmaceutical Dosage Forms: Tablets. Revised and Expanded. vol. 1-3
- Pharmaceutical Dosage Forms: Parenteral Medications. vol. 1-2
- Pharmaceutical Dosage Forms: Disperse Systems. Marcel Dekker, Inc, vol. 1-2